(19) Europäisches Patentamt
European Patent Office
Office européen des brevets

(11) EP 4 063 366 A1

(12) EUROPEAN PATENT APPLICATION
published in accordance with Art. 153(4) EPC

(43) Date of publication:
**28.09.2022 Bulletin 2022/39**

(21) Application number: **20890696.6**

(22) Date of filing: **20.11.2020**

(51) International Patent Classification (IPC):
***C07D 487/04*** *(2006.01)* ***A61K 31/407*** *(2006.01)*
***A61P 31/12*** *(2006.01)*

(52) Cooperative Patent Classification (CPC):
**A61K 31/407; A61P 31/12; C07D 487/04**

(86) International application number:
**PCT/CN2020/130612**

(87) International publication number:
**WO 2021/098850 (27.05.2021 Gazette 2021/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.11.2019 CN 201911171978**

(71) Applicant: **CHIA TAI TIANQING PHARMACEUTICAL GROUP CO., LTD.**
**Lianyungang,**
**Jiangsu 222062 (CN)**

(72) Inventors:
- **HE, Haiying**
  **Shanghai 200131 (CN)**
- **XIA, Jianhua**
  **Shanghai 200131 (CN)**
- **TAN, Haizhong**
  **Shanghai 200131 (CN)**

(74) Representative: **Grünecker Patent- und Rechtsanwälte**
**PartG mbB**
**Leopoldstraße 4**
**80802 München (DE)**

(54) **CRYSTAL FORM OF NUCLEOPROTEIN INHIBITOR AND USE THEREOF**

(57) The present invention relates to a crystal form of a compound of formula (I), a hydrate thereof, a solvate thereof, or a co-complex of water and a solvent, and the use thereof in the preparation of a drug for treating a disease associated with HBV.

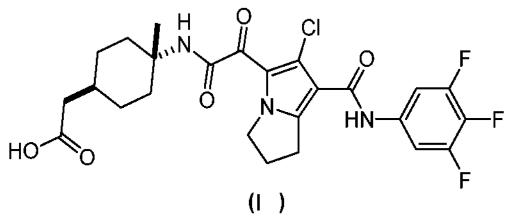

(I)

EP 4 063 366 A1

## Description

## CROSS-REFERENCE TO RELATED APPLICATIONS

The present application claims priority to the Chinese Patent Application No. 201911171978.3 filled on Nov. 22, 2019, which is incorporated herein by reference in its entirety.

## TECHNICAL FIELD

The present application relates to a crystal form of a nucleoprotein inhibitor and use thereof for preparing a medicament for treating HBV (hepatitis B virus) related diseases.

## BACKGROUND

Hepatitis B is an inflammatory reaction caused by the invasion of hepatitis B virus, is easy to develop into hepatic fibrosis and cirrhosis, and is a direct cause of 80% of primary liver cancers worldwide.

Hepatitis B is a global health problem. Currently, there is no specific medicine for treating hepatitis B. Nucleosides and interferons occupy the dominant position in the global anti-hepatitis B drug market, and they are major first-line drugs for treating hepatitis B. However, there are disadvantages of high cost, easy relapse and the like. Thus, there is a need to develop a novel anti-hepatitis B drug.

## SUMMARY

In one aspect, the present application provides a crystal form of a compound of formula (I), a hydrate thereof, a solvate thereof, or a combination of the hydrate and the solvate

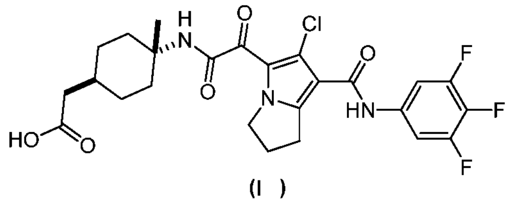

(I) .

The present application provides a crystal form A of a compound of formula (I) having characteristic diffraction peaks in an X-ray powder diffraction (XRPD) pattern thereof at the following 2θ: 6.20±0.20°, 8.90±0.20°, 16.30±0.20° and 24.78±0.20°. In some embodiments of the present application, the aforementioned crystal form A has characteristic diffraction peaks in an X-ray powder diffraction pattern at the following 2θ: 6.20±0.20°, 8.90±0.20°, 14.22±0.20°, 16.30±0.20°, 22.32±0.20° and 24.78±0.20°. In some embodiments of the present application, the aforementioned crystal form A has characteristic diffraction peaks in an X-ray powder diffraction pattern at the following 2θ: 6.20±0.20°, 8.90±0.20°, 11.26±0.20°, 14.22±0.20°, 16.30±0.20°, 17.89±0.20°, 22.32±0.20° and 24.78±0.20°. In some embodiments of the present application, the aforementioned crystal form A has characteristic diffraction peaks in an X-ray powder diffraction pattern at the following 2θ: 6.20±0.20°, 8.90±0.20°, 10.09±0.20°, 11.26±0.20°, 14.22±0.20°, 16.30±0.20°, 17.89±0.20°, 20.35±0.20°, 22.32±0.20°, 24.78±0.20° and 27.78±0.20°.

In some embodiments of the present application, the aforementioned crystal form A has an XRPD pattern as shown in FIG. 1.

In some embodiments of the present application, the aforementioned crystal form A has characteristic diffraction peaks in an XRPD pattern at the following 2θ, as shown in Table 1.

Table 1. XRPD pattern data for crystal form A

| No. | 2θ (±0.20°) | Relative intensity (%) | No. | 2θ (±0.20°) | Relative intensity (%) |
|---|---|---|---|---|---|
| 1 | 6.20 | 100.00 | 8 | 17.89 | 10.83 |
| 2 | 8.90 | 21.04 | 9 | 20.35 | 8.23 |
| 3 | 10.09 | 8.08 | 10 | 22.32 | 32.65 |
| 4 | 11.26 | 17.34 | 11 | 24.78 | 37.82 |

(continued)

| No. | 2θ (±0.20°) | Relative intensity (%) | No. | 2θ (±0.20°) | Relative intensity (%) |
|---|---|---|---|---|---|
| 5 | 11.63 | 8.91 | 12 | 27.78 | 7.17 |
| 6 | 14.22 | 18.82 | 13 | 28.58 | 6.05 |
| 7 | 16.30 | 22.65 | | | |

In some embodiments of the present application, the aforementioned crystal form A shows a weight loss of 2.50% in a thermogravimetric analysis (TGA) curve upon heating to 200.0±3 °C.

In some embodiments of the present application, the aforementioned crystal form A has a TGA pattern as shown in FIG. 2.

In some embodiments of the present application, the aforementioned crystal form A has an endothermic peak in a differential scanning calorimetry (DSC) curve at 234.8±3 °C.

In some embodiments of the present application, the aforementioned crystal form A has a DSC pattern as shown in FIG. 3.

In another aspect, the present application provides a preparation method for the aforementioned crystal form A, which comprises: 1) adding a compound of formula (I) or a crude product thereof to methyl *tert*-butyl ether; 2) optionally concentrating; and 3) lyophilizing to give the crystal form A.

The present application provides a crystal form B of a compound of formula (I) having characteristic diffraction peaks in an X-ray powder diffraction pattern thereof at the following 2θ: 9.13±0.20°, 10.53±0.20°, 21.17±0.20° and 22.64±020°. In some embodiments of the present application, the aforementioned crystal form B has characteristic diffraction peaks in an X-ray powder diffraction pattern at the following 2θ: 9.13±0.20°, 10.53±0.20°, 11.67±0.20°, 20.09±0.20°, 21.17±0.20° and 22.64±0.20°. In some embodiments of the present application, the aforementioned crystal form B has characteristic diffraction peaks in an X-ray powder diffraction pattern at the following 2θ: 9.13±0.20°, 10.53±0.20°, 11.67±0.20°, 13.52±0.20°, 20.09±0.20°, 21.17±0.20° and 22.64±0.20°.

In some embodiments of the present application, the aforementioned crystal form B has an XRPD pattern as shown in FIG. 4.

In some embodiments of the present application, the aforementioned crystal form B has characteristic diffraction peaks in an XRPD pattern at the following 2θ, as shown in Table 2.

Table 2. XRPD pattern data for crystal form B

| No. | 2θ (±0.20°) | Relative intensity (%) |
|---|---|---|
| 1 | 9.13 | 100.00 |
| 2 | 10.53 | 48.53 |
| 3 | 11.67 | 26.24 |
| 4 | 13.52 | 11.69 |
| 5 | 20.09 | 20.70 |
| 6 | 21.17 | 43.87 |
| 7 | 22.64 | 83.94 |

In some embodiments of the present application, the aforementioned crystal form B shows a weight loss of 14.37% in a thermogravimetric analysis curve upon heating to 160.0±3 °C.

In some embodiments of the present application, the aforementioned crystal form B has a TGA pattern as shown in FIG. 5.

In some embodiments of the present application, the aforementioned crystal form B has an endothermic peak in a differential scanning calorimetry (DSC) curve at 149.2±3 °C. In some embodiments of the present application, the aforementioned crystal form B has an endothermic peak in a differential scanning calorimetry (DSC) curve at 236.6±3 °C. In some embodiments of the present application, the aforementioned crystal form B has an endothermic peak in a differential scanning calorimetry (DSC) curve at 149.2±3 °C and/or 236.6±3 °C.

In some embodiments of the present application, the aforementioned crystal form B has a DSC pattern as shown in FIG. 6.

In some embodiments of the present application, the aforementioned crystal form B is a crystal form of a DMSO

solvate of the compound of formula (I). In some embodiments of the present application, a ratio of the number of molecules of the compound of formula (I) to DMSO in the crystal form B is selected from 1:0.8 to 1:2.0, and preferably 1:1.8.

In another aspect, the present application provides a preparation method for the aforementioned crystal form B, which comprises: 1) dissolving a compound of formula (I) in DMSO; and 2) adding water and precipitating a solid to give the crystal form B. In some embodiments of the present application, the crystal form B is prepared by adding the crystal form A of the compound of formula (I) to DMSO. In some embodiments of the present application, in the preparation method for the aforementioned crystal form B, water is added dropwise in step 2). The present application provides a crystal form C of a compound of formula (I) having characteristic diffraction peaks in an X-ray powder diffraction pattern thereof at the following 2θ: 8.94±0.20°, 9.83±0.20° and 10.99±0.20°. In some embodiments of the present application, the aforementioned crystal form C has characteristic diffraction peaks in an X-ray powder diffraction pattern at the following 2θ: 8.94±0.20°, 9.83±0.20°, 10.99±0.20°, 18.62±0.20° and 19.82±0.20°. In some embodiments of the present application, the crystal form C has characteristic diffraction peaks in an X-ray powder diffraction pattern at the following 2θ: 8.94±0.20°, 9.83±0.20°, 10.99±0.20°, 13.36±0.20°, 17.21±0.20°, 18.62±0.20°, 19.82±0.20° and 21.56±0.20°. In some embodiments of the present application, the aforementioned crystal form C has characteristic diffraction peaks in an X-ray powder diffraction pattern at the following 2θ: 8.94±0.20°, 9.39±0.20°, 9.83±0.20°, 10.48±0.20°±020°, 10.99±0.20°, 13.36±0.20°, 14.29±0.20°, 17.21±0.20°, 18.14±0.20°, 18.62±0.20°, 19.82±0.20° and 21.56±020°.

In some embodiments of the present application, the aforementioned crystal form C has an XRPD pattern as shown in FIG. 7.

In some embodiments of the present application, the aforementioned crystal form C has characteristic diffraction peaks in an XRPD pattern at the following 2θ, as shown in Table 3.

Table 3. XRPD pattern data for crystal form C

| No. | 2θ (±0.20°) | Relative intensity (%) | No. | 2θ (±0.20°) | Relative intensity (%) |
|---|---|---|---|---|---|
| 1 | 8.94 | 100.00 | 8 | 14.29 | 5.06 |
| 2 | 9.39 | 19.49 | 9 | 17.21 | 5.84 |
| 3 | 9.83 | 70.18 | 10 | 17.57 | 4.60 |
| 4 | 10.48 | 6.90 | 11 | 18.14 | 5.12 |
| 5 | 10.99 | 28.01 | 12 | 18.62 | 23.44 |
| 6 | 11.75 | 3.72 | 13 | 19.82 | 20.77 |
| 7 | 13.36 | 5.77 | 14 | 21.56 | 10.86 |

In some embodiments of the present application, the aforementioned crystal form C shows a weight loss of 18.33% in a thermogravimetric analysis curve upon heating to 140.0±3 °C.

In some embodiments of the present application, the aforementioned crystal form C has a TGA pattern as shown in FIG. 8.

In some embodiments of the present application, the aforementioned crystal form C has an endothermic peak in a differential scanning calorimetry (DSC) curve at 103.4±3 °C. In some embodiments of the present application, the aforementioned crystal form C has an endothermic peak in a differential scanning calorimetry (DSC) curve at 236.7±3 °C. In some embodiments of the present application, the aforementioned crystal form C has an exothermic peak in a differential scanning calorimetry (DSC) curve at 218.4±3 °C. In some embodiments of the present application, the aforementioned crystal form C has an endothermic peak in a differential scanning calorimetry (DSC) curve at 103.4±3 °C and/or 236.7±3 °C, and/or has an exothermic peak at 218.4±3 °C.

In some embodiments of the present application, the aforementioned crystal form C has a DSC pattern as shown in FIG. 9.

In some embodiments of the present application, the aforementioned crystal form C is a crystal form of a hydrate of the compound of formula (I). In some embodiments of the present application, a ratio of the number of molecules of the compound of formula (I) to water in the crystal form C is selected from 1:6 to 1:8, and preferably 1:6.9.

In another aspect, the present application provides a preparation method for the aforementioned crystal form C, which comprises: 1) dissolving a compound of formula (I) in THF; 2) adding MTBE; and 3) precipitating a solid to give the crystal form C. In some embodiments of the present application, the crystal form C is prepared by adding the crystal form A of the compound of formula (I) to THF. In some embodiments of the present application, in the preparation method for the crystal form C, MTBE is added dropwise in step 2).

The present application provides a crystal form D of a compound of formula (I) having characteristic diffraction peaks in an X-ray powder diffraction pattern thereof at the following 2θ: 7.52±0.20°, 11.21±0.20°, 12.40±0.20° and

14.41±020°. In some embodiments of the present application, the crystal form D has characteristic diffraction peaks in an X-ray powder diffraction pattern at the following 2θ: 7.52±0.20°, 8.70±0.20°, 11.21±0.20°, 12.40±0.20°, 14.41±0.20°, 17.49±0.20° and 22.92±020°. In some embodiments of the present application, the crystal form D has characteristic diffraction peaks in an X-ray powder diffraction pattern at the following 2θ: 7.52±0.20°, 8.70±0.20°, 11.21±0.20°, 12.40±0.20°, 14.41±0.20°, 16.06±0.20°, 17.49±020°, 20.98±0.20°, 21.98±0.20° and 22.92±0.20°. In some embodiments of the present application, the crystal form D has characteristic diffraction peaks in an X-ray powder diffraction pattern at the following 2θ: 7.17±0.20°, 7.52±0.20°, 7.99±0.20°, 8.70±0.20°, 9.99±0.20°, 10.74±0.20°, 11.21±0.20°, 12.40±0.20°, 14.41±0.20°, 14.88±0.20°, 16.06±0.20°, 17.05±0.20°, 17.49±0.20°, 20.98±0.20°, 21.98±0.20°, 22.48±0.20°, 22.92±0.20°, 23.50±0.20°, 26.47±0.20°, 27.05±0.20° and 28.04±020°.

In some embodiments of the present application, the aforementioned crystal form D has an XRPD pattern as shown in FIG. 10.

In some embodiments of the present application, the aforementioned crystal form D has characteristic diffraction peaks in an XRPD pattern at the following 2θ, as shown in Table 4.

Table 4. XRPD pattern data for crystal form D

| No. | 2θ (±0.20°) | Relative intensity (%) | No. | 2θ (±0.20°) | Relative intensity (%) |
|---|---|---|---|---|---|
| 1 | 7.17 | 35.98 | 14 | 17.49 | 28.25 |
| 2 | 7.52 | 100.00 | 15 | 19.20 | 8.14 |
| 3 | 7.99 | 12.67 | 16 | 20.33 | 6.21 |
| 4 | 8.70 | 24.05 | 17 | 20.98 | 28.26 |
| 5 | 9.99 | 11.35 | 18 | 21.98 | 22.62 |
| 6 | 10.74 | 13.34 | 19 | 22.48 | 17.10 |
| 7 | 11.21 | 76.38 | 20 | 22.92 | 29.19 |
| 8 | 12.40 | 42.52 | 21 | 23.50 | 17.36 |
| 9 | 13.98 | 8.87 | 22 | 24.19 | 10.18 |
| 10 | 14.41 | 33.89 | 23 | 26.47 | 10.61 |
| 11 | 14.88 | 10.72 | 24 | 27.05 | 12.45 |
| 12 | 16.06 | 22.26 | 25 | 28.04 | 13.30 |
| 13 | 17.05 | 12.42 | 26 | 31.33 | 8.57 |

In another aspect, the present application provides a preparation method for the crystal form D, which comprises: 1) dissolving a compound of formula (I) in THF; 2) adding DCM; and 3) precipitating a solid to give the crystal form D. In some embodiments of the present application, the crystal form D is prepared by adding the crystal form A of the compound of formula (I) to THF. In some embodiments of the present application, in the preparation method for the crystal form D, DCM is added dropwise in step 2).

The present application provides a crystal form E of a compound of formula (I) having characteristic diffraction peaks in an X-ray powder diffraction pattern thereof at the following 2θ: 6.72±020°, 8.53±0.20°, 17.76±020° and 20.38±0.20°. In some embodiments of the present application, the crystal form E has characteristic diffraction peaks in an X-ray powder diffraction pattern at the following 2θ: 6.72±0.20°, 8.53±0.20°, 10.50±0.20°, 13.53±0.20°, 17.76±0.20°, 18.83±0.20° and 20.38±020°. In some embodiments of the present application, the crystal form E has characteristic diffraction peaks in an X-ray powder diffraction pattern at the following 2θ: 6.72±0.20°, 8.53±0.20°, 10.50±0.20°, 13.53±0.20°, 17.76±0.20°, 18.83±0.20°, 20.38±0.20°, 21.06±0.20° and 24.00±0.20°. In some embodiments of the present application, the crystal form E has characteristic diffraction peaks in an X-ray powder diffraction pattern at the following 2θ: 6.72±0.20°, 8.53±0.20°, 10.50±0.20°, 11.41±0.20°, 13.53±0.20°, 17.76±0.20°, 18.83±0.20°, 19.99±0.20°, 20.38±0.20°, 21.06±0.20°, 22.23±0.20°, 24.00±0.20°, 24.42±0.20° and 25.90±020°.

In some embodiments of the present application, the aforementioned crystal form E has an XRPD pattern as shown in FIG. 11.

In some embodiments of the present application, the aforementioned crystal form E has characteristic diffraction peaks in an XRPD pattern at the following 2θ, as shown in Table 5.

Table 5. XRPD pattern data for crystal form E

| No. | 2θ (±0.20°) | Relative intensity (%) | No. | 2θ (±0.20°) | Relative intensity (%) |
|---|---|---|---|---|---|
| 1 | 6.72 | 22.92 | 10 | 19.99 | 20.73 |
| 2 | 8.53 | 100.00 | 11 | 20.38 | 29.75 |
| 3 | 10.50 | 19.66 | 12 | 21.06 | 11.44 |
| 4 | 11.41 | 11.66 | 13 | 22.23 | 6.20 |
| 5 | 13.53 | 21.98 | 14 | 24.00 | 14.09 |
| 6 | 14.62 | 5.42 | 15 | 24.42 | 11.76 |
| 7 | 17.21 | 5.25 | 16 | 25.90 | 9.97 |
| 8 | 17.76 | 51.32 | 17 | 26.12 | 5.64 |
| 9 | 18.83 | 27.17 | 18 | 38.27 | 6.33 |

In some embodiments of the present application, the aforementioned crystal form E shows a weight loss of 6.61% in a thermogravimetric analysis curve upon heating to 170.0±3 °C, and/or shows a weight loss of 3.53% upon heating from 170±3 °C to 210.0±3 °C.

In some embodiments of the present application, the aforementioned crystal form E has a TGA pattern as shown in FIG. 12.

In some embodiments of the present application, the aforementioned crystal form E has an endothermic peak in a differential scanning calorimetry (DSC) curve at 236.8±3 °C.

In some embodiments of the present application, the aforementioned crystal form E has a DSC pattern as shown in FIG. 13.

In some embodiments of the present application, the aforementioned crystal form E is a crystal form of a co-solvate/solvate of 1,4-dioxane and/or water of the compound of formula (I). In some embodiments of the present application, a ratio of the number of molecules of the compound of formula (I), 1,4-dioxane and water in the crystal form E is selected from 1:0.5-1.0:0.5-1.5, and preferably 1:0.5:1.

In another aspect, the present application provides a preparation method for the crystal form E, which comprises: 1) dissolving a compound of formula (I) in 1,4-dioxane; 2) adding acetonitrile to 1,4-dioxane; and 3) precipitating a solid to give the crystal form E. In some embodiments of the present application, the crystal form E is prepared by adding the crystal form A of the compound of formula (I) to 1,4-dioxane. In some embodiments of the present application, in the preparation method for the crystal form E, acetonitrile is added to 1,4-dioxane by evaporating acetonitrile into 1,4-dioxane in step 2).

The present application provides a crystal form F of a compound of formula (I) having characteristic diffraction peaks in an X-ray powder diffraction pattern thereof at the following 2θ: 4.18±0.20°, 8.35±0.20°, 10.58±0.20° and 16.86±0.20°. In some embodiments of the present application, the aforementioned crystal form F has characteristic diffraction peaks in an X-ray powder diffraction pattern at the following 2θ: 4.18±0.20°, 8.35±0.20°, 10.58±0.20°, 11.87±0.20°, 16.86±0.20° and 21.16±0.20°. In some embodiments of the present application, the aforementioned crystal form F has characteristic diffraction peaks in an X-ray powder diffraction pattern at the following 2θ: 4.18±0.20°, 8.35±0.20°, 10.58±0.20°, 11.87±0.20°, 12.32±020°, 16.86±0.20°, 21.16±0.20°, 25.47±0.20° and 29.17±0.20°.

In some embodiments of the present application, the aforementioned crystal form F has an XRPD pattern as shown in FIG. 14.

In some embodiments of the present application, the aforementioned crystal form F has characteristic diffraction peaks in an XRPD pattern at the following 2θ, as shown in Table 6.

Table 6. XRPD pattern data for crystal form F

| No. | 2θ (±0.20°) | Relative intensity (%) | No. | 2θ (±0.20°) | Relative intensity (%) |
|---|---|---|---|---|---|
| 1 | 4.18 | 15.97 | 6 | 16.86 | 100.00 |
| 2 | 8.35 | 49.02 | 7 | 21.16 | 17.25 |
| 3 | 10.58 | 16.50 | 8 | 25.47 | 6.85 |
| 4 | 11.87 | 8.35 | 9 | 29.17 | 7.10 |
| 5 | 12.32 | 6.29 | | | |

In some embodiments of the present application, the aforementioned crystal form F shows a weight loss of 3.22% in a thermogravimetric analysis curve upon heating to 150.0±3 °C.

In some embodiments of the present application, the aforementioned crystal form F has a TGA pattern as shown in FIG. 15.

In some embodiments of the present application, the aforementioned crystal form F has an endothermic peak in a differential scanning calorimetry (DSC) curve at 229.9±3 °C. In some embodiments of the present application, the aforementioned crystal form F has an endothermic peak in a differential scanning calorimetry (DSC) curve at 188.6±3 °C. In some embodiments of the present application, the aforementioned crystal form F has an endothermic peak in a differential scanning calorimetry (DSC) curve at 98.9±3 °C. In some embodiments of the present application, the aforementioned crystal form F has an endothermic peak in a differential scanning calorimetry (DSC) curve at 229.9±3 °C and/or 188.6±3 °C and/or 98.9±3 °C.

In some embodiments of the present application, the aforementioned crystal form F has a DSC pattern as shown in FIG. 16.

In some embodiments of the present application, the aforementioned crystal form F is a crystal form of a hydrate of the compound of formula (I). In some embodiments of the present application, a ratio of the number of molecules of the compound of formula (I) to water in the crystal form F is selected from 1:0.8 to 1:1.2, and preferably 1:1.

In another aspect, the present application provides a preparation method for the aforementioned crystal form F, which comprises: 1) dissolving a compound of formula (I) in DMF; 2) mixing the resulting solution with water; and 3) precipitating a solid to give the crystal form F. In some embodiments of the present application, the crystal form F is prepared by adding the crystal form A of the compound of formula (I) to DMF. In some embodiments of the present application, in the preparation method for the crystal form F, in step 2), the means for mixing the solution with water is selected from dropwise addition of the solution to water.

The present application provides a crystal form G of a compound of formula (I) having characteristic diffraction peaks in an X-ray powder diffraction pattern thereof at the following 2θ: 7.03±0.20°, 8.31±0.20°, 19.18±0.20° and 25.99±0.20°. In some embodiments of the present application, the crystal form G has characteristic diffraction peaks in an X-ray powder diffraction pattern at the following 2θ: 7.03±0.20°, 8.31±0.20°, 15.86±0.20°, 19.18±0.20°, 21.05±0.20° and 25.99±0.20°. In some embodiments of the present application, the crystal form G has characteristic diffraction peaks in an X-ray powder diffraction pattern at the following 2θ: 7.03±0.20°, 8.31±0.20°, 11.62±0.20°, 12.91±0.20°, 15.86±0.20°, 19.18±0.20°, 21.05±0.20°, 24.67±0.20° and 25.99±0.20°. In some embodiments of the present application, the crystal form G has characteristic diffraction peaks in an X-ray powder diffraction pattern at the following 2θ: 7.03±0.20°, 8.31±020°, 11.62±0.20°, 12.91±020°, 15.86±0.20°, 17.17±0.20°, 18.20±0.20°, 19.18±0.20°, 19.74±0.20°, 21.05±0.20°, 21.30±0.20°, 24.67±0.20° and 25.99±0.20°. In some embodiments of the present application, the aforementioned crystal form G has an XRPD pattern as shown in FIG. 17.

In some embodiments of the present application, the aforementioned crystal form G has characteristic diffraction peaks in an XRPD pattern at the following 2θ, as shown in Table 7.

Table 7. XRPD pattern data for crystal form G

| No. | 2θ (±0.20°) | Relative intensity (%) | No. | 2θ (±0.20°) | Relative intensity (%) |
|---|---|---|---|---|---|
| 1 | 7.03 | 33.31 | 8 | 19.18 | 39.43 |
| 2 | 8.31 | 100.00 | 9 | 19.76 | 21.14 |
| 3 | 11.62 | 13.71 | 10 | 21.05 | 27.73 |
| 4 | 12.91 | 22.79 | 11 | 21.30 | 16.25 |
| 5 | 15.86 | 25.81 | 12 | 24.67 | 19.44 |
| 6 | 17.12 | 8.42 | 13 | 25.99 | 38.36 |
| 7 | 18.20 | 14.31 | | | |

In some embodiments of the present application, the aforementioned crystal form G shows a weight loss of 7.39% in a thermogravimetric analysis curve upon heating to 150.0±3 °C.

In some embodiments of the present application, the aforementioned crystal form G has a TGA pattern as shown in FIG. 18.

In some embodiments of the present application, the aforementioned crystal form G has an endothermic peak in a differential scanning calorimetry (DSC) curve at 235.7±3 °C. In some embodiments of the present application, the aforementioned crystal form G has an exothermic peak in a differential scanning calorimetry (DSC) curve at 177.8±3 °C. In some embodiments of the present application, the aforementioned crystal form G has an endothermic peak in a

differential scanning calorimetry (DSC) curve at 235.7±3 °C, and/or has an exothermic peak at 177.8±3 °C.

In some embodiments of the present application, the aforementioned crystal form G has a DSC pattern as shown in FIG. 19.

In another aspect, the present application provides a preparation method for the crystal form G, which comprises: 1) adding a compound of formula (I) to a mixed solvent of $CHCl_3$ and THF; and 2) precipitating a solid and separating to give the crystal form G. In some embodiments of the present application, the crystal form A of the compound of formula (I) is added to the mixed solvent of $CHCl_3$ and THF.

The present application provides a crystal form H of a compound of formula (I) having characteristic diffraction peaks in an X-ray powder diffraction pattern thereof at the following 2θ: 7.05±0.20°, 9.10±0.20°, 10.78±0.20° and 22.90±0.20°. In some embodiments of the present application, the aforementioned crystal form H has characteristic diffraction peaks in an X-ray powder diffraction pattern at the following 2θ: 7.05±0.20°, 9.10±0.20°, 10.78±0.20°, 21.24±0.20°, 21.74±0.20° and 22.90±020°. In some embodiments of the present application, the aforementioned crystal form H has characteristic diffraction peaks in an X-ray powder diffraction pattern at the following 2θ: 7.05±0.20°, 9.10±0.20°, 10.78±0.20°, 13.07±0.20°, 19.57±020°, 21.24±0.20°, 21.74±0.20°, 22.24±0.20° and 22.90±0.20°. In some embodiments of the present application, the aforementioned crystal form H has characteristic diffraction peaks in an X-ray powder diffraction pattern at the following 2θ: 7.05±0.20°, 9.10±0.20°, 10.78±0.20°, 11.34±0.20°, 13.07±0.20°, 14.07±020°, 14.99±0.20°, 15.86±0.20°, 16.17±0.20°, 18.60±0.20°, 19.57±0.20°, 21.24±0.20°, 21.46±0.20°, 21.74±0.20°, 2224±0.20°, 22.72±0.20° and 22.90±0.20°. In some embodiments of the present application, the aforementioned crystal form H has an XRPD pattern as shown in FIG. 20.

In some embodiments of the present application, the aforementioned crystal form H has characteristic diffraction peaks in an XRPD pattern at the following 2θ, as shown in Table 8.

Table 8. XRPD pattern data for crystal form H

| No. | 2θ (±0.20°) | Relative intensity (%) | No. | 2θ (±0.20°) | Relative intensity (%) |
|---|---|---|---|---|---|
| 1 | 7.05 | 54.83 | 13 | 19.57 | 24.28 |
| 2 | 9.10 | 100.00 | 14 | 21.24 | 36.70 |
| 3 | 9.67 | 7.81 | 15 | 21.46 | 12.31 |
| 4 | 10.78 | 40.40 | 16 | 21.74 | 23.98 |
| 5 | 11.34 | 24.22 | 17 | 22.24 | 24.37 |
| 6 | 13.07 | 22.72 | 18 | 22.72 | 25.49 |
| 7 | 14.07 | 8.48 | 19 | 22.90 | 46.10 |
| 8 | 14.99 | 9.18 | 20 | 23.87 | 7.29 |
| 9 | 15.86 | 11.81 | 21 | 27.42 | 10.34 |
| 10 | 16.17 | 12.32 | 22 | 27.79 | 8.68 |
| 11 | 17.47 | 7.21 | 23 | 28.42 | 8.12 |
| 12 | 18.60 | 15.37 | 24 | 29.06 | 7.22 |

In some embodiments of the present application, the aforementioned crystal form H shows a weight loss of 11.77% in a thermogravimetric analysis curve upon heating to 160.0±3 °C.

In some embodiments of the present application, the aforementioned crystal form H has a TGA pattern as shown in FIG. 21.

In some embodiments of the present application, the aforementioned crystal form H has an endothermic peak in a differential scanning calorimetry (DSC) curve at 140.4±3 °C. In some embodiments of the present application, the aforementioned crystal form H has an endothermic peak in a differential scanning calorimetry (DSC) curve at 236.9±3 °C. In some embodiments of the present application, the aforementioned crystal form H has an endothermic peak in a differential scanning calorimetry (DSC) curve at 140.4±3 °C and/or 236.9±3 °C.

In some embodiments of the present application, the aforementioned crystal form H has a DSC pattern as shown in FIG. 22.

In some embodiments of the present application, the aforementioned crystal form H is a crystal form of a DMF solvate of the compound of formula (I). In some embodiments of the present application, a ratio of the number of molecules of the compound of formula (I) to DMF in the crystal form H is selected from 1:0.6 to 1:1.0, and preferably 1:0.8.

In another aspect, the present application provides a preparation method for the crystal form H, which comprises: 1) adding a compound of formula (I) to a mixed solvent of EtOH and DMF; and 2) precipitating a solid and separating to give the crystal form H. In some embodiments of the present application, the crystal form H is prepared by adding the crystal form A of the compound of formula (I) to the mixed solvent of EtOH and DMF. The present application provides a crystal form I of a compound of formula (I) having characteristic diffraction peaks in an X-ray powder diffraction pattern thereof at the following 2θ: 5.56±0.20°, 11.25±0.20° and 14.09±0.20°. In some embodiments of the present application, the crystal form I has characteristic diffraction peaks in an X-ray powder diffraction pattern at the following 2θ: 5.56±0.20°, 7.54±0.20°, 11.25±0.20°, 14.09±0.20° and 19.64±0.20°. In some embodiments of the present application, the crystal form I has characteristic diffraction peaks in an X-ray powder diffraction pattern at the following 2θ: 5.56±0.20°, 7.54±0.20°, 11.25±0.20°, 14.09±0.20°, 18.07±0.20°, 19.64±0.20° and 20.33±0.20°. In some embodiments of the present application, the aforementioned crystal form I has characteristic diffraction peaks in an X-ray powder diffraction pattern at the following 2θ: 5.56±0.20°, 7.54±0.20°, 11.25±0.20°, 14.09±0.20°, 18.07±0.20°, 19.64±0.20°, 20.33±0.20°, 21.65±0.20° and 22.31±020°.

In some embodiments of the present application, the aforementioned crystal form I has an XRPD pattern as shown in FIG. 23.

In some embodiments of the present application, the aforementioned crystal form I has characteristic diffraction peaks in an XRPD pattern at the following 2θ, as shown in Table 9.

Table 9. XRPD pattern data for crystal form I

| No. | 2θ (±0.20°) | Relative intensity (%) | No. | 2θ (±0.20°) | Relative intensity (%) |
|---|---|---|---|---|---|
| 1 | 5.56 | 100.00 | 7 | 20.33 | 8.33 |
| 2 | 7.54 | 11.90 | 8 | 21.65 | 5.77 |
| 3 | 11.25 | 16.35 | 9 | 22.31 | 6.69 |
| 4 | 14.09 | 13.45 | 10 | 25.43 | 4.70 |
| 5 | 18.07 | 8.01 | 11 | 30.23 | 4.67 |
| 6 | 19.64 | 8.70 | | | |

In some embodiments of the present application, the aforementioned crystal form I shows a weight loss of 3.52% in a thermogravimetric analysis curve upon heating to 100.0±3 °C.

In some embodiments of the present application, the aforementioned crystal form I has a TGA pattern as shown in FIG. 24.

In some embodiments of the present application, the aforementioned crystal form I has an endothermic peak in a differential scanning calorimetry (DSC) curve at 94.7±3 °C. In some embodiments of the present application, the aforementioned crystal form I has an endothermic peak in a differential scanning calorimetry (DSC) curve at 234.4±3 °C. In some embodiments of the present application, the aforementioned crystal form I has an exothermic peak in a differential scanning calorimetry (DSC) curve at 185.2±3 °C. In some embodiments of the present application, the aforementioned crystal form I has an endothermic peak in a differential scanning calorimetry (DSC) curve at 94.7±3 °C and/or 234.4±3 °C, and/or has an exothermic peak in the DSC curve at 185.2±3 °C.

In some embodiments of the present application, the aforementioned crystal form I has a DSC pattern as shown in FIG. 25.

In some embodiments of the present application, the aforementioned crystal form I is a crystal form of a hydrate of the compound of formula (I). In some embodiments of the present application, a ratio of the number of molecules of the compound of formula (I) to water in the crystal form I is selected from 1:1.0 to 1:1.2, and preferably 1:1.1.

In another aspect, the present application provides a preparation method for the crystal form I, which comprises: 1) adding a compound of formula (I) to water; and 2) suspending, stirring and separating to give the crystal form I. In some embodiments of the present application, the crystal form I is prepared by adding the crystal form A of the compound of formula (I) to water. In some embodiments of the present application, in the preparation method for the crystal form I, stirring is performed under heating. In some embodiments of the present application, in the preparation method for the crystal form I, the heating temperature is selected from 45 °C to 60 °C, or is 55 °C. The present application provides a crystal form J of a compound of formula (I) having characteristic diffraction peaks in an X-ray powder diffraction pattern thereof at the following 2θ: 9.28±0.20°, 10.34±0.20°, 22.66±0.20° and 26.12±0.20°. In some embodiments of the present application, the aforementioned crystal form J has characteristic diffraction peaks in an X-ray powder diffraction pattern at the following 2θ: 9.28±0.20°, 10.34±0.20°, 19.45±0.20°, 20.93±0.20°, 22.66±0.20° and 26.12±0.20°. In some embodiments of the present application, the aforementioned crystal form J has characteristic diffraction peaks in

an X-ray powder diffraction pattern at the following 2θ: 9.28±0.20°, 10.34±0.20°, 12.35±0.20°, 14.95±0.20°, 17.88±0.20°, 19.45±0.20°, 20.93±0.20°, 22.66±0.20° and 26.12±0.20°. In some embodiments of the present application, the aforementioned crystal form J has characteristic diffraction peaks in an X-ray powder diffraction pattern at the following 2θ: 3.47±0.20°, 9.28±0.20°, 10.34±0.20°, 10.92±0.20°, 12.35±0.20°, 14.95±0.20°, 17.62±020°, 17.88±0.20°, 19.09±0.20°, 19.45±0.20°, 20.08±0.20°, 20.93±0.20°, 22.66±0.20°, 23.98±0.20°, 26.12±0.20° and 28.63±0.20°. In some embodiments of the present application, the aforementioned crystal form J has an XRPD pattern as shown in FIG. 26.

In some embodiments of the present application, the aforementioned crystal form J has characteristic diffraction peaks in an XRPD pattern at the following 2θ, as shown in Table 10.

Table 10. XRPD pattern data for crystal form J

| No. | 2θ (±0.20°) | Relative intensity (%) | No. | 2θ (±0.20°) | Relative intensity (%) |
|---|---|---|---|---|---|
| 1 | 3.47 | 17.85 | 12 | 19.45 | 47.30 |
| 2 | 9.28 | 89.95 | 13 | 20.08 | 14.19 |
| 3 | 10.34 | 100.00 | 14 | 20.82 | 42.64 |
| 4 | 10.92 | 20.06 | 15 | 20.93 | 46.66 |
| 5 | 12.35 | 33.26 | 16 | 22.18 | 6.13 |
| 6 | 13.15 | 6.30 | 17 | 22.66 | 51.71 |
| 7 | 14.95 | 17.73 | 18 | 23.98 | 14.30 |
| 8 | 17.62 | 20.34 | 19 | 24.88 | 9.64 |
| 9 | 17.88 | 38.66 | 20 | 26.12 | 76.11 |
| 10 | 18.60 | 5.23 | 21 | 28.63 | 12.87 |
| 11 | 19.09 | 10.91 | 22 | 29.75 | 8.98 |

In some embodiments of the present application, the aforementioned crystal form J shows a weight loss of 27.46% in a thermogravimetric analysis curve upon heating to 140.0±3 °C.

In some embodiments of the present application, the aforementioned crystal form J has a TGA pattern as shown in FIG. 27.

In some embodiments of the present application, the aforementioned crystal form J has an endothermic peak in a differential scanning calorimetry (DSC) curve at 86.7±3 °C. In some embodiments of the present application, the aforementioned crystal form J has an endothermic peak in a differential scanning calorimetry (DSC) curve at 229.4±3 °C. In some embodiments of the present application, the aforementioned crystal form J has an endothermic peak in a differential scanning calorimetry (DSC) curve at 150.0±3 °C. In some embodiments of the present application, the aforementioned crystal form J has an exothermic peak in a differential scanning calorimetry (DSC) curve at 148.6±3 °C. In some embodiments of the present application, the aforementioned crystal form J has an endothermic peak in a differential scanning calorimetry (DSC) curve at 86.7±3 °C and/or 150.0±3 °C and/or 229.4±3 °C, and/or has an exothermic peak in the DSC curve at 148.6±3 °C.

In some embodiments of the present application, the aforementioned crystal form J has a DSC pattern as shown in FIG. 28.

In another aspect, the present application provides a preparation method for the crystal form J, which comprises: 1) adding the compound of formula (I) to 2-MeTHF; and 2) suspending, stirring and separating to give the crystal form J. In some embodiments of the present application, the crystal form J is prepared by adding the crystal form A of the compound of formula (I) to 2-MeTHF. In some embodiments of the present application, in the preparation method for the crystal form J, stirring is performed under heating; in some embodiments of the present application, the heating temperature is selected from 45 °C to 60 °C, or is 50 °C.

The present application provides a crystal form K of a compound of formula (I) having characteristic diffraction peaks in an X-ray powder diffraction pattern thereof at the following 2θ: 7.49±020°, 8.46±0.20°, 15.99±020° and 17.02±0.20°. In some embodiments of the present application, the aforementioned crystal form K has characteristic diffraction peaks in an X-ray powder diffraction pattern at the following 2θ: 7.49±0.20°, 8.46±0.20°, 13.18±0.20°, 14.43±0.20°, 15.99±0.20° and 17.02±020°. In some embodiments of the present application, the aforementioned crystal form K has characteristic diffraction peaks in an X-ray powder diffraction pattern at the following 2θ: 7.49±0.20°, 8.46±0.20°, 9.91±020°, 13.18±0.20°, 14.43±0.20°, 15.99±0.20°, 17.02±0.20°, 20.97±0.20° and 24.20±0.20°. In

some embodiments of the present application, the aforementioned crystal form K has characteristic diffraction peaks in an X-ray powder diffraction pattern at the following 2θ: 7.49±0.20°, 8.46±0.20°, 9.12±0.20°, 9.91±0.20°, 10.67±0.20°, 13.18±0.20°, 14.43±0.20°, 15.99±0.20°, 17.02±0.20°, 18.34±0.20°, 19.95±0.20°, 20.29±0.20°, 20.97±0.20°, 21.66±0.20°, 23.03±0.20°, 24.20±0.20°, 24.94±0.20° and 25.69±0.20°.

In some embodiments of the present application, the aforementioned crystal form K has an XRPD pattern as shown in FIG. 29.

In some embodiments of the present application, the aforementioned crystal form K has characteristic diffraction peaks in an XRPD pattern at the following 2θ, as shown in Table 11.

Table 11. XRPD pattern data for crystal form K

| No. | 2θ (±0.20°) | Relative intensity (%) | No. | 2θ (±0.20°) | Relative intensity (%) |
|---|---|---|---|---|---|
| 1 | 7.49 | 47.61 | 13 | 19.95 | 10.87 |
| 2 | 8.46 | 100.00 | 14 | 20.29 | 11.68 |
| 3 | 9.12 | 14.83 | 15 | 20.97 | 34.94 |
| 4 | 9.91 | 16.75 | 16 | 21.66 | 16.32 |
| 5 | 10.67 | 13.56 | 17 | 22.51 | 9.68 |
| 6 | 13.18 | 30.27 | 18 | 23.03 | 11.97 |
| 7 | 14.10 | 11.96 | 19 | 24.20 | 19.76 |
| 8 | 14.43 | 41.58 | 20 | 24.94 | 14.84 |
| 9 | 15.99 | 42.63 | 21 | 25.69 | 10.59 |
| 10 | 16.57 | 4.84 | 22 | 26.56 | 4.97 |
| 11 | 17.02 | 46.68 | 23 | 28.16 | 4.30 |
| 12 | 18.34 | 15.00 | 24 | 30.38 | 4.91 |

In some embodiments of the present application, the aforementioned crystal form K shows a weight loss of 1.35% in a thermogravimetric analysis curve upon heating to 150.0±3 °C.

In some embodiments of the present application, the aforementioned crystal form K has a TGA pattern as shown in FIG. 30.

In some embodiments of the present application, the aforementioned crystal form K has an endothermic peak in a differential scanning calorimetry (DSC) curve at 231.2±3 °C. In some embodiments of the present application, the aforementioned crystal form K has an exothermic peak in a differential scanning calorimetry (DSC) curve at 164.1±3 °C. In some embodiments of the present application, the aforementioned crystal form K has an endothermic peak in a differential scanning calorimetry (DSC) curve at 160.6±3 °C. In some embodiments of the present application, the aforementioned crystal form K has an endothermic peak in a differential scanning calorimetry (DSC) curve at 231.2±3 °C and/or 160.6±3 °C, and/or has an exothermic peak in the DSC curve at 164.1±3 °C.

In some embodiments of the present application, the aforementioned crystal form K has a DSC pattern as shown in FIG. 31.

In another aspect, the present application provides a preparation method for the crystal form K, which comprises: 1) adding the compound of formula (I) to a mixed solvent of DCM and MeOH; and 2) precipitating a solid and separating to give the crystal form K. In some embodiments of the present application, the crystal form K is prepared by adding the crystal form A of the compound of formula (I) to the mixed solvent of DCM and MeOH. In some embodiments of the present application, in the preparation method for the crystal form K, dissolving is performed under heating. In some embodiments of the present application, in the preparation method for the crystal form K, the heating temperature is selected from 45 °C to 60 °C, or is 50 °C. In some embodiments of the present application, in the preparation method for the crystal form K, the solid is precipitated by cooling in step 2). The present application provides a crystal form L of a compound of formula (I) having characteristic diffraction peaks in an X-ray powder diffraction pattern thereof at the following 2θ: 8.53±0.20°, 11.09±0.20°, 22.34±0.20° and 23.12±0.20°. In some embodiments of the present application, the aforementioned crystal form L has characteristic diffraction peaks in an X-ray powder diffraction pattern at the following 2θ: 8.53±0.20°, 11.09±0.20°, 15.00±0.20°, 20.76±0.20°, 22.34±0.20° and 23.12±0.20°. In some embodiments of the present application, the aforementioned crystal form L has characteristic diffraction peaks in an X-ray powder diffraction pattern at the following 2θ: 7.03±0.20°, 8.53±0.20°, 11.09±0.20°, 14.14±0.20°, 15.00±0.20°,

20.76±0.20°, 22.34±0.20°, 23.12±0.20° and 26.85±0.20°. In some embodiments of the present application, the aforementioned crystal form L has characteristic diffraction peaks in an X-ray powder diffraction pattern at the following 2θ: 7.03±0.20°, 8.53±0.20°, 10.50±0.20°, 11.09±0.20°, 14.14±0.20°, 15.00±0.20°, 20.76±020°, 22.34±0.20°, 23.12±0.20° and 26.85±0.20°.

In some embodiments of the present application, the aforementioned crystal form L has an XRPD pattern as shown in FIG. 32.

In some embodiments of the present application, the aforementioned crystal form L has characteristic diffraction peaks in an XRPD pattern at the following 2θ, as shown in Table 12.

Table 12. XRPD pattern data for crystal form L

| No. | 2θ (±0.20°) | Relative intensity (%) | No. | 2θ (±0.20°) | Relative intensity (%) |
|---|---|---|---|---|---|
| 1 | 7.03 | 13.73 | 9 | 20.76 | 35.20 |
| 2 | 7.91 | 5.21 | 10 | 21.30 | 11.53 |
| 3 | 8.53 | 76.66 | 11 | 21.55 | 5.15 |
| 4 | 10.50 | 18.12 | 12 | 22.34 | 49.63 |
| 5 | 11.09 | 82.87 | 13 | 23.12 | 100.00 |
| 6 | 14.14 | 11.11 | 14 | 25.89 | 4.63 |
| 7 | 15.00 | 25.17 | 15 | 26.85 | 22.11 |
| 8 | 20.15 | 7.41 | 16 | 32.16 | 6.88 |

In some embodiments of the present application, the aforementioned crystal form L shows a weight loss of 10.37% in a thermogravimetric analysis curve upon heating to 160.0±3 °C.

In some embodiments of the present application, the aforementioned crystal form L has a TGA pattern as shown in FIG. 33.

In some embodiments of the present application, the aforementioned crystal form L has an endothermic peak in a differential scanning calorimetry (DSC) curve at 150.1±3 °C. In some embodiments of the present application, the aforementioned crystal form L has an endothermic peak in a differential scanning calorimetry (DSC) curve at 210.7±3 °C. In some embodiments of the present application, the aforementioned crystal form L has an endothermic peak in a differential scanning calorimetry (DSC) curve at 150.1±3 °C and/or 210.7±3 °C.

In some embodiments of the present application, the aforementioned crystal form L has a DSC pattern as shown in FIG. 34.

In some embodiments of the present application, the aforementioned crystal form L is a crystal form of an NMP solvate of the compound of formula (I). In some embodiments of the present application, a ratio of the number of molecules of the compound of formula (I) to NMP in the crystal form L is selected from 1:0.5 to 1:1.0, and preferably 1:0.8.

In another aspect, the present application provides a preparation method for the aforementioned crystal form L, which comprises: 1) dissolving a compound of formula (I) in NMP; 2) adding EtOAc to the resulting NMP solution; and 3) precipitating a solid and separating to give the crystal form L. In some embodiments of the present application, the crystal form L is prepared by adding the crystal form A of the compound of formula (I) to NMP. In some embodiments of the present application, in the preparation method for the crystal form L, EtOAc is added to NMP by evaporating EtOAc into NMP in step 2).

The present application provides a crystal form M of a compound of formula (I) having characteristic diffraction peaks in an X-ray powder diffraction pattern thereof at the following 2θ: 9.32±0.20°, 10.43±0.20°, 12.46±0.20° and 19.62±0.20°. In some embodiments of the present application, the aforementioned crystal form M has characteristic diffraction peaks in an X-ray powder diffraction pattern at the following 2θ: 9.32±0.20°, 10.43±0.20°, 10.81±0.20°, 12.46±0.20°, 19.62±0.20° and 21.03±0.20°. In some embodiments of the present application, the aforementioned crystal form M has characteristic diffraction peaks in an X-ray powder diffraction pattern at the following 2θ: 9.32±0.20°, 10.43±0.20°, 10.81±0.20°, 12.46±0.20°, 17.55±0.20°, 17.99±0.20°, 19.62±0.20°, 21.03±0.20° and 22.90±0.20°. In some embodiments of the present application, the crystal form M has characteristic diffraction peaks in an X-ray powder diffraction pattern at the following 2θ: 9.32±0.20°, 10.43±0.20°, 10.81±0.20°, 12.46±0.20°, 13.00±0.20°, 15.06±0.20°, 17.55±0.20°, 17.99±0.20°, 19.62±0.20°, 21.03±0.20° and 22.90±0.20°.

In some embodiments of the present application, the aforementioned crystal form M has an XRPD pattern as shown in FIG. 35.

In some embodiments of the present application, the aforementioned crystal form M has characteristic diffraction

peaks in an XRPD pattern at the following 2θ, as shown in Table 13.

Table 13. XRPD pattern data for crystal form M

| No. | 2θ (±0.20°) | Relative intensity (%) | No. | 2θ (±0.20°) | Relative intensity (%) |
|---|---|---|---|---|---|
| 1 | 9.32 | 100.00 | 7 | 17.55 | 10.37 |
| 2 | 10.43 | 79.23 | 8 | 17.99 | 6.83 |
| 3 | 10.81 | 17.00 | 9 | 19.62 | 19.48 |
| 4 | 12.46 | 20.62 | 10 | 21.03 | 19.15 |
| 5 | 13.00 | 6.65 | 11 | 22.90 | 14.14 |
| 6 | 15.06 | 5.75 | 12 | 26.43 | 3.59 |

In some embodiments of the present application, the aforementioned crystal form M shows a weight loss of 10.98% in a thermogravimetric analysis curve upon heating to 130.0±3 °C.

In some embodiments of the present application, the aforementioned crystal form M has a TGA pattern as shown in FIG. 36.

In some embodiments of the present application, the aforementioned crystal form M has an endothermic peak in a differential scanning calorimetry (DSC) curve at 109.7±3 °C. In some embodiments of the present application, the aforementioned crystal form M has an endothermic peak in a differential scanning calorimetry (DSC) curve at 235.9±3 °C. In some embodiments of the present application, the aforementioned crystal form M has an endothermic peak in a differential scanning calorimetry (DSC) curve at 109.7±3 °C and/or 235.9±3 °C.

In some embodiments of the present application, the aforementioned crystal form M has a DSC pattern as shown in FIG. 37.

In some embodiments of the present application, the aforementioned crystal form M is a crystal form of a THF solvate of the compound of formula (I). In some embodiments of the present application, a ratio of the number of molecules of the compound of formula (I) to TMF in the crystal form M is selected from 1:0.6 to 1:1.0, and preferably 1:0.8.

In another aspect, the present application provides a preparation method for the aforementioned crystal form M, which comprises: 1) dissolving a compound of formula (I) in THF; and 2) precipitating a solid and separating to give the crystal form M. In some embodiments of the present application, the crystal form M is prepared by adding the crystal form A of the compound of formula (I) to THF.

The present application provides a crystal form N of a compound of formula (I) having characteristic diffraction peaks in an X-ray powder diffraction pattern thereof at the following 2θ: 8.47°±0.20°, 12.62±0.20°, 15.70±0.20° and 18.41±0.20°. In some embodiments of the present application, the aforementioned crystal form N has characteristic diffraction peaks in an X-ray powder diffraction pattern at the following 2θ: 8.47±0.20°, 11.23±0.20°, 12.62±0.20°, 15.70±0.20°, 18.41±0.20° and 21.49±0.20°. In some embodiments of the present application, the aforementioned crystal form N has characteristic diffraction peaks in an X-ray powder diffraction pattern at the following 2θ: 7.04±0.20°, 8.47±0.20°, 10.01±0.20°, 11.23±0.20°, 12.62±0.20°, 15.70±0.20°, 18.41±0.20°, 21.49±0.20° and 22.53±0.20°. In some embodiments of the present application, the aforementioned crystal form N has characteristic diffraction peaks in an X-ray powder diffraction pattern at the following 2θ: 7.04±0.20°, 8.47±0.20°, 10.01±0.20°, 11.23±0.20°, 12.62±0.20°, 15.70±0.20°, 17.32±020°, 18.41±0.20°, 20.31±0.20°, 21.49±0.20°, 22.53±0.20° and 26.34±0.20°.

In some embodiments of the present application, the aforementioned crystal form N has an XRPD pattern as shown in FIG. 38.

In some embodiments of the present application, the aforementioned crystal form N has characteristic diffraction peaks in an XRPD pattern at the following 2θ, as shown in Table 14.

Table 14. XRPD pattern data for crystal form N

| No. | 2θ (±0.20°) | Relative intensity (%) | No. | 2θ (±0.20°) | Relative intensity (%) |
|---|---|---|---|---|---|
| 1 | 7.04 | 12.38 | 8 | 18.41 | 100.00 |
| 2 | 8.47 | 14.77 | 9 | 20.31 | 6.19 |
| 3 | 10.01 | 16.20 | 10 | 21.49 | 27.42 |
| 4 | 11.23 | 43.59 | 11 | 22.53 | 20.36 |
| 5 | 12.62 | 76.09 | 12 | 26.34 | 8.28 |

(continued)

| No. | 2θ (±0.20°) | Relative intensity (%) | No. | 2θ (±0.20°) | Relative intensity (%) |
|---|---|---|---|---|---|
| 6 | 15.70 | 69.18 | 13 | 30.67 | 5.98 |
| 7 | 17.32 | 6.52 | | | |

In some embodiments of the present application, the aforementioned crystal form N shows a weight loss of 1.99% in a thermogravimetric analysis curve upon heating to 200.0±3 °C.

In some embodiments of the present application, the aforementioned crystal form N has a TGA pattern as shown in FIG. 39.

In some embodiments of the present application, the aforementioned crystal form N has an endothermic peak in a differential scanning calorimetry (DSC) curve at 236.3±3 °C.

In some embodiments of the present application, the aforementioned crystal form N has a DSC pattern as shown in FIG. 40.

In another aspect, the present application provides a preparation method for the aforementioned crystal form N, which comprises: 1) dissolving a compound of formula (I) in EtOH; and 2) precipitating a solid and separating to give the crystal form N. In some embodiments of the present application, the crystal form N is prepared by adding the crystal form A of the compound of formula (I) to EtOH.

The present application provides a crystal form O of a compound of formula (I) having characteristic diffraction peaks in an X-ray powder diffraction pattern thereof at the following 2θ: 7.50±0.20°, 10.65±0.20° and 11.10±0.20°. In some embodiments of the present application, the aforementioned crystal form O has characteristic diffraction peaks in an X-ray powder diffraction pattern at the following 2θ: 7.18±0.20°, 7.50±0.20°, 10.65±0.20°, 11.10±0.20°, 14.04±0.20° and 21.48±0.20°. In some embodiments of the present application, the aforementioned crystal form O has characteristic diffraction peaks in an X-ray powder diffraction pattern at the following 2θ: 7.18±0.20°, 7.50±0.20°, 10.65±0.20°, 11.10±0.20°, 14.04±0.20°, 21.48±0.20°, 22.79±0.20° and 27.02±0.20°. In some embodiments of the present application, the crystal form O has characteristic diffraction peaks in an X-ray powder diffraction pattern at the following 2θ: 7.18±0.20°, 7.50±0.20°, 10.65±0.20°, 11.10±0.20°, 14.04±0.20°, 15.67±0.20°, 19.16±0.20°, 21.48±0.20°, 22.79±0.20°, 23.39±0.20°, 26.28±0.20° and 27.02±0.20°.

In some embodiments of the present application, the aforementioned crystal form O has an XRPD pattern as shown in FIG. 41.

In some embodiments of the present application, the aforementioned crystal form O has characteristic diffraction peaks in an XRPD pattern at the following 2θ, as shown in Table 15.

Table 15. XRPD pattern data for crystal form O

| No. | 2θ (±0.20°) | Relative intensity (%) | No. | 2θ (±0.20°) | Relative intensity (%) |
|---|---|---|---|---|---|
| 1 | 7.18 | 51.90 | 7 | 19.16 | 5.60 |
| 2 | 7.50 | 100.00 | 8 | 21.48 | 10.95 |
| 3 | 10.65 | 15.11 | 9 | 22.79 | 8.75 |
| 4 | 11.10 | 40.78 | 10 | 23.39 | 5.06 |
| 5 | 14.04 | 9.85 | 11 | 26.28 | 7.39 |
| 6 | 15.67 | 4.86 | 12 | 27.02 | 9.30 |

In some embodiments of the present application, the aforementioned crystal form O shows a weight loss of 2.23% in a thermogravimetric analysis curve upon heating to 140.0±3 °C.

In some embodiments of the present application, the aforementioned crystal form O has a TGA pattern as shown in FIG. 42.

In some embodiments of the present application, the aforementioned crystal form O has an endothermic peak in a differential scanning calorimetry (DSC) curve at 123.3±3 °C. In some embodiments of the present application, the aforementioned crystal form O has an exothermic peak in a differential scanning calorimetry (DSC) curve at 128.5±3 °C. In some embodiments of the present application, the aforementioned crystal form O has an endothermic peak in a differential scanning calorimetry (DSC) curve at 231.1±3 °C. In some embodiments of the present application, the aforementioned crystal form O has an endothermic peak in a differential scanning calorimetry (DSC) curve at 237.1±3 °C. In some embodiments of the present application, the aforementioned crystal form O has an endothermic peak in a

differential scanning calorimetry (DSC) curve at 123.3±3 °C and/or 231.1±3 °C and/or 237.1±3 °C, and/or has an exothermic peak in the DSC curve at 128.5±3 °C.

In some embodiments of the present application, the aforementioned crystal form O has a DSC pattern as shown in FIG. 43.

In some embodiments of the present application, the aforementioned crystal form O is a crystal form of a hydrate of the compound of formula (I). In some embodiments of the present application, a ratio of the number of molecules of the compound of formula (I) to water in the crystal form O is selected from 1:0.6 to 1:1.0, and preferably 1:0.7.

In another aspect, the present application provides a preparation method for the crystal form O, which comprises: drying the aforementioned crystal form D under vacuum at room temperature to give the crystal form O.

The present application provides a crystal form P of a compound of formula (I) having characteristic diffraction peaks in an X-ray powder diffraction pattern thereof at the following 2θ: 7.08±0.20° and 21.48±0.20°. In some embodiments of the present application, the aforementioned crystal form P has characteristic diffraction peaks in an X-ray powder diffraction pattern at the following 2θ: 7.08±0.20°, 21.25±0.20° and 21.48±0.20°. In some embodiments of the present application, the aforementioned crystal form P has characteristic diffraction peaks in an X-ray powder diffraction pattern at the following 2θ: 7.08±0.20°, 20.39±0.20°, 21.25±0.20°, 21.48±0.20°, 26.74±0.20° and 27.46±0.20°. In some embodiments of the present application, the aforementioned crystal form P has characteristic diffraction peaks in an X-ray powder diffraction pattern at the following 2θ: 7.08±0.20°, 20.39±0.20°, 21.48±0.20°, 26.74±0.20° and 27.46±020°. In some embodiments of the present application, the aforementioned crystal form P has characteristic diffraction peaks in an X-ray powder diffraction pattern at the following 2θ: 7.08±020°, 12.92±0.20°, 18.61±0.20°, 20.39±0.20°, 21.48±0.20°, 22.71±0.20°, 26.74±0.20°, 27.46±0.20° and 27.83±0.20°. In some embodiments of the present application, the aforementioned crystal form P has characteristic diffraction peaks in an X-ray powder diffraction pattern at the following 2θ: 7.08±0.20°, 12.92±0.20°, 18.61±0.20°, 20.39±0.20°, 21.25±0.20°, 21.48±0.20°, 22.71±0.20°, 25.01±0.20°, 26.74±0.20°, 27.46±0.20° and 27.83±0.20°.

In some embodiments of the present application, the crystal form P has characteristic diffraction peaks in an X-ray powder diffraction pattern at the following 2θ: 7.08±0.20°, 15.36±0.20°, 21.25±0.20°, 21.48±0.20° and 22.71±0.20°. In some embodiments of the present application, the crystal form P has characteristic diffraction peaks in an X-ray powder diffraction pattern at the following 2θ: 7.08±020°, 12.92±0.20°, 15.36±0.20°, 21.25±0.20°, 21.48±0.20°, 22.71±0.20° and 27.46±020°. In some embodiments of the present application, the crystal form P has characteristic diffraction peaks in an X-ray powder diffraction pattern at the following 2θ: 7.08±0.20°, 12.92±0.20°, 15.36±020°, 20.39±0.20°, 21.25±0.20°, 21.48±0.20°, 22.71±020°, 26.74±020° and 27.46±0.20°. In some embodiments of the present application, the crystal form P has characteristic diffraction peaks in an X-ray powder diffraction pattern at the following 2θ: 7.08±020°, 12.92±0.20°, 15.36±0.20°, 18.61±0.20°, 20.39±0.20°, 21.25±0.20°, 21.48±0.20°, 22.71±0.20°, 26.74±0.20°, 27.46±0.20° and 27.83±0.20°. In some embodiments of the present application, the crystal form P has characteristic diffraction peaks in an X-ray powder diffraction pattern at the following 2θ: 7.08±0.20°, 11.61±0.20°, 12.92±0.20°, 15.36±0.20°, 15.89±0.20°, 18.61±0.20°, 20.39±0.20°, 21.25±0.20°, 21.48±0.20°, 22.71±0.20°, 26.74±0.20°, 27.46±0.20° and 27.83±0.20°. In some embodiments of the present application, the crystal form P has characteristic diffraction peaks in an X-ray powder diffraction pattern at the following 2θ: 7.08±0.20°, 11.61±0.20°, 12.92±0.20°, 15.36±0.20°, 15.89±0.20°, 18.61±0.20°, 19.89±0.20°, 20.39±0.20°, 21.25±0.20°, 21.48±0.20°, 22.71±0.20°, 26.05±0.20°, 26.74±0.20°, 27.46±0.20° and 27.83±0.20°.

In some embodiments of the present application, the aforementioned crystal form P has an XRPD pattern as shown in FIG. 44A or FIG. 44B.

In some embodiments of the present application, the aforementioned crystal form P has characteristic diffraction peaks in an XRPD pattern at the following 2θ, as shown in Table 16A.

Table 16A. XRPD pattern data for crystal form P

| No. | 2θ (±0.20°) | Relative intensity (%) | No. | 2θ (±0.20°) | Relative intensity (%) |
|---|---|---|---|---|---|
| 1 | 7.08 | 100.00 | 14 | 21.48 | 27.17 |
| 3 | 12.92 | 3.83 | 15 | 22.71 | 4.17 |
| 4 | 14.25 | 2.64 | 16 | 25.01 | 3.17 |
| 5 | 15.36 | 2.73 | 18 | 26.74 | 6.42 |
| 9 | 18.61 | 4.23 | 19 | 27.46 | 5.07 |
| 12 | 20.39 | 5.87 | 20 | 27.83 | 4.86 |
| 13 | 21.25 | 15.87 | | | |

In some embodiments of the present application, the aforementioned crystal form P has characteristic diffraction peaks in an XRPD pattern at the following 2θ, as shown in Table 16B.

Table 16B. XRPD pattern data for crystal form P

| No. | 2θ (±0.20°) | Relative intensity (%) | No. | 2θ (±0.20°) | Relative intensity (%) |
|---|---|---|---|---|---|
| 1 | 7.08 | 100.00 | 16 | 25.01 | 3.17 |
| 2 | 11.61 | 0.40 | 17 | 26.05 | 0.36 |
| 3 | 12.92 | 3.83 | 18 | 26.74 | 6.42 |
| 4 | 14.25 | 2.64 | 19 | 27.46 | 5.07 |
| 5 | 15.36 | 2.73 | 20 | 27.83 | 4.86 |
| 6 | 15.89 | 1.20 | 21 | 28.46 | 1.79 |
| 7 | 16.43 | 1.40 | 22 | 31.34 | 0.76 |
| 8 | 17.51 | 1.19 | 23 | 32.12 | 0.58 |
| 9 | 18.61 | 4.23 | 24 | 33.21 | 1.26 |
| 10 | 19.03 | 1.79 | 25 | 34.34 | 0.66 |
| 11 | 19.89 | 0.83 | 26 | 34.74 | 0.89 |
| 12 | 20.39 | 5.87 | 27 | 36.17 | 1.08 |
| 13 | 21.25 | 15.87 | 28 | 36.89 | 0.24 |
| 14 | 21.48 | 27.17 | 29 | 37.75 | 0.60 |
| 15 | 22.71 | 4.17 | 30 | 38.70 | 0.42 |

In some embodiments of the present application, the aforementioned crystal form P shows a weight loss of 2.76% in a thermogravimetric analysis curve upon heating to 150.0±3 °C.

In some embodiments of the present application, the aforementioned crystal form P has a TGA pattern as shown in FIG. 45.

In some embodiments of the present application, the aforementioned crystal form P has an endothermic peak in a differential scanning calorimetry (DSC) curve at 236.1±3 °C.

In some embodiments of the present application, the aforementioned crystal form P has a DSC pattern as shown in FIG. 46.

In some other embodiments of the present application, the crystal form P has characteristic diffraction peaks in an X-ray powder diffraction pattern at the following 2θ: 7.12±0.20° and 21.46±0.20°. In some embodiments of the present application, the aforementioned crystal form P has characteristic diffraction peaks in an X-ray powder diffraction pattern at the following 2θ: 7.12±0.20°, 21.28±0.20° and 21.46±0.20°. In some embodiments of the present application, the aforementioned crystal form P has characteristic diffraction peaks in an X-ray powder diffraction pattern at the following 2θ: 7.12±0.20°, 20.38±0.20°, 21.28±0.20°, 21.46±0.20°, 26.72±0.20° and 27.48±0.20°. In some embodiments of the present application, the aforementioned crystal form P has characteristic diffraction peaks in an X-ray powder diffraction pattern at the following 2θ: 7.12±0.20°, 20.38±0.20°, 21.46±0.20°, 26.72±0.20° and 27.48±0.20°. In some embodiments of the present application, the aforementioned crystal form P has characteristic diffraction peaks in an X-ray powder diffraction pattern at the following 2θ: 7.12±0.20°, 12.96±0.20°, 18.66±0.20°, 20.38±0.20°, 21.46±0.20°, 22.74±0.20°, 26.72±0.20°, 27.48±0.20° and 27.82±0.20°. In some embodiments of the present application, the aforementioned crystal form P has characteristic diffraction peaks in an X-ray powder diffraction pattern at the following 2θ: 7.12±0.20°, 12.96±0.20°, 18.66±0.20°, 20.38±0.20°, 21.28±0.20°, 21.46±0.20°, 22.74±0.20°, 24.84±0.20°, 26.72±0.20°, 27.48±0.20° and 27.82±0.20°.

In some other embodiments of the present application, the crystal form P has characteristic diffraction peaks in an X-ray powder diffraction pattern at the following 2θ: 7.12±0.20°, 15.40±0.20°, 21.28±0.20°, 21.46±0.20° and 22.74±0.20°. In some embodiments of the present application, the aforementioned crystal form P has characteristic diffraction peaks in an X-ray powder diffraction pattern at the following 2θ: 7.12±0.20°, 12.96±0.20°, 15.40±0.20°, 21.28±0.20°, 21.46±0.20°, 22.74±0.20° and 27.48±0.20°. In some embodiments of the present application, the aforementioned crystal form P has characteristic diffraction peaks in an X-ray powder diffraction pattern at the following 2θ: 7.12±0.20°, 12.96±0.20°, 15.40±0.20°, 20.38±0.20°, 21.28±0.20°, 21.46±0.20°, 22.74±0.20°, 26.72±0.20° and

27.48±0.20°. In some embodiments of the present application, the aforementioned crystal form P has characteristic diffraction peaks in an X-ray powder diffraction pattern at the following 2θ: 7.12±0.20°, 12.96±0.20°, 15.40±0.20°, 18.66±0.20°, 20.38±0.20°, 21.28±0.20°, 21.46±0.20°, 22.74±0.20°, 26.72±0.20°, 27.48±0.20° and 27.82±0.20°.

In some embodiments of the present application, the aforementioned crystal form P has characteristic diffraction peaks in an X-ray powder diffraction pattern at the following 2θ: 7.12±0.20°, 8.74±0.20°, 12.96±0.20°, 15.40±0.20°, 15.92±0.20°, 18.66±0.20°, 20.38±0.20°, 21.28±0.20°, 21.46±0.20°, 22.74±0.20°, 26.72±0.20° and 27.48±0.20°. In some embodiments of the present application, the aforementioned crystal form P has characteristic diffraction peaks in an X-ray powder diffraction pattern at the following 2θ: 7.12±0.20°, 8.74±0.20°, 11.66±0.20°, 12.96±0.20°, 15.40±0.20°, 15.92±0.20°, 16.22±0.20°, 18.66±0.20°, 20.38±0.20°, 21.28±0.20°, 21.46±0.20°, 22.74±0.20°, 26.72±0.20°, 27.48±0.20° and 27.82±0.20°. In some embodiments of the present application, the aforementioned crystal form P has characteristic diffraction peaks in an X-ray powder diffraction pattern at the following 2θ: 7.12±0.20°, 8.74±0.20°, 929±0.20°, 11.66±0.20°, 12.96±0.20°, 15.40±0.20°, 15.92±0.20°, 16.22±0.20°, 17.54±0.20°, 18.66±0.20°, 20.38±0.20°, 21.28±0.20°, 21.46±0.20°, 22.74±0.20°, 26.72±0.20°, 27.48±0.20° and 27.82±0.20°.

In some embodiments of the present application, the aforementioned crystal form P has an XRPD pattern as shown in FIG. 59.

In some embodiments of the present application, the aforementioned crystal form P has characteristic diffraction peaks in an XRPD pattern at the following 2θ, as shown in Table 16C.

Table 16C. XRPD pattern data for crystal form P

| No. | 2θ (±0.20°) | Relative intensity (%) | No. | 2θ (±0.20°) | Relative intensity (%) |
|---|---|---|---|---|---|
| 1 | 7.12 | 53.4 | 24 | 25.22 | 17.1 |
| 2 | 8.74 | 9.2 | 25 | 25.96 | 11.1 |
| 3 | 9.29 | 5.8 | 26 | 26.72 | 16.3 |
| 4 | 10.86 | 3.5 | 27 | 27.48 | 56.5 |
| 5 | 11.66 | 8.6 | 28 | 27.82 | 14.0 |
| 6 | 12.96 | 15.5 | 29 | 28.44 | 11.5 |
| 7 | 14.28 | 2.9 | 30 | 29.38 | 5.3 |
| 8 | 14.56 | 4.4 | 31 | 31.12 | 12.2 |
| 9 | 15.40 | 22.4 | 32 | 31.65 | 4.2 |
| 10 | 15.92 | 40.9 | 33 | 32.20 | 8.4 |
| 11 | 16.22 | 33.9 | 34 | 33.20 | 8.8 |
| 12 | 16.46 | 9.1 | 35 | 33.61 | 5.8 |
| 13 | 17.54 | 25.2 | 36 | 34.13 | 4.6 |
| 14 | 18.66 | 45.4 | 37 | 34.72 | 3.8 |
| 15 | 18.96 | 7.6 | 38 | 35.16 | 6.4 |
| 16 | 19.66 | 5.9 | 39 | 35.50 | 5.7 |
| 17 | 19.88 | 11.7 | 40 | 36.16 | 4.8 |
| 18 | 20.38 | 19.7 | 41 | 36.84 | 2.9 |
| 19 | 21.28 | 66.8 | 42 | 37.24 | 2.9 |
| 20 | 21.46 | 30.4 | 43 | 37.58 | 3.2 |
| 21 | 22.74 | 100.0 | 44 | 38.04 | 1.7 |
| 22 | 24.56 | 13.4 | 45 | 38.42 | 2.3 |
| 23 | 24.84 | 17.4 | 46 | 38.86 | 4.1 |

In some other embodiments of the present application, the aforementioned crystal form P belongs to a triclinic crystal system, with a space group being P-1, unit cell parameters being a = 9.407(2)Å, b = 11.531(3)Å, c = 13.574(4)Å, α = 66.982(8)°, β = 75.337(8)° and γ = 68.492(8)°, a volume of a unit cell being V = 1250.4(6)Å^3, and a number of

asymmetric units in a unit cell being Z = 2.

The present application provides a crystal form P of a compound of formula (I) having characteristic diffraction peaks in an X-ray powder diffraction pattern thereof at the following 2θ: 7.12±0.20° and 21.46±0.20°. In some embodiments of the present application, the aforementioned crystal form P has characteristic diffraction peaks in an X-ray powder diffraction pattern at the following 2θ: 7.12±0.20°, 21.28±0.20° and 21.46±0.20°. In some embodiments of the present application, the aforementioned crystal form P has characteristic diffraction peaks in an X-ray powder diffraction pattern at the following 2θ: 7.12±0.20°, 20.38±0.20°, 21.28±0.20°, 21.46±0.20°, 26.72±0.20° and 27.48±0.20°. In some embodiments of the present application, the aforementioned crystal form P has characteristic diffraction peaks in an X-ray powder diffraction pattern at the following 2θ: 7.12±0.20°, 20.38±0.20°, 21.46±0.20°, 26.72±0.20° and 27.48±0.20°. In some embodiments of the present application, the aforementioned crystal form P has characteristic diffraction peaks in an X-ray powder diffraction pattern at the following 2θ: 7.12±0.20°, 12.96±0.20°, 18.66±0.20°, 20.38±0.20°, 21.46±0.20°, 22.74±0.20°, 26.72±0.20°, 27.48±0.20° and 27.82±0.20°. In some embodiments of the present application, the aforementioned crystal form P has characteristic diffraction peaks in an X-ray powder diffraction pattern at the following 2θ: 7.12±0.20°, 12.96±0.20°, 18.66±0.20°, 20.38±0.20°, 21.28±0.20°, 21.46±0.20°, 22.74±0.20°, 24.84±0.20°, 26.72±0.20°, 27.48±0.20° and 27.82±0.20°.

The present application provides a crystal form P of a compound of formula (I) having characteristic diffraction peaks in an X-ray powder diffraction pattern thereof at the following 2θ: 7.12±0.20°, 15.40±0.20°, 21.28±0.20°, 21.46±0.20° and 22.74±0.20°. In some embodiments of the present application, the aforementioned crystal form P has characteristic diffraction peaks in an X-ray powder diffraction pattern at the following 2θ: 7.12±0.20°, 12.96±0.20°, 15.40±0.20°, 21.28±0.20°, 21.46±0.20°, 22.74±0.20° and 27.48±0.20°. In some embodiments of the present application, the aforementioned crystal form P has characteristic diffraction peaks in an X-ray powder diffraction pattern at the following 2θ: 7.12±0.20°, 12.96±0.20°, 15.40±0.20°, 20.38±0.20°, 21.28±0.20°, 21.46±0.20°, 22.74±0.20°, 26.72±0.20° and 27.48±0.20°. In some embodiments of the present application, the aforementioned crystal form P has characteristic diffraction peaks in an X-ray powder diffraction pattern at the following 2θ: 7.12±0.20°, 12.96±0.20°, 15.40±0.20°, 18.66±0.20°, 20.38±0.20°, 21.28±0.20°, 21.46±0.20°, 22.74±0.20°, 26.72±0.20°, 27.48±0.20° and 27.82±0.20°.

The present application provides a crystal form P of a compound of formula (I) having characteristic diffraction peaks in an X-ray powder diffraction pattern thereof at the following 2θ: 7.12±0.20°, 8.74±0.20°, 12.96±0.20°, 15.40±0.20°, 15.92±0.20°, 18.66±0.20°, 20.38±0.20°, 21.28±0.20°, 21.46±0.20°, 22.74±0.20°, 26.72±0.20° and 27.48±0.20°. In some embodiments of the present application, the aforementioned crystal form P has characteristic diffraction peaks in an X-ray powder diffraction pattern at the following 2θ: 7.12±0.20°, 8.74±0.20°, 11.66±0.20°, 12.96±0.20°, 15.40±0.20°, 15.92±0.20°, 16.22±0.20°, 18.66±0.20°, 20.38±0.20°, 21.28±0.20°, 21.46±0.20°, 22.74±0.20°, 26.72±0.20°, 27.48±0.20° and 27.82±0.20°. In some embodiments of the present application, the aforementioned crystal form P has characteristic diffraction peaks in an X-ray powder diffraction pattern at the following 2θ: 7.12±0.20°, 8.74±0.20°, 9.29±0.20°, 11.66±0.20°, 12.96±0.20°, 15.40±0.20°, 15.92±0.20°, 16.22±0.20°, 17.54±0.20°, 18.66±0.20°, 20.38±0.20°, 21.28±0.20°, 21.46±0.20°, 22.74±0.20°, 26.72±0.20°, 27.48±0.20° and 27.82±0.20°.

In some embodiments of the present application, the aforementioned crystal form P has an XRPD pattern as shown in FIG. 59.

In some embodiments of the present application, the aforementioned crystal form P has characteristic diffraction peaks in an XRPD pattern at the following 2θ, as shown in Table 16D.

Table 16D. XRPD pattern data for crystal form P

| No. | 2θ (±0.20°) | Relative intensity (%) | No. | 2θ (±0.20°) | Relative intensity (%) |
|---|---|---|---|---|---|
| 1 | 7.12 | 53.4 | 24 | 25.22 | 17.1 |
| 2 | 8.74 | 9.2 | 25 | 25.96 | 11.1 |
| 3 | 9.29 | 5.8 | 26 | 26.72 | 16.3 |
| 4 | 10.86 | 3.5 | 27 | 27.48 | 56.5 |
| 5 | 11.66 | 8.6 | 28 | 27.82 | 14.0 |
| 6 | 12.96 | 15.5 | 29 | 28.44 | 11.5 |
| 7 | 14.28 | 2.9 | 30 | 29.38 | 5.3 |
| 8 | 14.56 | 4.4 | 31 | 31.12 | 12.2 |
| 9 | 15.40 | 22.4 | 32 | 31.65 | 4.2 |

(continued)

| No. | 2θ (±0.20°) | Relative intensity (%) | No. | 2θ (±0.20°) | Relative intensity (%) |
|---|---|---|---|---|---|
| 10 | 15.92 | 40.9 | 33 | 32.20 | 8.4 |
| 11 | 16.22 | 33.9 | 34 | 33.20 | 8.8 |
| 12 | 16.46 | 9.1 | 35 | 33.61 | 5.8 |
| 13 | 17.54 | 25.2 | 36 | 34.13 | 4.6 |
| 14 | 18.66 | 45.4 | 37 | 34.72 | 3.8 |
| 15 | 18.96 | 7.6 | 38 | 35.16 | 6.4 |
| 16 | 19.66 | 5.9 | 39 | 35.50 | 5.7 |
| 17 | 19.88 | 11.7 | 40 | 36.16 | 4.8 |
| 18 | 20.38 | 19.7 | 41 | 36.84 | 2.9 |
| 19 | 21.28 | 66.8 | 42 | 37.24 | 2.9 |
| 20 | 21.46 | 30.4 | 43 | 37.58 | 3.2 |
| 21 | 22.74 | 100.0 | 44 | 38.04 | 1.7 |
| 22 | 24.56 | 13.4 | 45 | 38.42 | 2.3 |
| 23 | 24.84 | 17.4 | 46 | 38.86 | 4.1 |

The crystal form P of the compound of formula (I) provided in the present application belongs to a triclinic crystal system, with a space group being P-1, unit cell parameters being a = 9.407(2)Å, b = 11.531(3)Å, c = 13.574(4)Å, $\alpha$ = 66.982(8)°, $\beta$ = 75.337(8)° and $\gamma$ = 68.492(8)°, a volume of a unit cell being V = 1250.4(6)Å^3, and a number of asymmetric units in a unit cell being Z = 2.

In another aspect, the present application provides a preparation method for the crystal form P, which comprises: 1) adding a compound of formula (I) to a mixed solvent of MTBE and MeOH; and 2) precipitating a solid and separating to give the crystal form P. In some embodiments of the present application, the crystal form P is prepared by adding the crystal form A of the compound of formula (I) to the mixed solvent of MTBE and MeOH. In some embodiments of the present application, in the preparation method for the crystal form P, the volume ratio of MTBE to MeOH is 3:2. In some embodiments of the present application, in the preparation method for the crystal form P, the mass-to-volume ratio of the compound of formula (I) to MTBE and MeOH is selected from 1 mg:0.01-0.4 mL:0.005-0.3 mL, or from 1 mg:0.02-0.1 mL:0.01-0.1 mL, or from 1 mg:0.04 mL:0.027 mL.

In another aspect, the present application provides a preparation method for the crystal form P, which comprises: 1) dissolving a compound of formula (I) in methanol or acetone; and 2) precipitating a solid and separating to give the crystal form P.

The present application provides a crystal form Q of a compound of formula (I) having characteristic diffraction peaks in an X-ray powder diffraction pattern thereof at the following 2θ: 7.03±0.20°, 8.22±0.20° and 14.10±0.20°. In some embodiments of the present application, the aforementioned crystal form Q has characteristic diffraction peaks in an X-ray powder diffraction pattern at the following 2θ: 7.03±0.20°, 8.22±0.20°, 10.34±0.20°, 14.10±0.20°, 14.66±0.20° and 21.61±0.20°. In some embodiments of the present application, the aforementioned crystal form Q has characteristic diffraction peaks in an X-ray powder diffraction pattern at the following 2θ: 7.03±0.20°, 8.22±0.20°, 8.53±0.20°, 10.34±0.20°, 14.10±0.20°, 14.66±0.20°, 16.47±0.20°, 17.07±0.20° and 21.61±0.20°. In some embodiments of the present application, the aforementioned crystal form Q has characteristic diffraction peaks in an X-ray powder diffraction pattern at the following 2θ: 7.03±0.20°, 8.22±0.20°, 8.53±0.20°, 8.95±0.20°, 10.34±0.20°, 14.10±0.20°, 14.66±0.20°, 15.90±0.20°, 16.47±0.20°, 17.07±0.20°, 19.45±0.20°, 21.61±0.20°, 22.89±0.20° and 23.36±0.20°.

In some embodiments of the present application, the aforementioned crystal form Q has an XRPD pattern as shown in FIG. 47.

In some embodiments of the present application, the aforementioned crystal form Q has characteristic diffraction peaks in an XRPD pattern at the following 2θ, as shown in Table 17.

Table 17. XRPD pattern data for crystal form Q

| No. | 2θ (±0.20°) | Relative intensity (%) | No. | 2θ (±0.20°) | Relative intensity (%) |
|---|---|---|---|---|---|
| 1 | 7.03 | 100.00 | 8 | 15.90 | 12.63 |
| 2 | 8.22 | 92.66 | 9 | 16.47 | 11.22 |
| 3 | 8.53 | 37.31 | 10 | 17.07 | 15.61 |
| 4 | 8.95 | 12.78 | 11 | 19.45 | 13.79 |
| 5 | 10.34 | 36.99 | 12 | 21.61 | 19.89 |
| 6 | 14.10 | 82.75 | 13 | 22.89 | 19.98 |
| 7 | 14.66 | 39.68 | 14 | 23.36 | 15.53 |

In some embodiments of the present application, the aforementioned crystal form Q shows a weight loss of 9.29% in a thermogravimetric analysis curve upon heating to 140.0±3 °C.

In some embodiments of the present application, the aforementioned crystal form Q has a TGA pattern as shown in FIG. 48.

In some embodiments of the present application, the aforementioned crystal form Q has an endothermic peak in a differential scanning calorimetry (DSC) curve at 162.9±3 °C. In some embodiments of the present application, the aforementioned crystal form Q has an endothermic peak in a differential scanning calorimetry (DSC) curve at 235.6±3 °C. In some embodiments of the present application, the aforementioned crystal form Q has an exothermic peak in a differential scanning calorimetry (DSC) curve at 168.5±3 °C. In some embodiments of the present application, the aforementioned crystal form Q has an endothermic peak in a differential scanning calorimetry (DSC) curve at 162.9±3 °C and/or 235.6±3 °C, and/or has an exothermic peak in the DSC curve at 168.5±3 °C.

In some embodiments of the present application, the aforementioned crystal form Q has a DSC pattern as shown in FIG. 49.

In some embodiments of the present application, the aforementioned crystal form Q is a crystal form of a hydrate of the compound of formula (I). In some embodiments of the present application, a ratio of the number of molecules of the compound of formula (I) to water in the crystal form Q is selected from 1:2.5 to 1:3.5, and preferably 1:3.1.

In another aspect, the present application provides a preparation method for the aforementioned crystal form Q, which comprises: 1) dissolving a compound of formula (I) in MeOH, and adding ACN dropwise; and 2) precipitating a solid and separating to give the crystal form Q. In some embodiments of the present application, the crystal form Q is prepared by adding the crystal form A of the compound of formula (I) to MeOH.

The present application provides a crystal form R of a compound of formula (I) having characteristic diffraction peaks in an X-ray powder diffraction pattern thereof at the following 2θ: 6.60±0.20°, 8.55±0.20° and 15.21±0.20°. In some embodiments of the present application, the aforementioned crystal form R has characteristic diffraction peaks in an X-ray powder diffraction pattern at the following 2θ: 6.60±0.20°, 8.55±0.20°, 11.67±0.20°, 15.21±0.20°, 17.60±0.20° and 24.56±0.20°. In some embodiments of the present application, the aforementioned crystal form R has characteristic diffraction peaks in an X-ray powder diffraction pattern at the following 2θ: 6.60±0.20°, 8.55±0.20°, 11.67±0.20°, 15.21±0.20°, 17.12±0.20°, 17.60±0.20°, 2325±0.20°, 24.56±0.20° and 27.31±0.20°.

In some embodiments of the present application, the aforementioned crystal form R has an XRPD pattern as shown in FIG. 50.

In some embodiments of the present application, the aforementioned crystal form R has characteristic diffraction peaks in an XRPD pattern at the following 2θ, as shown in Table 18.

Table 18. XRPD pattern data for crystal form R

| No. | 2θ (±0.20°) | Relative intensity (%) | No. | 2θ (±0.20°) | Relative intensity (%) |
|---|---|---|---|---|---|
| 1 | 6.60 | 63.44 | 6 | 17.12 | 13.85 |
| 2 | 8.55 | 100.00 | 7 | 17.60 | 21.00 |
| 3 | 11.67 | 14.28 | 8 | 23.25 | 9.95 |
| 4 | 13.35 | 4.94 | 9 | 24.56 | 22.29 |
| 5 | 15.21 | 42.43 | 10 | 27.31 | 16.12 |

In another aspect, the present application provides a preparation method for the aforementioned crystal form R, which comprises: heating the aforementioned crystal form O to 120 °C to 180 °C, and cooling to room temperature to give the crystal form R. In some embodiments of the present application, the crystal form R is prepared by heating the crystal form O to 150 °C.

The present application provides a crystal form S of a compound of formula (I) having characteristic diffraction peaks in an X-ray powder diffraction pattern thereof at the following 2θ: 6.15±0.20°, 8.43±0.20°, 21.57±0.20° and 23.90±0.20°. In some embodiments of the present application, the aforementioned crystal form S has characteristic diffraction peaks in an X-ray powder diffraction pattern at the following 2θ: 6.15±0.20°, 8.43±0.20°, 14.94±0.20°, 16.29±0.20°, 16.84±0.20°, 21.57±0.20° and 23.90±0.20°. In some embodiments of the present application, the aforementioned crystal form S has characteristic diffraction peaks in an X-ray powder diffraction pattern at the following 2θ: 6.15±0.20°, 6.57±0.20°, 8.43±0.20°, 11.27±0.20°, 14.94±0.20°, 16.29±0.20°, 16.84±0.20°, 17.71±0.20°, 21.57±0.20° and 23.90±0.20°. In some embodiments of the present application, the aforementioned crystal form S has characteristic diffraction peaks in an X-ray powder diffraction pattern at the following 2θ: 6.15±0.20°, 6.57±0.20°, 8.43±0.20°, 8.83±0.20°, 11.27±0.20°, 13.97±0.20°, 14.23±0.20°, 14.94±0.20°, 16.29±0.20°, 16.84±0.20°, 17.20±0.20°, 17.71±0.20°, 18.48±0.20°, 19.19±0.20°, 20.36±0.20°, 20.74±0.20°, 21.57±0.20°, 22.61±0.20°, 23.02±0.20°, 23.90±0.20°, 26.16±0.20°, 26.67±0.20° and 27.74±0.20°. In some embodiments of the present application, the aforementioned crystal form S has characteristic diffraction peaks in an X-ray powder diffraction pattern at the following 2θ: 6.15±0.20°, 6.57±0.20°, 8.43±0.20°, 8.83±0.20°, 9.90±0.20°, 11.27±0.20°, 11.54±0.20°, 13.13±0.20°, 13.97±020°, 14.23±0.20°, 14.94±0.20°, 16.29±0.20°, 16.84±0.20°, 17.20±0.20°, 17.71±0.20°, 18.48±0.20°, 19.19±0.20°, 20.36±0.20°, 20.74±0.20°, 21.57±0.20°, 22.20±0.20°, 22.61±0.20°, 23.02±0.20°, 23.90±0.20°, 24.92±0.20°, 25.58±0.20°, 26.16±0.20°, 26.67±0.20°, 27.74±0.20°, 28.25±0.20° and 31.28±0.20°.

In some embodiments of the present application, the aforementioned crystal form S has an XRPD pattern as shown in FIG. 51.

In some embodiments of the present application, the aforementioned crystal form S has characteristic diffraction peaks in an XRPD pattern at the following 2θ, as shown in Table 19.

Table 19. XRPD pattern data for crystal form S

| No. | 2θ (±0.20°) | Relative intensity (%) | No. | 2θ (±0.20°) | Relative intensity (%) |
|---|---|---|---|---|---|
| 1 | 6.15 | 99.35 | 18 | 20.36 | 25.95 |
| 2 | 6.57 | 33.84 | 19 | 20.74 | 23.50 |
| 3 | 8.43 | 68.73 | 20 | 21.57 | 82.60 |
| 4 | 8.83 | 25.56 | 21 | 22.20 | 19.53 |
| 5 | 9.90 | 14.81 | 22 | 22.61 | 26.97 |
| 6 | 11.27 | 30.19 | 23 | 23.02 | 26.03 |
| 7 | 11.54 | 15.13 | 24 | 23.90 | 100.00 |
| 8 | 13.13 | 12.48 | 25 | 24.92 | 15.27 |
| 9 | 13.97 | 21.96 | 26 | 25.58 | 16.16 |
| 10 | 14.23 | 32.50 | 27 | 26.16 | 26.24 |
| 11 | 14.94 | 55.37 | 28 | 26.67 | 38.52 |
| 12 | 16.29 | 64.20 | 29 | 27.74 | 26.86 |
| 13 | 16.84 | 44.34 | 30 | 28.25 | 13.63 |
| 14 | 17.20 | 34.86 | 31 | 30.09 | 7.50 |
| 15 | 17.71 | 43.99 | 32 | 31.28 | 10.99 |
| 16 | 18.48 | 21.95 | 33 | 33.80 | 5.61 |
| 17 | 19.19 | 33.14 | | | |

In another aspect, the present application provides a preparation method for the aforementioned crystal form S, which comprises: heating the aforementioned crystal form E to 180 °C to 240 °C to give the crystal form S. In some embodiments of the present application, the crystal form S is prepared by heating to 210 °C.

The present application provides a crystal form T of a compound of formula (I) having characteristic diffraction peaks in an X-ray powder diffraction pattern thereof at the following 2θ: 7.23±0.20°, 8.45±0.20°, 16.18±0.20° and 26.35±0.20°. In some embodiments of the present application, the aforementioned crystal form T has characteristic diffraction peaks in an X-ray powder diffraction pattern at the following 2θ: 7.23±0.20°, 8.45±0.20°, 12.85±0.20°, 16.18±0.20°, 19.41±0.20° and 26.35±0.20°. In some embodiments of the present application, the aforementioned crystal form T has characteristic diffraction peaks in an X-ray powder diffraction pattern at the following 2θ: 7.23±0.20°, 8.45±0.20°, 12.85±0.20°, 16.18±0.20°, 18.46±0.20°, 19.41±0.20°, 19.97±0.20°, 21.46±0.20° and 26.35±0.20°. In some embodiments of the present application, the aforementioned crystal form T has characteristic diffraction peaks in an X-ray powder diffraction pattern at the following 2θ: 7.23±0.20°, 8.45±0.20°, 9.72±0.20°, 12.85±0.20°, 14.41±0.20°, 16.18±0.20°, 18.46±0.20°, 19.41±0.20°, 19.97±0.20°, 21.46±0.20°, 24.95±0.20° and 26.35±0.20°.

In some embodiments of the present application, the aforementioned crystal form T has an XRPD pattern as shown in FIG. 52.

In some embodiments of the present application, the aforementioned crystal form T has characteristic diffraction peaks in an XRPD pattern at the following 2θ, as shown in Table 20.

Table 20. XRPD pattern data for crystal form T

| No. | 2θ (±0.20°) | Relative intensity (%) | No. | 2θ (±0.20°) | Relative intensity (%) |
|---|---|---|---|---|---|
| 1 | 7.23 | 84.39 | 7 | 18.46 | 17.35 |
| 2 | 8.45 | 100.00 | 8 | 19.41 | 21.11 |
| 3 | 9.72 | 6.02 | 9 | 19.97 | 17.27 |
| 4 | 12.85 | 24.26 | 10 | 21.46 | 24.98 |
| 5 | 14.41 | 11.88 | 11 | 24.95 | 11.46 |
| 6 | 16.18 | 26.32 | 12 | 26.35 | 30.07 |

In some embodiments of the present application, the aforementioned crystal form T shows a weight loss of 0.47% in a thermogravimetric analysis curve upon heating to 150.0±3 °C.

In some embodiments of the present application, the aforementioned crystal form T has a TGA pattern as shown in FIG. 53.

In some embodiments of the present application, the aforementioned crystal form T has an endothermic peak in a differential scanning calorimetry (DSC) curve at 235.3±3 °C. In some embodiments of the present application, the aforementioned crystal form T has an exothermic peak in a differential scanning calorimetry (DSC) curve at 178.1±3 °C. In some embodiments of the present application, the aforementioned crystal form T has an endothermic peak in a differential scanning calorimetry (DSC) curve at 235.3±3 °C, and/or has an exothermic peak in the DSC curve at 178.1±3 °C.

In some embodiments of the present application, the aforementioned crystal form T has a DSC pattern as shown in FIG. 54.

In another aspect, the present application provides a preparation method for the aforementioned crystal form T, which comprises: heating the aforementioned crystal form G to 120 °C to 180 °C, and cooling to room temperature to give the crystal form T. In some embodiments of the present application, the crystal form T is prepared by heating the crystal form G to 150 °C.

The present application provides a crystal form U of a compound of formula (I) having characteristic diffraction peaks in an X-ray powder diffraction pattern thereof at the following 2θ: 3.50±0.20°, 6.97±0.20°, 9.51±0.20° and 19.13±0.20°. In some embodiments of the present application, the aforementioned crystal form U has characteristic diffraction peaks in an X-ray powder diffraction pattern at the following 2θ: 3.50±0.20°, 6.97±0.20°, 9.51±0.20°, 11.55±0.20°, 17.53±0.20°, 19.13±0.20° and 19.62±0.20°. In some embodiments of the present application, the aforementioned crystal form U has characteristic diffraction peaks in an X-ray powder diffraction pattern at the following 2θ: 3.50±0.20°, 6.97±0.20°, 9.51±0.20°, 10.29±0.20°, 11.55±0.20°, 14.00±0.20°, 17.53±0.20°, 19.13±0.20°, 19.62±0.20° and 21.09±0.20°. In some embodiments of the present application, the aforementioned crystal form U has characteristic diffraction peaks in an X-ray powder diffraction pattern at the following 2θ: 3.50±0.20°, 6.97±0.20°, 9.51±0.20°, 9.98±0.20°, 10.29±0.20°, 11.55±0.20°, 14.00±0.20°, 17.53±0.20°, 19.13±0.20°, 19.62±0.20°, 20.71±0.20°, 21.09±0.20°, 21.41±0.20°, 22.32±0.20°, 24.35±0.20°, 26.98±0.20° and 35.53±0.20°.

In some embodiments of the present application, the aforementioned crystal form U has an XRPD pattern as shown in FIG. 55.

In some embodiments of the present application, the aforementioned crystal form U has characteristic diffraction

peaks in an XRPD pattern at the following 2θ, as shown in Table 21.

Table 21. XRPD pattern data for crystal form U

| No. | 2θ (±0.20°) | Relative intensity (%) | No. | 2θ (±0.20°) | Relative intensity (%) |
|---|---|---|---|---|---|
| 1 | 3.50 | 19.33 | 11 | 19.62 | 76.64 |
| 2 | 6.97 | 29.95 | 12 | 20.71 | 9.47 |
| 3 | 9.51 | 71.34 | 13 | 21.09 | 24.98 |
| 4 | 9.98 | 20.68 | 14 | 21.41 | 5.57 |
| 5 | 10.29 | 31.90 | 15 | 22.32 | 12.21 |
| 6 | 10.48 | 30.73 | 16 | 24.35 | 8.51 |
| 7 | 11.55 | 31.95 | 17 | 24.88 | 4.04 |
| 8 | 14.00 | 25.34 | 18 | 26.98 | 10.50 |
| 9 | 17.53 | 34.14 | 19 | 32.87 | 4.68 |
| 10 | 19.13 | 100.00 | 20 | 35.53 | 9.18 |

In another aspect, the present application provides a preparation method for the aforementioned crystal form U, which comprises: 1) dissolving the compound of formula (I) in DMAc; 2) adding toluene to the resulting DMAc solution; and 3) precipitating a solid and separating to give the crystal form U. In some embodiments of the present application, the crystal form U is prepared by dissolving the crystal form A of the compound of formula (I) to DMAc. In some embodiments of the present application, in the preparation method for the crystal form U, toluene is added to the DMAc solution by evaporating toluene into the DMAc solution.

The present application provides a crystal form V of a compound of formula (I) having characteristic diffraction peaks in an X-ray powder diffraction pattern thereof at the following 2θ: 7.17±0.20°, 9.44±0.20°, 19.06±0.20° and 19.56±0.20°. In some embodiments of the present application, the aforementioned crystal form V has characteristic diffraction peaks in an X-ray powder diffraction pattern at the following 2θ: 7.17±0.20°, 9.44±0.20°, 10.22±0.20°, 11.48±0.20°, 19.06±0.20°, 19.56±0.20° and 21.35±0.20°. In some embodiments of the present application, the aforementioned crystal form V has characteristic diffraction peaks in an X-ray powder diffraction pattern at the following 2θ: 6.90±0.20°, 7.17±0.20°, 8.99±0.20°, 9.44±0.20°, 9.91±0.20°, 10.22±0.20°, 11.48±0.20°, 19.06±0.20°, 19.56±0.20° and 21.35±0.20°. In some embodiments of the present application, the aforementioned crystal form V has characteristic diffraction peaks in an X-ray powder diffraction pattern at the following 2θ: 6.90±0.20°, 7.17±0.20°, 8.99±0.20°, 9.44±0.20°, 9.91±0.20°, 10.22±0.20°, 11.48±0.20°, 14.50±0.20°, 17.46±0.20°, 19.06±0.20°, 19.56±0.20°, 21.01±0.20°, 21.35±0.20°, 21.85±0.20°, 22.25±0.20° and 24.27±0.20°. In some embodiments of the present application, the aforementioned crystal form V has characteristic diffraction peaks in an X-ray powder diffraction pattern at the following 2θ: 6.90±0.20°, 7.17±0.20°, 8.99±0.20°, 9.44±0.20°, 9.91±0.20°, 10.22±0.20°, 10.41±0.20°, 10.57±0.20°, 11.48±0.20°, 13.93±0.20°, 14.50±0.20°, 17.46±0.20°, 19.06±0.20°, 19.56±0.20°, 20.64±0.20°, 21.01±0.20°, 21.35±0.20°, 21.85±0.20°, 22.25±0.20°, 24.27±0.20°, 26.88±0.20° and 29.30±0.20°.

In some embodiments of the present application, the aforementioned crystal form V has an XRPD pattern as shown in FIG. 56.

In some embodiments of the present application, the aforementioned crystal form V has characteristic diffraction peaks in an XRPD pattern at the following 2θ, as shown in Table 22.

Table 22. XRPD pattern data for crystal form V

| No. | 2θ (±0.20°) | Relative intensity (%) | No. | 2θ (±0.20°) | Relative intensity (%) |
|---|---|---|---|---|---|
| 1 | 3.41 | 8.90 | 17 | 19.56 | 83.13 |
| 2 | 6.90 | 26.31 | 18 | 20.64 | 13.03 |
| 3 | 7.17 | 28.92 | 19 | 21.01 | 18.57 |
| 4 | 8.99 | 31.72 | 20 | 21.35 | 31.16 |
| 5 | 9.44 | 77.32 | 21 | 21.85 | 14.62 |
| 6 | 9.91 | 24.59 | 22 | 22.25 | 24.66 |

(continued)

| No. | 2θ (±0.20°) | Relative intensity (%) | No. | 2θ (±0.20°) | Relative intensity (%) |
|---|---|---|---|---|---|
| 7 | 10.22 | 45.47 | 23 | 22.74 | 5.53 |
| 8 | 10.41 | 27.34 | 24 | 24.27 | 19.54 |
| 9 | 10.57 | 30.39 | 25 | 24.80 | 8.20 |
| 10 | 11.48 | 40.45 | 26 | 25.02 | 5.26 |
| 11 | 13.93 | 19.73 | 27 | 26.88 | 13.28 |
| 12 | 14.50 | 16.03 | 28 | 27.86 | 5.49 |
| 13 | 17.46 | 24.23 | 29 | 29.30 | 11.20 |
| 14 | 17.77 | 7.24 | 30 | 32.79 | 5.60 |
| 15 | 19.06 | 100.00 | 31 | 33.72 | 5.06 |
| 16 | 19.24 | 20.88 | 32 | 35.45 | 6.98 |

In some embodiments of the present application, the aforementioned crystal form V shows a weight loss of 14.05% in a thermogravimetric analysis curve upon heating to 110.0±3 °C. In some embodiments of the present application, the aforementioned crystal form V shows a weight loss of 16.46% in a thermogravimetric analysis (TGA) curve upon heating from 110.0±3 °C to 160.0±3 °C. In some embodiments of the present application, the aforementioned crystal form V shows a weight loss of 14.05% in a thermogravimetric analysis (TGA) curve upon heating to 110.0±3 °C, and/or shows a weight loss of 16.46% in the TGA curve upon heating from 110.0±3 °C to 160.0±3 °C.

In some embodiments of the present application, the aforementioned crystal form V has a TGA pattern as shown in FIG. 57.

In some embodiments of the present application, the aforementioned crystal form V has an endothermic peak in a differential scanning calorimetry (DSC) curve at 231.1±3 °C. In some embodiments of the present application, the aforementioned crystal form V has an endothermic peak in a differential scanning calorimetry (DSC) curve at 153.9±3 °C. In some embodiments of the present application, the aforementioned crystal form V has an endothermic peak in a differential scanning calorimetry (DSC) curve at 231.1±3 °C and/or 153.9±3 °C.

In some embodiments of the present application, the aforementioned crystal form V has a DSC pattern as shown in FIG. 58.

In another aspect, the present application provides a preparation method for the aforementioned crystal form V, which comprises: drying the aforementioned crystal form U under vacuum at 50 °C to give the crystal form V.

In yet another aspect, the present application provides a crystal form composition comprising the aforementioned crystal form, wherein the crystal form accounts for 50% or more, preferably 80% or more, more preferably 90% or more and most preferably 95% or more of the weight of the crystal form composition.

In yet another aspect, the present application provides a pharmaceutical composition comprising a therapeutically effective amount of the crystal form or the crystal form composition thereof disclosed herein. The pharmaceutical composition disclosed herein may or may not contain a pharmaceutically acceptable excipient. In addition, the pharmaceutical composition disclosed herein may further comprise one or more additional therapeutic agents. The present application also provides use of the aforementioned crystal form, the crystal form composition thereof or the pharmaceutical composition thereof for preparing a medicament for inhibiting nucleoprotein.

The present application also provides a method for inhibiting nucleoprotein, which comprises administering to a mammal, preferably a human, in need of such treatment or prevention a therapeutically effective amount of the aforementioned crystal form, the crystal form composition thereof or the pharmaceutical composition thereof. The present application also provides the aforementioned crystal form, the crystal form composition thereof or the pharmaceutical composition thereof for use as a nucleoprotein inhibitor.

The present application also provides use of the aforementioned crystal form, the crystal form composition thereof or the pharmaceutical composition thereof for inhibiting nucleoprotein.

In some embodiments of the present application, the aforementioned use or method is characterized in that the pharmaceutical nucleoprotein inhibitor is a medicament for treating or preventing HBV infection related diseases. The present application also provides use of the aforementioned crystal form, the crystal form composition thereof or the pharmaceutical composition thereof for preparing a medicament for treating or preventing HBV infection related diseases.

The present application also provides use of the aforementioned crystal form, the crystal form composition

thereof or the pharmaceutical composition thereof for treating or preventing HBV infection related diseases.

The present application also provides the aforementioned crystal form, the crystal form composition thereof or the pharmaceutical composition thereof for use in treating or preventing HBV infection related diseases.

The present application also provides use of the aforementioned crystal form, the crystal form composition thereof or the pharmaceutical composition thereof for treating or preventing HBV infection related diseases.

**Technical Effects**

The compound of formula (I) disclosed herein has good *in-vivo* drug administration effect, stable crystal forms, small influence of light, heat and humidity, good solubility, and a wide prospect of druggability . The crystal form disclosed herein demonstrates good pharmacokinetics which can be verified by preclinical (e.g., in SD rats and beagle dogs) and clinical trials, and is suitable for use as a medicament. The crystal form disclosed herein contribute to the solid form of the compound.

**Definitions and Description**

Unless otherwise stated, the following terms and phrases used herein are intended to have the following meanings. A particular phrase or term, unless otherwise specifically defined, should not be considered as uncertain or unclear, but construed according to its common meaning. When referring to a trade name, it is intended to refer to its corresponding commercial product or its active ingredient.

It should be noted that in the X-ray powder diffraction pattern, the position and relative intensity of a peak may vary due to measuring instruments, measuring methods/conditions, and other factors. For any specific crystal form, the position of a peak may have an error, and the measurement of 2θ may have an error of ±0.2°. Therefore, this error should be considered when determining each crystal form, and crystal forms within this margin of error are within the scope of the present application.

The phenomenon that lattice planes or edges of some grains in a crystal are predominantly aligned along certain directions and planes is called preferential orientation. In the powder method, the calculation of the relative intensity of the lines requires that the crystals in the powder sample be in a completely random orientation, and if the number of oriented crystals in the sample is increased, the increase in intensity is necessarily caused. This allows a certain correspondence between the intensity of the diffraction lines and the degree of orientation. (X-ray Structure Analysis, edited by Qi Jingyu, Tongji University Press, April 2003, 1st edition).

The phenomenon that the grains in the sample have a remarkable tendency toward a certain crystallographic direction is called preferential orientation, and the preferential orientation phenomenon can be easily found by visual inspection for a strongly cleaved substance. For such materials, for example, flaky or needle crystals, preferential orientation readily occurs when test samples are made. Like plate crystals, in a cylindrical sample tube, the finding of flaky crystal faces tends to coincide with the axis of the sample tube. In the flat sample holder of the diffractometer, the normal to the flaky crystal face tends to be perpendicular to the basal plane of the sample holder. The diffraction intensity of the preferentially oriented crystal faces is enhanced abnormally when diffraction data are collected using a standard Bragg-Brentano type diffractometer coupled with θ-2θ. Even though some improvement can be made by making samples many times, it is still difficult to completely overcome the preferential orientation phenomenon (Measurement of Crystal Structure by Powder Diffraction Method, edited by Liang Jingkui, Science Press, April 2003, 1st edition).

The preferential orientation influences the measurement result of the crystal structure determined by the powder diffraction method. XRPD measurement results of different batches of the crystal form differ, but this does not prevent those skilled in the art from making a decision as to whether the crystal form is the same.

It should be noted that, for the same crystal form, the position of an endothermic peak in the DSC pattern may vary due to measuring instruments, measuring methods/conditions, and other factors. For any particular crystal form, there may be an error in the position of the endothermic peak, which may be ±5 °C or may be ±3 °C. Therefore, this error should be considered when determining each crystal form, and crystal forms within this margin of error are within the scope of the present application.

The term "solvate" refers to a substance formed by combining the compound of formula (I) with a pharmaceutically acceptable solvent, and water is not included herein. The pharmaceutically acceptable solvent includes ethanol, acetic acid and the like. The solvate includes both stoichiometric and non-stoichiometric amounts of solvates.

The word "comprise" and variations thereof such as "comprises" or "comprising" will be understood in an open, non-exclusive sense, i.e., "including but not limited to".

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio. The term "pharmaceutically acceptable excipient" refers to an

inert substance administered with active ingredient to facilitate administration of the active ingredient, including, but not limited to, any glidant, sweetener, diluent, preservative, dye/coloring agent, flavor enhancer, surfactant, wetting agent, dispersant, disintegrant, suspending agent, stabilizer, isotonizing agent, solvent or emulsifier acceptable for use in humans or animals (e.g., domesticated animals) as permitted by the National Medical Products Administration, PRC. Non-limiting examples of the excipients include calcium carbonate, calcium phosphate, various sugars and types of starch, cellulose derivatives, gelatin, vegetable oils, and polyethylene glycols.

The term "pharmaceutical composition" refers to a mixture consisting of one or more of the compounds or the salts thereof of the present application and a pharmaceutically acceptable excipient. The pharmaceutical composition is intended to facilitate the administration of the compound to an organic entity.

The pharmaceutical composition of the present application can be prepared by combining the compound of the present application with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the crystal forms or the pharmaceutical composition thereof disclosed herein include, but are not limited to, oral, rectal, topical, inhalation, parenteral, sublingual, intravaginal, intranasal, intraocular, intraperitoneal, intramuscular, subcutaneous and intravenous administration.

The pharmaceutical composition of the present application can be manufactured using methods well known in the art, such as conventional mixing, dissolving, granulating, dragee-making, levigating, emulsifying, and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compound of the present application to be formulated into tablets, pills, pastilles, dragees, capsules, liquids, gels, slurries, suspensions, etc. for oral administration to a patient.

Therapeutic dosages of the compounds disclosed herein may be determined by, for example, the specific use of a treatment, the route of administration of the compound, the health and condition of a patient, and the judgment of a prescribing physician. The proportion or concentration of the compound disclosed herein in a pharmaceutical composition may not be constant and depends on a variety of factors including dosages, chemical properties (e.g., hydrophobicity), and routes of administration.

The term "treat" or "treatment" means administering the compound or formulation described in the present application to ameliorate or eliminate a disease or one or more symptoms associated with the disease, and includes:

(i) inhibiting a disease or disease state, i.e., arresting its development; and
(ii) alleviating a disease or disease state, i.e., causing its regression.

The term "prevent" or "prevention" means administering the compound or formulation described herein to prevent a disease or one or more symptoms associated with the disease, and includes: preventing the occurrence of the disease or disease state in a mammal, particularly when such a mammal is predisposed to the disease state but has not yet been diagnosed with it.

For drugs and pharmacological active agents, the term "therapeutically effective amount" refers to an amount of a drug or a medicament that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of routine tests.

The therapeutically effective amount of the crystal forms disclosed herein is from about 0.0001 to 20 mg/kg body weight (bw)/day, for example from 0.001 to 10 mg/kg bw/day.

The dosage frequency of the crystal forms disclosed herein depends on needs of an individual patient, e.g., once or twice daily or more times daily. Administration may be intermittent, for example, in a period of several days, the patient receives a daily dose of the crystal forms, and in the following period of several days or more days, the patient does not receive the daily dose of the crystal forms.

The intermediate compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples disclosed herein.

The chemical reactions of the embodiments disclosed herein are carried out in a proper solvent that must be suitable for the chemical changes in the present application and the reagents and materials required. In order to acquire the compounds disclosed herein, it is sometimes necessary for those skilled in the art to modify or select a synthesis procedure or a reaction scheme based on the existing embodiments.

The present application is described in detail below by way of examples, which are not intended to limit the present application in any way.

All solvents used in the present application are commercially available and can be used without further purification.

The solvents used in the present application are commercially available. The following abbreviations are used in the present application: DCM represents dichloromethane; DMF represents N,N-dimethylformamide; DMSO represents dimethyl sulfoxide; EtOH represents ethanol; MeOH represents methanol; TFA represents trifluoroacetic acid; ATP represents adenosine triphosphate; HEPES represents 4-hydroxyethylpiperazine ethanesulfonic acid; EGTA represents glycol-bis-(2-aminoethylether)tetraacetic acid; $MgCl_2$ represents magnesium dichloride; NMP represents *N*-methylpyrrolidone; THF represents tetrahydrofuran; 2-MeTHF represents 2-methyltetrahydrofuran; MTBE represents methyl *tert*-butyl ether; DMAc represents dimethylacetamide; ACN represents acetonitrile.

Specific methods of XRPD, DSC and TGA (including equipment model and parameters) X-ray powder diffractometer (XRPD) method of the present application

The instrument model is as follows: X'Pert$^3$/Empyrean type of X-ray diffractometer from PANalytical.

The test method is as follows: approximately 10 mg of the sample was used for XRPD detection.

The detailed XRPD parameters are as follows:

Ray source: Cu, K$\alpha$ (K$\alpha$1 = 1.540598 Å, K$\alpha$2 = 1.544426 Å, the strength ratio of K$\alpha$2/K$\alpha$1: 0.5)
Voltage of X-ray tube: 45 kV, current of X-ray tube: 40 mA
Divergent slit: fixed 1/8 deg
First Soller slit: 0.04 rad, second Soller slit: 0.04 rad
Receiving slit: none, anti-scatter-slit: 7.5 mm
Measurement time: 5 min
Scanning angle range: 3-40 deg
Step width angle: 0.0263 deg (X'Pert$^3$)/0.0167 deg (Empyrean)
Step length: 46.665 seconds (X'Pert$^3$)/17.780 seconds (Empyrean)
Rotation speed of sample plate: 15 rpm

**Differential scanning calorimetry (DSC) method of the present application**

The instrument model is as follows: TA Q2000/Discovery DSC 2500 differential scanning calorimeter

The test method is as follows: a sample (1-5 mg) was placed in a DSC aluminum pan for testing and then heated from 25 °C (room temperature) to a temperature at which the sample would be decomposed at a heating rate of 10 °C/min at 50 mL/min under $N_2$ atmosphere.

**Thermal gravimetric analyzer (TGA) method of the present application**

The instrument model is as follows: TA Discovery TGA 5500 thermogravimetric analyzer

The test method is as follows: a sample (1-5 mg) was placed in a TGA aluminum pan for testing and then heated from room temperature to 350 °C at a heating rate of 10 °C/min at 10 mL/min under $N_2$ atmosphere.

**Single crystal detection method of the present invention**

Diffraction intensity data were collected using a Bruker D8 venture diffractometer with Cuk$\alpha$ radiation as the light source and $\varphi/\omega$ scanning as the scanning mode.

**BRIEF DESCRIPTION OF THE DRAWINGS**

FIG. 1: an XRPD pattern of the crystal form A;
FIG. 2: a TGA pattern of the crystal form A;
FIG. 3: a DSC pattern of the crystal form A;
FIG. 4: an XRPD pattern of the crystal form B;
FIG. 5: a TGA pattern of the crystal form B;
FIG. 6: a DSC pattern of the crystal form B;
FIG. 7: an XRPD pattern of the crystal form C;
FIG. 8: a TGA pattern of the crystal form C;

FIG. 9: a DSC pattern of the crystal form C;
FIG. 10: an XRPD pattern of the crystal form D;
FIG. 11: an XRPD pattern of the crystal form E;
FIG. 12: a TGA pattern of the crystal form E;
FIG. 13: a DSC pattern of the crystal form E;
FIG. 14: an XRPD pattern of the crystal form F;
FIG. 15: a TGA pattern of the crystal form F;
FIG. 16: a DSC pattern of the crystal form F;
FIG. 17: an XRPD pattern of the crystal form G;
FIG. 18: a TGA pattern of the crystal form G;
FIG. 19: a DSC pattern of the crystal form G;
FIG. 20: an XRPD pattern of the crystal form H;
FIG. 21: a TGA pattern of the crystal form H;
FIG. 22: a DSC pattern of the crystal form H;
FIG. 23: an XRPD pattern of the crystal form I;
FIG. 24: a TGA pattern of the crystal form I;
FIG. 25: a DSC pattern of the crystal form I;
FIG. 26: an XRPD pattern of the crystal form J;
FIG. 27: a TGA pattern of the crystal form J;
FIG. 28: a DSC pattern of the crystal form J;
FIG. 29: an XRPD pattern of the crystal form K;
FIG. 30: a TGA pattern of the crystal form K;
FIG. 31: a DSC pattern of the crystal form K;
FIG. 32: an XRPD pattern of the crystal form L;
FIG. 33: a TGA pattern of the crystal form L;
FIG. 34: a DSC pattern of the crystal form L;
FIG. 35: an XRPD pattern of the crystal form M;
FIG. 36: a TGA pattern of the crystal form M;
FIG. 37: a DSC pattern of the crystal form M;
FIG. 38: an XRPD pattern of the crystal form N;
FIG. 39: a TGA pattern of the crystal form N;
FIG. 40: a DSC pattern of the crystal form N;
FIG. 41: an XRPD pattern of the crystal form O;
FIG. 42: a TGA pattern of the crystal form O;
FIG. 43: a DSC pattern of the crystal form O;
FIG. 44A: an XRPD pattern 1 of the crystal form P;
FIG. 44B: an XRPD pattern 2 of the crystal form P;
FIG. 45: a TGA pattern 1 of the crystal form P;
FIG. 46: a DSC pattern 1 of the crystal form P;
FIG. 47: an XRPD pattern of the crystal form Q;
FIG. 48: a TGA pattern of the crystal form Q;
FIG. 49: a DSC pattern of the crystal form Q;
FIG. 50: an XRPD pattern of the crystal form R;
FIG. 51: an XRPD pattern of the crystal form S;
FIG. 52: an XRPD pattern of the crystal form T;
FIG. 53: a TGA pattern of the crystal form T;
FIG. 54: a DSC pattern of the crystal form T;
FIG. 55: an XRPD pattern of the crystal form U;
FIG. 56: an XRPD pattern of the crystal form V;
FIG. 57: a TGA pattern of the crystal form V;
FIG. 58: a DSC pattern of the crystal form V;
FIG. 59: an XRPD pattern 3 of the crystal form P;
FIG. 60: a DSC pattern 2 of the crystal form P;
FIG. 61: an XRPD pattern of the crystal form P obtained by calculation through single crystal data;
FIG. 62: a polarizing microscope (PLM) picture of the crystal form P; and
FIG. 63: a stacking diagram of FIG. 44B (upper), FIG. 59 (middle) and FIG. 61 (lower).

## DETAILED DESCRIPTION

In order to better understand the content of the present application, further description is given with reference to specific examples, but the specific embodiments are not intended to limit the content of the present application.

**Example 1:** Compound of Formula (I)

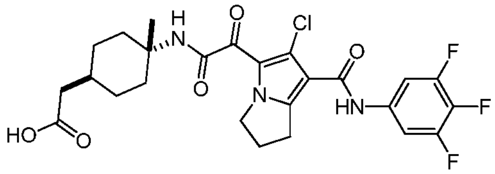

(I )

Synthetic route:

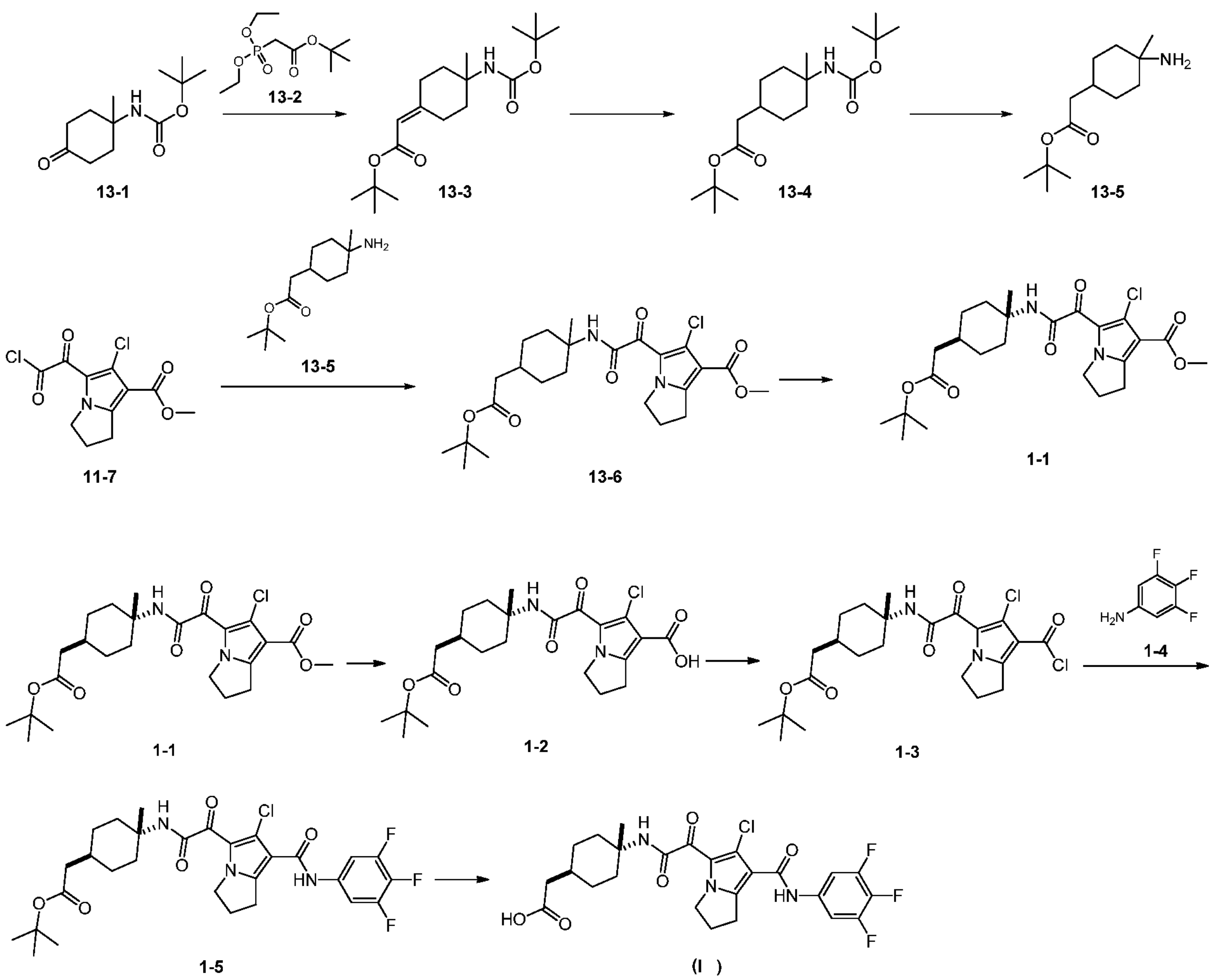


13-1 13-3 13-4 13-5 11-7 13-6 1-1 1-1 1-2 1-3 1-5 (I )

Step 1.

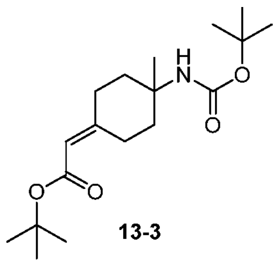

13-3

Compound **13-1** (1 g, 4.40 mmol, 1 eq.) was dissolved in tetrahydrofuran (20 mL) in a single-neck flask, and sodium hydride (615.93 mg, purity: 60%, 3.5 eq.) was added at 0 °C under nitrogen atmosphere. After stirring for 0.5 h, compound **13-2** (2.33 g, 9.24 mmol, 2.1 eq.) was added, and the reaction system was stirred for 30 min at 30 °C. After the reaction was completed as indicated by TLC, the reaction system was poured into 0.5 M diluted hydrochloric acid (100 mL), and the crude product was separated and purified by column chromatography (petroleum ether:ethyl acetate = 50:1 to 20:1) to give compound **13-3.**

$^1$H NMR (400MHz, $CDCl_3$) $\delta$ = 5.55 (s, 1H), 4.43 (s, 1H), 3.32 - 3.26 (m, 1H), 2.41 - 2.38 (m, 1H), 2.30 - 2.28 (m, 1H), 2.15 - 2.14 (m, 1H), 2.13 - 2.04 (m, 1H), 2.03 - 2.01 (m, 1H), 1.54 - 1.49 (m, 2H), 1.46 (s, 9H), 1.43 (s, 9H), 1.33 (s, 3H).

Step 2.

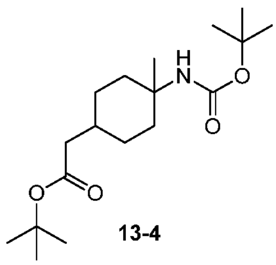

13-4

Wet palladium on carbon (200 mg, content: 10%) was dissolved in tetrahydrofuran (40 mL) in a hydrogenation flask, and compound **13-3** (1.35 g, 4.15 mmol, 1 eq.) was added. The reaction system was stirred at 25 °C for 2 h under $H_2$ atmosphere (15 psi). After the reaction was completed as indicated by TLC, the reaction solution was filtered and concentrated to give compound **13-4.**

Step 3.

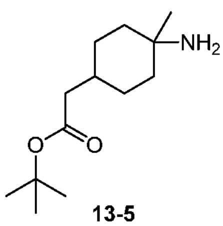

13-5

Compound **13-4** (1.3 g, 3.97 mmol, 1 eq.) was dissolved in ethyl acetate (30 mL) in a dry single-neck flask, and hydrochloric acid/ethyl acetate (4 M, 10 mL, 10.08 eq.) was added. The reaction system was stirred at 30 °C for 16 h. The reaction solution was directly concentrated to give compound **13-5.**

$^1$H NMR (400MHz, MeOH-*d4*) $\delta$ = 1.85 - 1.83 (m, 4H), 1.77 - 1.76 (m, 2H), 1.63 - 1.60 (m, 3H), 1.45 (s, 9H), 1.35 - 1.31 (m, 5H).

Step 4.

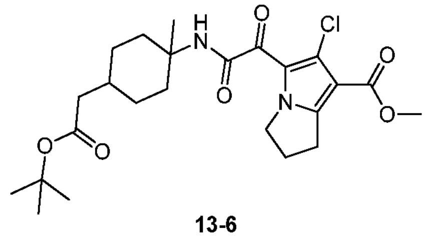

**13-6**

Compound **13-5** (250 mg, 861.77 μmol, 1 eq.) was dissolved in dichloromethane (10 mL) in a dry single-neck flask, and triethylamine (261.61 mg, 2.59 mmol, 359.85 μL, 3 eq.) was added. The resulting mixture was added with a solution of compound **11-7** (293.88 mg, 1.29 mmol, 1.5 eq.) in dichloromethane (10 mL) dropwise under nitrogen atmosphere at 0 °C. The reaction system was stirred at 30 °C for 1 h. After the reaction was completed as indicated by LCMS, the reaction solution was poured into water (50 mL). Then the aqueous phase was extracted with dichloromethane (20 mL × 3), and the organic phase was washed with 0.5 M diluted hydrochloric acid (20 mL × 3), dried over anhydrous sodium sulfate, filtered and concentrated under reduced pressure to give compound **13-6.**

MS(ESI) m/s :425.2 [M+H-(*t*-Bu)]$^+$.

$^1$H NMR (400MHz, $CDCl_3$) $\delta$ = 5.99 (s, 1H), 4.32 (t, *J* = 7.6 Hz, 2H), 3.84 (s, 2H), 3.14 (t, *J* = 7.6 Hz, 2H), 2.52 - 2.48 (m, 2H), 2.31 - 2.28 (m, 2H), 2.14 - 2.12 (m, 2H), 1.68 - 1.65 (m, 2H), 1.45 - 1.42 (m, 2H), 1.39 - 1.36 (m, 12H), 1.24 - 1.22 (m, 2H), 1.18 - 1.14 (m, 2H).

Compound **13-6** was separated by SFC (column: DAICELCHIRALCELOJ (250 mm × 30 mm, 10 μm); mobile phase: Neu-ETOH; B%: 30%-30%, 9 min) to give compound **1-1** (SFC retention time: 1.7 min), SFC analysis conditions (column: Daicel OJ-3 chiral column, with a specification of 0.46 cm id × 5 cm; mobile phase: A: carbon dioxide, B: ethanol for chromatography (containing 0.05% isopropylamine); B%: 5%-40%; flow rate: 4 mL/min; 4 min; system back pressure: 100 bar)).

Compound **1-1:** $^1$H NMR (400MHz, $CDCl_3$) $\delta$ = 6.08 (s, 1H), 4.31 (t, *J*=7.4 Hz, 2H), 3.83 (s, 3H), 3.12 (t, *J*=7.6 Hz, 2H), 2.51 - 2.47 (m, 2H), 2.17 - 2.15 (m, 2H), 2.14 - 2.00 (m, 2H), 1.83 - 1.70 (m, 6H), 1.48 (s, 3H), 1.47 - 1.45 (m, 11H), 1.45 - 1.26 (m, 3H).

Step 5.

Compound **1-1** (550.00 mg, 1.14 mmol, 1 eq.) was dissolved in tetrahydrofuran (10 mL) in a single-neck flask, and water (10 mL) and methanol (10 mL) and lithium hydroxide monohydrate (239.91 mg, 5.72 mmol, 5 eq.) were added. The reaction system was stirred at 30 °C for 16 h. After the reaction was completed as indicated by LCMS, the reaction solution was adjusted to pH = 1 with 0.5 M diluted hydrochloric acid, the aqueous phase was extracted with ethyl acetate (20 mL × 3), and the organic phase was dried over anhydrous sodium sulfate, filtered and concentrated under reduced pressure to give compound **1-2.**

Step 6.

Compound **1-2** (500 mg, 1.07 mmol, 1 eq.) was dissolved in dichloromethane (20 mL), and oxalyl chloride (271.83 mg, 2.14 mmol, 187.47 μL, 2 eq.) and *N,N*-dimethylformamide (7.83 mg, 107.08 μmol, 8.24 μL, 0.1 eq.) were added dropwise at 0 °C under nitrogen atmosphere. The reaction system was stirred at 25 °C for 1 h. Two drops of the reaction solution were taken and added with methanol to quench the reaction, and after the reaction was completed as indicated by LCMS, the reaction solution was concentrated under reduced pressure to give compound **1-3.**

Step 7.

Compound **1-3** was dissolved in dichloromethane (15 mL), TEA (312.70 mg, 3.09 mmol, 430.12 μL, 3 eq.) was added, and then compound **1-4** (500.00 mg, 1.03 mmol, 1 eq.) was added dropwise into the resulting dichloromethane (5 mL) solution at 0 °C under nitrogen atmosphere. The reaction system was stirred at 25 °C for 1 h. After the reaction was completed as indicated by LCMS, the reaction solution was poured into 0.5 mol/L diluted hydrochloric acid (50 mL), the aqueous phase was extracted with dichloromethane (20 mL × 3), and the organic phase was dried over anhydrous sodium sulfate, filtered and concentrated under reduced pressure. The crude product was separated and purified by

column chromatography (100-200 mesh silica gel, petroleum ether:ethyl acetate = 10:1 to 4:1, V/V) to give compound **1-5.** MS(ESI) m/s :540.1 [M+H-(*t*-Bu)]$^+$.

$^1$H NMR (400MHz, $CDCl_3$) $\delta$ = 8.47 (s, 1H), 7.38 - 7.34 (m, 2H), 6.23 (s, 1H), 4.35 - 4.31 (m, 2H), 3.28 - 3.23 (m, 2H), 2.52 - 2.49 (m, 2H), 2.18 - 2.14 (m, 2H), 2.05 - 2.00 (m, 2H), 1.93 - 1.81(m, 3H), 1.80 - 1.71 (m, 2H), 1.55 (s, 3H), 1.50 - 1.45 (m, 9H), 1.32 - 1.26 (m, 2H).

Step 8.

Compound **1-5** (100.00 mg, 167.78 μmol, 1 eq.) was dissolved in dichloromethane (1 mL), and trifluoroacetic acid (382.59 mg, 3.36 mmol, 248.44 μL, 20 eq.) was added. The reaction system was stirred at 25 °C for 1 h. After the reaction was completed as indicated by LCMS and HPLC, the reaction solution was concentrated under reduced pressure. The crude product was separated and purified by prep-HPLC (neutral system, column: Xtimate C18 150 mm × 25 mm × 5 μm; mobile phase: water (containing 10 mM ammonium bicarbonate)-acetonitrile; acetonitrile%: 10%-50%, 20 min) to give the compound of formula (I). MS(ESI) m/s :540.2 [M+H]$^+$.

$^1$H NMR (400MHz, DMSO-$d_6$) $\delta$ = 9.99 (s, 1H), 8.29 (s, 1H), 7.65 - 7.57 (m, 2H), 4.30 - 4.26 (m, 2H), 3.09 - 3.05 (m, 2H), 2.47 - 2.43 (m, 2H), 2.16 - 2.12 (m, 2H), 1.85 - 1.80 (m, 2H), 1.75 - 1.61 (m, 5H), 1.35 (s, 3H), 1.21 - 1.16(m, 2H).

**Example 2: Preparation Method for Crystal Form**

The preparation method for the crystal form A was as follows: 7.5 g of compound **1-5** was dissolved in dichloromethane (80 mL), and then trifluoroacetic acid (40 mL) was added. The reaction system was stirred at 15 °C for 1 h. After the reaction was completed as indicated by HPLC, the reaction solution was directly concentrated under reduced pressure. The crude product was purified by slurrying with methyl *tert*-butyl ether (100 mL), filtered and concentrated, and lyophilized to give the product in the form of a white solid which was separated and subjected to XRPD analysis to give the crystal form A.

The preparation method for the crystal form B was as follows: about 15 mg of the crystal form A sample was dissolved in 0.04 mL of DMSO, and water was added dropwise until a solid was precipitated. The solid was suspended and stirred overnight, and then separated and subjected to XRPD analysis to give the crystal form B.

The preparation method for the crystal form C was as follows: about 15 mg of the crystal form A sample was dissolved in 0.18 mL of THF, and MTBE was added dropwise until a solid was precipitated. The solid was suspended and stirred overnight, and then separated and subjected to XRPD analysis to give the crystal form C. The preparation method for the crystal form D was as follows: about 15 mg of the crystal form A sample was dissolved in 0.13 mL of THF, and DCM was added dropwise until a solid is precipitated. The solid was suspended and stirred overnight, and then separated and subjected to XRPD analysis to give the crystal form D.

The preparation method for the crystal form E was as follows: about 15 mg of the crystal form A sample was dissolved in 0.6 mL of 1,4-dioxane, the above solution was placed in a 3 mL glass bottle, the 3 mL glass bottle containing the above solution was placed in a 20 mL glass bottle containing 3 mL of ACN (acetonitrile) to enable ACN (acetonitrile) to be slowly evaporated into the 1,4-dioxane solution to give a solid in the 1,4-dioxane solution, and the solid was then separated and subjected to XRPD analysis to give the crystal form E.

The preparation method for the crystal form F was as follows: about 15 mg of the crystal form A sample was dissolved in 0.3 mL of DMF to give a clear solution which was then added dropwise into 3 mL of $H_2O$. The reaction system was suspended and stirred overnight at room temperature, and the solid was separated and subjected to XRPD analysis to give the crystal form F.

The preparation method for the crystal form G was as follows: about 15 mg of the crystal form A sample was dispersed in 0.1 mL of $CHCl_3$/THF (9:1, v/v), suspended and stirred at room temperature for about 2 weeks, and then separated and subjected to XRPD analysis to give the crystal form G.

The preparation method for the crystal form H was as follows: about 15 mg of the crystal form A sample was dispersed in 0.1 mL of EtOH/DMF (19:1, v/v), suspended and stirred at room temperature for about 2 weeks, and then separated and subjected to XRPD analysis to give the crystal form H.

The preparation method for the crystal form I was as follows: about 15 mg of the crystal form A sample was dispersed in 0.1 mL of water, suspended and stirred at 50 °C for about 2 weeks, and then separated and subjected to XRPD analysis to give the crystal form I.

The preparation method for the crystal form J was as follows: about 15 mg of the crystal form A sample was dispersed in 0.1 mL of 2-MeTHF, suspended and stirred at 50 °C for about 2 weeks, and then separated and subjected to XRPD analysis to give the crystal form J.

The preparation method for the crystal form K was as follows: about 15 mg of the crystal form A sample was dissolved in 0.2 mL of DCM/MeOH (4:1, v/v) at 50 °C, directly cooled to 5 °C, and then separated and subjected to

XRPD analysis to give the crystal form K.

The preparation method for the crystal form L was as follows: about 15 mg of the crystal form A sample was dissolved in 0.1 mL of NMP (*N*-methylpyrrolidone), the above solution was placed in a 3 mL glass bottle, the 3 mL glass bottle containing the above solution was placed in a 20 mL glass bottle containing 3 mL of EtOAc (ethyl acetate) to enable EtOAc (ethyl acetate) to be slowly evaporated into the NMP (*N*-methylpyrrolidone) solution to give a solid in the NMP (*N*-methylpyrrolidone) solution, and the solid was separated and subjected to XRPD analysis to give the crystal form L.

The preparation method for the crystal form M was as follows: about 15 mg of the crystal form A sample was dissolved in 0.4 mL of THF and slowly evaporated at room temperature to give a solid, and the solid was separated and subjected to XRPD analysis to give the crystal form M.

The preparation method for the crystal form N was as follows: about 15 mg of the crystal form A sample was dissolved in 1.5 mL of EtOH at 50 °C, directly cooled to 5 °C, and then separated and subjected to XRPD analysis to give the crystal form N.

The preparation method for the crystal form O was as follows: the crystal form D was dried for about 1 h at room temperature under vacuum, and then subjected to XRPD analysis to give the crystal form O.

Preparation method 1 for the crystal form P was as follows: about 15 mg of the crystal form A sample was dissolved in 1.0 mL of MTBE/MeOH (3:2, v/v) and slowly evaporated at room temperature to give a solid, and the solid was separated and subjected to XRPD analysis to give the crystal form P. The XRPD measurement results of the resulting crystal form P are shown in FIG. 44A and FIG. 44B, and the TGA and DSC measurement results are shown in FIG. 45 and FIG. 46, respectively.

The preparation method for the crystal form Q was as follows: about 15 mg of the crystal form A sample was dissolved in 1.5 mL of MeOH, about 3 mL of ACN was added dropwise to give a clear solution, and the clear solution was suspended and stirred at -20 °C. The solid was separated and subjected to XRPD analysis to give the crystal form Q.

The preparation method for the crystal form R was as follows: the crystal form O was heated to 150 °C, then cooled to room temperature, and then subjected to XRPD analysis to give the crystal form R.

The preparation method for the crystal form S was as follows: the crystal form E was heated to 210 °C, and then subjected to in-situ XRPD analysis to give the crystal form S.

The preparation method for the crystal form T was as follows: the crystal form G was heated to 150 °C, and then cooled to room temperature to give the crystal form T.

The preparation method for the crystal form U was as follows: about 15 mg of the crystal form A sample was dissolved in 0.7 mL of DMAc, toluene was slowly evaporated into the DMAc solution at room temperature to give a clear solution, and the clear solution was evaporated at room temperature, and then separated and subjected to XRPD analysis to give the crystal form U.

The preparation method for the crystal form V was as follows: the crystal form U was dried for 2-3 h at 50 °C under vacuum, and then subjected to XRPD analysis to give the crystal form V.

**Example 3: Preparation Method 2 for Crystal Form P**

Acetone (10.06 L) was added into a reaction kettle, the temperature was controlled at 10-25 °C, and then the crystal form A sample (1342.02 g, 2.49 mol) was added. The reaction system was then stirred at 40 °C for 16-24 h. The reaction was stopped, the reaction solution was directly filtered, and the filter cake was concentrated under reduced pressure at 45 °C to give the compound of formula (I) (1142.51 g, crude). The above procedure was repeated to homogenize the sample twice, the reaction solution was directly filtered, and the filter cake was concentrated under reduced pressure at 45 °C to give the compound of formula (I) (989.01 g, 73.44% yield). The sample was taken and subjected to XRPD analysis, the results are shown in FIG. 59, and the DSC measurement results are shown in FIG. 60.

**Example 4: Single Crystal Preparation Method for Crystal Form P**

The compound of formula (I) was dissolved in methanol and then incubated at room temperature for 10 days by solvent evaporation.

The unit cell parameters, crystallographic data and atomic coordinates and the like of a single crystal of the crystal form P of the compound of formula (I) are shown in Tables 23 and 24 below.

Table 23. Crystallographic data and structure refinement

| | |
|---|---|
| Experimental molecular formula | $C_{25}H_{25}ClF_3N_3O_5$ |
| Molecular weight | 539.93 |

(continued)

| | |
|---|---|
| Experimental molecular formula | $C_{25}H_{25}ClF_3N_3O_5$ |
| Temperature | 295(2) K |
| Wavelength | 1.54178 A |
| Crystal system | Triclinic crystal system |
| Space group | P-1 |
| Unit cell parameters | a = 9.407(2) A<br>b = 11.531(3) Å<br>c = 13.574(4) Å<br>α = 66.982(8) °<br>β = 75.337(8) °<br>γ = 68.492(8) ° |
| Volume of unit cell | 1250.4(6) $Å^3$ |
| Z | 2 |
| Calculating density | 1.434 $Mg/m^3$ |
| Absorption correction parameter | 1.925 $mm^{-1}$ |
| F(000) | 560 |
| Size of crystal | 0.220 ×0.180 × 0.150 mm |
| Angle range for data collection | 4.368 degrees to 66.594 degrees |
| Collection range for hkl | -11 <=h<= 11, -13<=k<= 13, -16<=1<= 16 |
| Reflection data collection/independence | 18006 / 4315 [R(int) = 0.0827] |
| Data integrity for theta = 66.66 | 97.4 % |
| Absorption correction | From equivalent semi-experience |
| Maximum and minimum transmission | 0.7531 and 0.6141 |
| Refinement method | F2 full matrix least square method |
| Number of data/number of usage restrictions/number of parameters | 4315/0/334 |
| Degree of fitting of $F_2$ | 1.951 |
| Final R index [I>2sigma(I)] | R1 = 0.0687, wR2 =0.1999 |
| R index (all data) | R1 =0.2322, wR2 =0.2821 |
| Extinction coefficient | n/a |
| Maximum difference (peak top and valley) | 1.119 and -1.571 e.A^-3 |

Table 24. Atomic coordinate (× $10^4$) and equivalent isotropic displacement parameters (Å2×$10^3$)

| | x | y | z | U(eq) |
|---|---|---|---|---|
| Cl(1) | 3668(1) | 4061(1) | 5464(1) | 54(1) |
| F(1) | 5177(4) | -811(4) | 3807(4) | 111(1) |
| F(2) | 3136(4) | -790(4) | 2711(3) | 103(1) |
| F(3) | 885(4) | 1471(4) | 1938(3) | 98(1) |
| O(1) | 8263(5) | -175(5) | 10621(4) | 101(2) |
| O(2) | 8996(5) | 1614(5) | 10024(4) | 106(2) |
| O(3) | 4357(3) | 6198(4) | 6898(2) | 69(1) |
| O(4) | 2029(5) | 8251(4) | 5707(3) | 89(1) |
| O(5) | 261(3) | 5018(3) | 3128(2) | 64(1) |

(continued)

| | x | y | z | U(eq) |
|---|---|---|---|---|
| N(1) | 2472(3) | 5198(3) | 7459(2) | 50(1) |
| N(2) | 786(3) | 7559(3) | 4396(2) | 48(1) |
| N(3) | 2618(3) | 3658(4) | 3581(3) | 55(1) |
| C(1) | 3034(4) | 4130(5) | 8462(3) | 56(1) |
| C(2) | 4581(6) | 3212(6) | 8146(3) | 82(2) |
| C(3) | 5392(7) | 2176(6) | 9124(4) | 87(2) |
| C(4) | 5584(4) | 2857(5) | 9832(3) | 60(1) |
| C(5) | 4032(4) | 3694(5) | 10193(3) | 62(1) |
| C(6) | 3228(4) | 4756(5) | 9223(3) | 53(1) |
| C(7) | 1787(7) | 3447(7) | 8968(4) | 95(2) |
| C(8) | 6404(5) | 1802(6) | 10816(3) | 78(2) |
| C(9) | 7968(6) | 1008(6) | 10475(3) | 73(2) |
| C(10) | 3168(4) | 6069(5) | 6781(3) | 51(1) |
| C(11) | 2340(4) | 7095(5) | 5805(3) | 56(1) |
| C(12) | 1896(3) | 6658(4) | 5080(2) | 47(1) |
| C(13) | 2329(3) | 5496(4) | 4843(2) | 42(1) |
| C(14) | 1498(3) | 5674(4) | 4032(2) | 47(1) |
| C(15) | 549(4) | 6971(4) | 3800(3) | 48(1) |
| C(16) | -686(5) | 7898(5) | 3106(3) | 64(1) |
| C(17) | -1022(7) | 9185(6) | 3308(5) | 96(2) |
| C(18) | -205(4) | 8923(5) | 4253(3) | 58(1) |
| C(19) | 1405(3) | 4766(4) | 3538(2) | 47(1) |
| C(20) | 2720(4) | 2578(5) | 3305(3) | 54(1) |
| C(21) | 1689(4) | 2592(5) | 2718(3) | 56(1) |
| C(22) | 1870(5) | 1469(6) | 2524(3) | 67(1) |
| C(23) | 3013(6) | 313(7) | 2887(4) | 76(2) |
| C(24) | 4044(5) | 323(7) | 3451(4) | 79(2) |
| C(25) | 3928(5) | 1423(6) | 3654(4) | 67(1) |
| H(2A) | 8688 | 2331 | 10118 | 159 |
| H(1A) | 1611 | 5252 | 7309 | 59 |
| H(3A) | 3419 | 3615 | 3806 | 66 |
| H(2B) | 5250 | 3738 | 7684 | 98 |
| H(2C) | 4417 | 2759 | 7734 | 98 |
| H(3B) | 4788 | 1578 | 9547 | 105 |
| H(3C) | 6395 | 1663 | 8875 | 105 |
| H(4A) | 6227 | 3433 | 9402 | 72 |
| H(5A) | 4164 | 4117 | 10642 | 74 |
| H(5B) | 3388 | 3132 | 10625 | 74 |
| H(6A) | 3831 | 5361 | 8825 | 63 |
| H(6B) | 2223 | 5258 | 9482 | 63 |
| H(7A) | 2082 | 2743 | 9622 | 142 |
| H(7B) | 834 | 4074 | 9127 | 142 |
| H(7C) | 1658 | 3095 | 8473 | 142 |
| H(8A) | 6490 | 2240 | 11270 | 94 |
| H(8B) | 5784 | 1217 | 11239 | 94 |
| H(16A) | -1593 | 7600 | 3323 | 77 |
| H(16B) | -324 | 7992 | 2352 | 77 |
| H(17A) | -2124 | 9556 | 3475 | 115 |
| H(17B) | -662 | 9816 | 2665 | 115 |

(continued)

| | x | y | z | U(eq) |
|---|---|---|---|---|
| H(18A) | 396 | 9523 | 4069 | 70 |
| H(18B) | -931 | 8989 | 4894 | 70 |
| H(21A) | 892 | 3354 | 2464 | 67 |
| H(25A) | 4648 | 1406 | 4023 | 81 |

The XRPD pattern obtained by calculation through software simulation based on single crystal data is shown in FIG. 61.

Experimental Example 1: Study on Solid Stability of Crystal Form P

In order to evaluate the solid stability of the crystal form P, the crystal form P was investigated for influence factors (high temperature, high humidity and light), stability under accelerated conditions and stability under intermediate conditions. The crystal form P was placed at high temperature (60 °C, closed), high humidity (room temperature, 92.5% RH, sealing film wrapping and pricking 5-10 small holes) for 5 days and 10 days, then placed under visible light and ultraviolet light (a light-shielding control group was wrapped by tinfoil paper) according to ICH conditions (visible light illumination reached 1.2E+06 Lux hrs, ultraviolet light illumination reached 200 W.hrs/m) in a closed manner, and meanwhile, placed with stability under accelerated conditions (60 °C/75% RH, sealing film wrapping and pricking 5-10 small holes) for 10 days and 1-2 months. XRPD and HPLC characterizations were carried out on the placed sample so as to detect the change of crystal form and purity; the results of Table 25 show that the crystal form of the crystal form P was unchanged under all stability conditions.

Table 25. Evaluation results of solid stability of the crystal form P

| **Conditions** | **Point taking conditions** | **Crystal form change** | **Relative purity* (%)** |
|---|---|---|---|
| Starting crystal form P | / | Unchanged | - |
| 60°C | Day 5 | Unchanged | 100.0 |
| | Day 10 | Unchanged | 100.0 |
| 92.5%RH | Day 5 | Unchanged | 100.0 |
| | Day 10 | Unchanged | 100.0 |
| Visible light # | The illumination reaches 1.2E+06 Lux·hrs | Unchanged | 100.0 |
| Light-shielding control group | Taking points simultaneously with the visible light group | Unchanged | 99.9 |
| Visible + ultraviolet # | The illumination reaches 200 W·hrs/$m^2$ | Unchanged | 100.0 |
| Light-shielding control group | Taking points simultaneously with the visible light + ultraviolet groups | Unchanged | 100.0 |
| 60 °C/75%RH | Day 10 | Unchanged | 100.0 |
| | 1 month | Unchanged | 100.0 |
| | Month 2 | Unchanged | 100.0 |
| Note: "#" indicates ICH conditions. * Relative purity = ratio of stability sample purity to starting sample purity. The aforementioned results indicate that the crystal form P has good stability. | | | |

**Experimental Example 2: qPCR Assay for *In Vitro* Testing of HBV**

1. Objective

HBV DNA content in HepG2.2.15 cells was detected by a real time-qPCR assay, and the inhibitory action of

the compound on HBV was evaluated by taking the $EC_{50}$ value of the compound as an index.

2. Experimental materials

2.1. cell lines: HepG2.2.15 cells

HepG2.2.15 cell culture medium (DMEM/F12, Invitrogen-11330057; 10% serum, Invitrogen-10099141; 100 units/mL penicillin and 10 μg/mL streptomycin, Invitrogen-15140122; 1% non-essential amino acids, Invitrogen-11140076; 2 mM L-Glutamine, Invitrogen-25030081; 300 μg/mL geneticin, Invitrogen-10131027)

2.2. reagents

Pancreatin (Invitrogen-25300062)
DPBS (Hyclone-SH30028.01B)
DMSO (Sigma-D2650-100ML)
High-throughput DNA purification Kit (QIAamp 96 DNA Blood Kit, Qiagen-51162)
Quantitative faststart universal probe reagent (FastStart Universal Probe Master, Roche-04914058001)
2.3. consumables and instrument
96-well cell culture plate (Corning-3599)
$CO_2$ incubator (HERA-CELL-240)
Optical sealing film (ABI-4311971)
Quantitative PCR 96-well plate (Applied Biosystems-4306737)
Fluorescent quantitative PCR instrument (Applied Biosystems-7500 real time PCR system)

3. Experimental procedures and method

3.1. HepG2.2.15 cells ($4\times10^4$ cells/well) were plated in a 96-well plate and cultured overnight at 37 °C and 5 % $CO_2$.
3.2. On the next day, the compound was diluted for a total of 8 concentrations, with 3-fold gradient dilutions. The compound at different concentrations was added into the culture wells in duplicate. The final concentration of DMSO in the culture medium was 1%. 1 μM GLS4 served as 100% inhibition control (WO2008154817A1

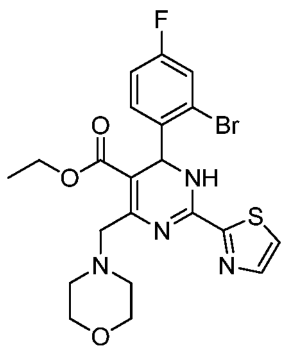

discloses the structure of GLS4 as follows: GLS4 ); DMSO at 1% served as 0% inhibition control.
3.3. On the fifth day, the original culture medium was replaced with a fresh culture medium containing the compound.
3.4. On the eighth day, the culture medium in the culture wells was collected, and the high-throughput DNA purification kit (Qiagen-51162) was used to extract DNA. For specific steps, refer to the product manual.
3.5 The preparation of the PCR reaction solution is shown in Table 26.

Table 26. Preparation of PCR reaction solution

| Items | Volume required for 1 well (μL) | Volume required for 80 wells (μL) |
|---|---|---|
| Quantitative faststart universal probe reagent | 12.5 | 1000 |
| Upstream primer (10 mol) | 1 | 80 |
| Downstream primer (10 mol) | 1 | 80 |
| Probe (10 mol) | 0.5 | 40 |

The sequence of the upstream primer is as follows: GTGTCTGCGGCGTTTTATCA.

The sequence of the downstream primer is as follows: GACAAACGGGCAACATACCTT.

The sequence of the probe is as follows: 5' + FAM + CCTCTKCATCCTGCTGCTATGCCTCATC + TAMRA -3'
3.6. 15 μL of the reaction mixture was added into each well of the 96-well PCR plate, and then 10 μL of sample DNA or HBV DNA standard was added into each well.

3.7. Reaction conditions for PCR were as follows: heating at 95 °C for 10 min; then denaturating at 95 °C for 15 s and extending at 60 °C for 1 min for 40 cycles.

3.8. **Data analysis**

3.8.1. Calculation of inhibition percentage: % Inh. = [1 - (DNA copy number in the sample - DNA copy number in 1 μM GLS4)/(DNA copy number in the DMSO control - DNA copy number in 1 μM GLS4)] × 100.

3.8.2. Calculation of $EC_{50}$: the concentration for 50% of maximal effect ($EC_{50}$) values of the compound against HBV were calculated using GraphPad Prism software.

3.8.3. The experimental results are shown in Table 27.

Table 27. $EC_{50}$ results of the qPCR assay

| Sample (title compound) | Concentration for 50% of maximal effect ($EC_{50}$) of HBV |
|---|---|
| **Compound of formula (I)** | 4nM |

**Experimental Example 3: Inhibition Test of the hERG Potassium Channel**

1. Objective

The effect of the compound disclosed herein on hERG potassium channel was detected by using automatic patch-clamp method.

2. Experimental method

2.1. Cell culture

The cells stably expressing the hERG potassium channel used in the experiment were CHO-hERE from Aviva Biosciences. CHO-hERG was cultured at 37 °C and 5% $CO_2$. CHO **hERG** culture medium is shown in Table 28.

Table 28. CHO **hERG** culture medium

| Reagent | Supplier | Volume (mL) |
|---|---|---|
| F12 culture medium | Invitrogen | 500 |
| Fetal bovine serum | Invitrogen | 50 |
| G418/Geneticin | Invitrogen | 1 |
| Hygromycin B | Invitrogen | 1 |

2.2. Preliminary preparation for cells

The CHO-hERG cells used in the experiment were cultured for at least two days, and the cell density reached

75% or more. Before the experiment, the cells were digested with TrypLE, and then the collected cells were resuspended in extracellular fluid.

2.3. Preparation of the intracellular and extracellular fluids

The extracellular fluid needed to be prepared once a month. The intracellular fluid must be frozen in aliquots at - 20 °C. Compositions of the intracellular and extracellular fluids are shown in Table 29.

Table 29. Compositions of the intracellular and extracellular fluids

| Composition | Extracellular fluid (mM) | Intracellular fluid (mM) |
|---|---|---|
| NaCl | 145 | - |
| KCl | 4 | 120 |
| KOH | - | 31.25 |
| $CaCl_2$ | 2 | 5.374 |
| $MgCl_2$ | 1 | 1.75 |
| Glucose | 10 | - |
| $Na_2ATP$ | - | 4 |
| HEPES | 10 | 10 |
| EGTA | - | 10 |
| pH | pH was adjusted to 7.4 with sodium hydroxide | pH was adjusted to 7.4 with potassium hydroxide |
| Osmotic pressure | 295mOsm | 285mOsm |

2.4. Preparation of compound

The test compound and its positive control amitriptyline were dissolved in DMSO to obtain a stock solution at a certain concentration. Then the stock solution was diluted for different gradients, and finally added to an extracellular fluid at a certain ratio to be diluted to a concentration for test. Precipitation was checked with the naked eye before the experiment. Finally, the concentration of DMSO in the solution to be tested and the positive control amitriptyline should not exceed 0.3%.

2.5. Voltage stimulation scheme

With a holding potential of -80 mv, a voltage stimulation of -50 mv was applied for 80 ms to record the cell leakage current value; then **hERG** channel was opened by a depolarization to +20 mv for 4800 ms and **hERG** tail current was elicited by a repolarization to -50 mv for 5000 ms and recorded; and finally, the voltage was restored to the holding potential of-80 mv and maintained for 3100 ms. The above voltage stimulation was repeated every 15000 ms.

2.6. QPatch$^{HTX}$ whole-cell patch clamp recording

The **hERG** QPatch$^{HTX}$ experiment was performed at room temperature. Whole-cell scheme, voltage stimulation scheme and compound detection scheme were established on QPatch Assay Software 5.2 (Sophion Bioscience).

First, 30 repetitive set voltage stimulations were performed, of which the section on a current spectrum was the baseline section for subsequent analysis. Then 5 μL of extracellular fluid was added, and the voltage stimulation was repeated three times. Each compound at the action concentration was added in sequence with a volume of 5 μL, and the voltage stimulation was repeated three times. The cells were incubated for at least 5 min with the compound at each tested concentration. During the entire recording process, all indicators needed to meet the data analysis acceptance standard. If the standard was not met, the cell would not be included in the analysis range and the compound would be tested again. The above recording process was automatically operated by the Qpatch analysis software. The tested concentrations of the compounds were 0.24 μM, 1.20 μM, 6.00 μM and 30.00 μM, and each concentration was repeated for at least two cells.

2.7. Data analysis

In each complete current recording, the inhibition percentage of each compound at the action concentration could be calculated based on the percentage of peak current in the negative control. The dose-effect relationship curve was obtained by fitting with the standard Hill Equation, and the specific equation is as follows:

$$I_{(C)} = I_b + (I_{fr} - I_b)*c^n/(IC_{50}{}^n + c^n)$$

C is the tested concentration of the compound, n is the slope, and I is the current.

The curve fitting and inhibition rate calculation were all completed by Qpatch analysis software. If the inhibition rate exceeded 50% at the lowest concentration or the inhibition rate did not reach 50% at the highest concentration, the corresponding $IC_{50}$ of the compound was lower than the lowest concentration or the $IC_{50}$ value was greater than the highest concentration.

2.8. Test results

The **hERG** $IC_{50}$ values of the compounds in the examples are shown in Table 30.

Table 30. **hERG** $IC_{50}$ values of the compounds in the examples

| Sample | hERG IC50 (μM) |
|---|---|
| Compound of formula (I) | >40 |

**Experimental Example 4: Study on Inhibition Against Cytochrome P450 Isoenzyme**

Objective: The inhibitory effect of the test compound on the activity of human liver microsomal cytochrome P450 isoenzymes (CYP1A2, CYP2C9, CYP2C19, CYP2D6 and CYP3A4) was determined.

Experimental procedures: firstly, the test compound (10 mM) was diluted in gradient to prepare working solutions (100× final concentration) at concentrations of: 5 mM, 1.5 mM, 0.5 mM, 0.15 mM, 0.05 mM, 0.015 mM and 0.005 mM, and working solutions of positive inhibitors for P450 isoenzymes (CYP1A2, CYP2C9, CYP2C19, CYP2D6 and CYP3A4) and the specific substrate mixtures thereof were prepared simultaneously; human liver microsomes frozen in a -80 °C refrigerator were thawed on ice, and after all thawed, the human liver microsomes were diluted with PB (phosphate buffered saline) to prepare a working solution at a specific concentration (0.253 mg/mL); 20 μL of the substrate mixture (20 μL of PB was added into the blank well) and 158 μL of the working solution of human liver microsomes were added into the reaction plate which was then placed on ice for use; then 2 μL of the test compound at each concentration (N=1) and a specific inhibitor (N=2) were added into the corresponding well, and the group without the inhibitor (test compound or the positive inhibitor) was added with a corresponding organic solvent as a control sample (the test compound control sample was 1:1 DMSO:MeOH; the positive control sample was 1:9 DMSO:MeOH); after pre-incubation under a 37 °C water bath for 10 min, 20 μL of a coenzyme factor (NADPH) solution was added into the reaction plate and incubated under a 37 °C water bath for 10 min, 400 μL of a cold acetonitrile solution (the internal standard was 200 ng/mL Tolbutamide and Labetalol) was added to terminate the reaction, and the reaction plate was placed on a shaker and shaken for 10 min; after centrifugation at 4,000 rpm for 20 min, 200 μL of the supernatant was collected and added to 100 μL of water to dilute the sample, and, finally, the plate was sealed, oscillated, shaken evenly, and subjected to LC/MS/MS detection. The experimental results are shown in Table 31.

Table 31. Results of inhibitory effect of the test compound on the activity of human liver microsomal cytochrome P450 isoenzymes

| Compound | $IC_{50}$ (μM) | | | | |
|---|---|---|---|---|---|
| | CYP1A2 | CYP2C9 | CYP2C19 | CYP2D6 | CYP3A4-M |
| Compound of formula (I) | >50 | >50 | >50 | >50 | >50 |

**Experimental Example 5: Experiment on Cytotoxicity**

1. The compound was diluted with DMSO (dimethyl sulfoxide) in a 3-fold gradient for 9 concentrations, and added into a 96-well plate in duplicate. The compound concentration was 200-fold of the final test concentration.
2. The cells were rinsed with PBS (phosphate buffered saline) once, added with 0.25% trypsin and digested for about 2-5 min in a 37 °C, 5% $CO_2$ incubator. Then the digestion was terminated with a cell culture medium and the cells were dispersed into single cells by pipetting with a pipettor.
3. The cell density was counted with a cell counter, and adjusted to the required density with the medium.
4. The cells were added into a 96-well plate that had been added with the compound. The final concentration of DMSO in each well was 0.5%. Wells containing 0.5% DMSO were used as non-toxic negative controls, and wells containing the cell culture medium were used as 100% cytotoxicity controls. Then the cell plate was incubated in a 37 °C, 5% $CO_2$ cell incubator for 3 days.
5. The chemiluminescence signal (RLU, relative chemiluminescence unit) of each well in the cell plate was detected with the cell viability detection kit CellTiter-Glo using the multi-functional microplate reader Envision, with instructions on the kit followed.
6. The raw data (RLU) were substituted into the following formula to calculate the cell viability of each well (cell viability%):

$$\text{Cell viability \%} = \left(RLU_{Sample} - AverageRLU_{Mediumcontrol}\right) / \left(AverageRLU_{Cellcontrol} - AverageRLU_{Mediumcontrol}\right) \times 100\ \%$$

*$RLU_{Sample}$:* signal value of the sample well; *Average $RLU_{Cell}$ control:* average signal value of the cell control well; *Average $RLU_{Medium\ control}$:* average signal value of the medium control well.

7. Using GraphPad Prism software, the cell viability data were nonlinearly fitted to draw a dose-response curve and the 50% cytotoxic concentration ($CC_{50}$) value of the compound was obtained. The results are shown in Table 32.

Table 32. Test results of 50% cytotoxic concentration ($CC_{50}$)

| Compound | $CC_{50}$(μM) |
|---|---|
| Compound of formula (I) | >50 |

**Experimental Example 6: Study on *In Vitro* Microsomal Stability**

**Metabolic stability of the compound in CD-1 mice and human liver microsomes**

Objective: The metabolic stability of the test compound in CD-1 mice and human liver microsomes was determined.

Experimental procedures: firstly, the test compound (10 mM) was subjected to a two-step dilution, where the compound was diluted to an intermediate concentration of 100 μM with 100% methanol, and the working solution was diluted to 10 μM with a potassium phosphate buffer; eight 96-well incubation plates were prepared, and named T0, T5, T10, T20, T30, T60, Blank and NCF60, respectively; the reaction time points corresponding to the first 6 incubation plates were 0 min, 5 min, 10 min, 20 min, 30 min and 60 min, respectively; for the Blank plate, neither the test compound nor a control compound was added, and for the NCF60 plate, potassium phosphate buffer was used in an incubation of 60 min in place of a NADPH regeneration system solution; 10 μL of the test compound working solution and 80 μL of the microsome working solution (liver microsome protein concentration was 0.625 mg/mL) were added to the T0, T5, T10, T20, T30, T60 and NCF60 plates, while only the microsome working solution was added to the Blank plate, and all the incubation plates were then placed in a 37 °C water bath for pre-incubation for about 10 min; after the pre-incubation, 10 μL of a NADPH regeneration system working solution was added into each sample well of all the plates except the NCF60 plate and T0 plate to start the reaction, and 10 μL of potassium phosphate buffer was added to each well of the NCF60 plate; therefore, in the test compound or control compound samples, the final reaction concentrations of the compound, testosterone, diclofenac and propafenone were 1 μM, the concentration of the liver microsomes was 0.5 mg/mL, and the final concentrations of DMSO and acetonitrile in the reaction system were 0.01% (v/v) and 0.99% (v/v), respectively; after incubation for an appropriate time (such as 5 min, 10 min, 20 min, 30 min, and 60 min), 300 μL of a stop solution (acetonitrile solution containing 100 ng/mL tolbutamide and 100 ng/mL labetalol) was added to each sample well to stop the reaction; 300 μL of the stop solution and then 10 μL of the NADPH working solution were added to the T0 plate, all the sample plates were shaken and centrifuged in a centrifuge (3220 × g) for 20 min, and then 100 μL of supernatant was taken from each well and diluted with 300 μL of pure water for liquid chromatography-tandem

mass spectrometry analysis.

The experimental results are shown in Table 33.

Table 33. Metabolic stability results of the test compound in CD-1 mice and human liver microsomes

| Compound | Species | $T_{1/2}$ (min) | $CL_{int(mic)}$ (μL/min/mg) | $CL_{int(liver)}$ (mL/min/kg) |
|---|---|---|---|---|
| Compound of formula (I) | CD-1 mice | >145 | <9.6 | <38.0 |
| | Human | >145 | <9.6 | <8.6 |

**Metabolic stability of the compound in liver microsomes of SD rats, beagle dogs and cynomolgus monkeys**

Objective: The metabolic stability of the tested compound of formula (I) in liver microsomes of rats, beagle dogs and cynomolgus monkeys was determined.

Experimental procedures: firstly, the test compound (10 mM) was subjected to a two-step dilution, where the compound was diluted to an intermediate concentration of 100 μM with 100% methanol, and the working solution was diluted to 10 μM with a potassium phosphate buffer; eight 96-well incubation plates were prepared, and named T0, T5, T10, T20, T30, T60, Blank and NCF60, respectively; the reaction time points corresponding to the first 6 incubation plates were 0 min, 5 min, 10 min, 20 min, 30 min and 60 min, respectively; for the Blank plate, neither the test compound nor a control compound was added, and for the NCF60 plate, potassium phosphate buffer was used in an incubation of 60 min in place of a NADPH regeneration system solution; 10 μL of the test compound working solution and 80 μL of the microsome working solution (liver microsome protein concentration was 0.625 mg/mL) were added to the T0, T5, T10, T20, T30, T60 and NCF60 plates, while only the microsome working solution was added to the Blank plate, and all the incubation plates were then placed in a 37 °C water bath for pre-incubation for about 10 min; after the pre-incubation, 10 μL of a NADPH regeneration system working solution was added into each sample well of all the plates except the NCF60 plate and T0 plate to start the reaction, and 10 μL of potassium phosphate buffer was added to each well of the NCF60 plate; therefore, in the test compound or control compound samples, the final reaction concentrations of the compound, testosterone, diclofenac and propafenone were 1 μM, the concentration of the liver microsomes was 0.5 mg/mL, and the final concentrations of DMSO and acetonitrile in the reaction system were 0.01% (v/v) and 0.99% (v/v), respectively; after incubation for an appropriate time (such as 5 min, 10 min, 20 min, 30 min, and 60 min), 300 μL of a stop solution (acetonitrile solution containing 100 ng/mL tolbutamide and 100 ng/mL labetalol) was added to each sample well to stop the reaction; 300 μL of the stop solution and then 10 μL of the NADPH working solution were added to the T0 plate, all the sample plates were shaken and centrifuged in a centrifuge (3220 × g) for 20 min, and then 100 μL of supernatant was taken from each well and diluted with 300 μL of pure water for liquid chromatography-tandem mass spectrometry analysis.

The experimental results are shown in Table 34.

Table 34. Metabolic stability results of the test compound in liver microsomes of SD rats, beagle dogs and cynomolgus monkeys

| Compound | Species | $T_{1/2}$ (min) | $CL_{int(mic)}$ (μL/min/mg protein) | $CL_{int(liver)}$ (mL/min/kg) |
|---|---|---|---|---|
| Compound of formula (I) | SD rats | >145 | <9.6 | <17.3 |
| | Beagle dogs | >145 | <9.6 | <13.8 |
| | Cynomolgus monkeys | >145 | <9.6 | <13.0 |

**Experimental Example 7: Pharmacokinetic Study**

Pharmacokinetic study of the test compound in Balb/c mice by oral administration and intravenous injection: The test compound was mixed with a solution containing dimethyl sulfoxide (10%), polyethylene glycol 400 (60%) and water (30%), and the mixture was vortexed and sonicated to prepare a 0.2 mg/mL clear solution, which was filtered through a millipore filter for later use. Balb/c female mice aged 7 to 10 weeks were intravenously injected with the candidate compound solution at a dose of 1 mg/kg.

The test compound was mixed with an aqueous solution containing 10% polyoxyethylene stearate, and the mixture was vortexed and sonicated to prepare a 0.3 mg/mL clear solution for later use. Balb/c female mice aged 7 to 10 weeks were orally administered with the candidate compound solution at a dose of 3 mg/kg. Whole-blood was collected

and plasma was prepared. Drug concentration was analyzed by LC-MS/MS and pharmacokinetic parameters were calculated with Phoenix WinNonlin software. The results are shown in Table 35.

Table 35. Pharmacokinetic results of the test compound

| Dosage | Pharmacokinetic parameters | Compound of formula (I) |
|---|---|---|
| IV(1 mg/kg) | Half-life $T_{1/2}$ (h) | 1.95 |
| | Clearance (CL, mL/min/kg) | 20 |
| | Apparent volume of distribution ($Vd_{ss}$, L/kg) | 3.1 |
| | Area under the plasma concentration-time curve $AUC_{o\text{-}24h}$ (nM. h) | 1488 |
| PO(3 mpk) | Peak time $T_{max}$ (h) | 0.5 |
| | Peak concentration $C_{max}$ (nM) | 1480 |
| | Area under the plasma concentration-time curve AUC (nM. h) | 2274 |
| | Bioavailability (F%) | 51% |

**Experimental Example 8: Study on Liver-to-Blood Ratio in Mice**

Experiment on the liver-to-blood ratio in Balb/c mice orally administered with the test compound

The compound of formula (I) was mixed with an aqueous solution containing 10% polyethylene glycol-15 hydroxystearate, and the mixture was vortexed and sonicated to prepare a 0.3 mg/mL clear solution for later use. Balb/c female mice aged 7 to 10 weeks were orally administered with the candidate compound solution at a dose of 3 mg/kg.

Whole blood at a certain time point was collected and plasma was prepared. Liver tissues at the corresponding time point were collected to prepare a tissue homogenate. Drug concentration was analyzed by LC-MS/MS and pharmacokinetic parameters were calculated with Phoenix WinNonlin software. The results are shown in Table 36.

Table 36. Liver-to-blood ratio of the test compound

| Compound | Compound of formula (I) | |
|---|---|---|
| Matrix | Plasma | Liver |
| $AUC_{0\text{-}last}$ (nM.h) or (nmol/kg.h) | 3476 | 86036 |
| AUC ratio (L/P) Liver/plasma exposure ratio | -- | 24.7 |

**Experimental Example 9: *In Vivo* Efficacy Study**

HDI/HBV model

Objective: The efficacy of the compound against hepatitis B virus in mice was determined through the HDI/HBV mouse model.

Preparation of compound: the solvent was 10% polyethylene glycol-15 hydroxystearate; a certain amount of the tested compound of formula (I) was dissolved in an aqueous solution containing 10% polyethylene glycol-15 hydroxystearate, and the mixture was vortexed and sonicated to prepare a homogeneous suspension, and the suspension was stored at 4 °C for later use.

High pressure injection of the HBV plasmid DNA solution via the tail vein of mice: the day of plasmid injection was day 0, the day after injection was day 1, and so on. On day 0, all animals were injected by tail vein with a saline solution containing 10μg of plasmid DNA at a volume of 8% of body weight, and the injection was completed within 5 seconds.

Administration: all mice were administered intragastrically twice a day (8/16 h interval) on day 1-6 and once on day 7. All mice were euthanized in the afternoon on day 7. The body weight of the mice, which was monitored every day, remained stable throughout the experiment.

Sample collection: blood was collected from the submandibular vein 4 h after the first administration in the morning on days 1, 3, and 5. All blood samples were collected in $K_2$-EDTA anticoagulation tubes, and centrifuged for 10 min at 4 °C, 7000 g to prepare about 40 μL of plasma. All mice were euthanized by $CO_2$ four hours after administration in the morning on day 7. Blood was collected from the heart, and the plasma preparation method was the same as

above. Two liver tissues were collected with 70-100 mg each, and quick-frozen by liquid nitrogen. After all samples were collected, they were stored in a refrigerator (-80 °C) for HBV DNA content detection.

Sample analysis: all plasma samples and liver samples were detected for HBV DNA by qPCR. The experimental results are shown in Table 37.

Table 37

| Test compound | Dosage | HBV-DNA reduction | |
|---|---|---|---|
| | | Plasma (day 5) Δ Log10 copies/μL | Liver (day 7) Δ Log10 copies/100 ng |
| Compound of formula (I) | 30 mg/kg | 3.57 | 2.36 |
| | 10 mg/kg | 2.0 | 0.94 |

## Claims

1. A crystal form of a compound of formula (I), a hydrate thereof, a solvate thereof, or a combination of the hydrate and the solvate

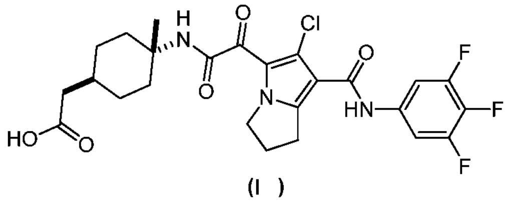

(I )

.

2. The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 6.20±020°, 8.90±0.20°, 16.30±0.20° and 24.78±0.20°;

   typically, the crystal form has characteristic diffraction peaks in an X-ray powder diffraction pattern thereof at the following 2θ: 6.20±0.20°, 8.90±0.20°, 14.22±0.20°, 16.30±0.20°, 22.32±0.20° and 24.78±0.20°; typically, the crystal form has characteristic diffraction peaks in an X-ray powder diffraction pattern thereof at the following 2θ: 6.20±0.20°, 8.90±0.20°, 11.26±0.20°, 14.22±020°, 16.30±0.20°, 17.89±0.20°, 22.32±0.20° and 24.78±0.20°;
   typically, the crystal form has characteristic diffraction peaks in an X-ray powder diffraction pattern thereof at the following 2θ: 6.20±0.20°, 8.90±0.20°, 10. 09±0.20°, 11.26±0.20°, 14.22±0.20°, 16.30±0.20°, 17.89±0.20°, 20.35±0.20°, 22.32±0.20°, 24.78±0.20° and 27.78±0.20°.

3. The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 9.13±0.20°, 10.53±0.20°, 21.17±0.20° and 22.64±0.20°; typically, the crystal form has characteristic diffraction peaks in an X-ray powder diffraction pattern thereof at the following 2θ: 9.13±0.20°, 10.53±0.20°, 11.67±0.20°, 20.09±0.20°, 21.17±0.20° and 22.64±0.20°; typically, the crystal form has characteristic diffraction peaks in an X-ray powder diffraction pattern thereof at the following 2θ: 9.13±0.20°, 10.53±0.20°, 11.67±0.20°, 13.52±0.20°, 20.09±0.20°, 21.17±0.20° and 22.64±0.20°.

4. The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 8.94±0.20°, 9.83±0.20° and 10.99±0.20°;

   typically, the crystal form has characteristic diffraction peaks in an X-ray powder diffraction pattern thereof at the following 2θ: 8.94±0.20°, 9.83±0.20°, 10.99±0.20°, 18.62±0.20° and 19.82±0.20°;
   typically, the crystal form has characteristic diffraction peaks in an X-ray powder diffraction pattern thereof at the following 2θ: 8.94±0.20°, 9.83±0.20°, 10.99±0.20°, 13.36±020°, 17.21±0.20°, 18.62±0.20°, 19.82±0.20° and 21.56±0.20°;
   typically, the crystal form has characteristic diffraction peaks in an X-ray powder diffraction pattern thereof at

the following 2θ: 8.94±0.20°, 9.39±020°, 9.83±0.20°, 10.48±0.20°±0.20°, 10.99±020°, 13.36±0.20°, 14.29±0.20°, 17.21±0.20°, 18.14±0.20°, 18.62±0.20°, 19.82±0.20° and 21.56±0.20°.

**5.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 7.52±0.20°, 11.21±0.20°, 12.40±0.20° and 14.41±0.20°;

typically, the crystal form has characteristic diffraction peaks in an X-ray powder diffraction pattern thereof at the following 2θ: 7.52±0.20°, 8.70±0.20°, 11.21±0.20°, 12.40±0.20°, 14.41±0.20°, 17.49±020° and 22.92±0.20°;
typically, the crystal form has characteristic diffraction peaks in an X-ray powder diffraction pattern thereof at the following 2θ: 7.52±0.20°, 8.70±0.20°, 11.21±0.20°, 12.40±0.20°, 14.41±0.20°, 16.06±0.20°, 17.49±0.20°, 20.98±0.20°, 21.98±0.20° and 22.92±020°;
typically, the crystal form has characteristic diffraction peaks in an X-ray powder diffraction pattern thereof at the following 2θ: 7.17±020°, 7.52±0.20°, 7.99±0.20°, 8.70±020°, 9.99±0.20°, 10.74±0.20°, 11.21±0.20°, 12.40±0.20°, 14.41±0.20°, 14.88±0.20°, 16.06±0.20°, 17.05±0.20°, 17.49±0.20°, 20.98±0.20°, 21.98±0.20°, 22.48±0.20°, 22.92±0.20°, 23.50±0.20°, 26.47±0.20°, 27.05±0.20° and 28.04±0.20°.

**6.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 6.72±0.20°, 8.53±0.20°, 17.76±0.20° and 20.38±0.20°;

typically, the crystal form has characteristic diffraction peaks in an X-ray powder diffraction pattern thereof at the following 2θ: 6.72±0.20°, 8.53±0.20°, 10.50±0.20°, 13.53±0.20°, 17.76±0.20°, 18.83±020° and 20.38±0.20°;
typically, the crystal form has characteristic diffraction peaks in an X-ray powder diffraction pattern thereof at the following 2θ: 6.72±0.20°, 8.53±0.20°, 10.50±0.20°, 13.53±0.20°, 17.76±0.20°, 18.83±0.20°, 20.38±0.20°, 21.06±0.20° and 24.00±0.20°;
typically, the crystal form has characteristic diffraction peaks in an X-ray powder diffraction pattern thereof at the following 2θ: 6.72±0.20°, 8.53±0.20°, 10.50±0.20°, 11.41±0.20°, 13.53±0.20°, 17.76±0.20°, 18.83±0.20°, 19.99±0.20°, 20.38±0.20°, 21.06±0.20°, 22.23±0.20°, 24.00±0.20°, 24.42±0.20° and 25.90±0.20°.

**7.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 4.18±0.20°, 8.35±0.20°, 10.58±0.20° and 16.86±0.20°; typically, the crystal form has characteristic diffraction peaks in an X-ray powder diffraction pattern thereof at the following 2θ: 4.18±0.20°, 8.35±0.20°, 10.58±0.20°, 11.87±0.20°, 16.86±0.20° and 21.16±0.20°; typically, the crystal form has characteristic diffraction peaks in an X-ray powder diffraction pattern thereof at the following 2θ: 4.18±0.20°, 8.35±0.20°, 10.58±0.20°, 11.87±0.20°, 12.32±0.20°, 16.86±0.20°, 21.16±0.20°, 25.47±0.20° and 29.17±0.20°.

**8.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 7.03±020°, 8.31±0.20°, 19.18±0.20° and 25.99±0.20°;

typically, the crystal form has characteristic diffraction peaks in an X-ray powder diffraction pattern thereof at the following 2θ: 7.03±0.20°, 8.31±0.20°, 15.86±0.20°, 19.18±0.20°, 21.05±0.20° and 25.99±0.20°; typically, the crystal form has characteristic diffraction peaks in an X-ray powder diffraction pattern thereof at the following 2θ: 7.03±0.20°, 8.31±0.20°, 11.62±0.20°, 12.91±0.20°, 15.86±0.20°, 19.18±0.20°, 21.05±0.20°, 24.67±0.20° and 25.99±0.20°;
typically, the crystal form has characteristic diffraction peaks in an X-ray powder diffraction pattern thereof at the following 2θ: 7.03±0.20°, 8.31±0.20°, 11.62±0.20°, 12.91±0.20°, 15.86±0.20°, 17.17±0.20°, 18.20±0.20°, 19.18±0.20°, 19.74±0.20°, 21.05±0.20°, 21.30±0.20°, 24.67±0.20° and 25.99±0.20°.

**9.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 7.05±0.20°, 9.10±0.20°, 10.78±0.20° and 22.90±0.20°;

typically, the crystal form has characteristic diffraction peaks in an X-ray powder diffraction pattern thereof at the following 2θ: 7.05±0.20°, 9.10±0.20°, 10.78±0.20°, 21.24±0.20°, 21.74±0.20° and 22.90±0.20°; typically, the crystal form has characteristic diffraction peaks in an X-ray powder diffraction pattern thereof at the following 2θ: 7.05±0.20°, 9.10±0.20°, 10.78±0.20°, 13.07±0.20°, 19.57±0.20°, 21.24±0.20°, 21.74±0.20°, 22.24±0.20° and 22.90±0.20°;
typically, the crystal form has characteristic diffraction peaks in an X-ray powder diffraction pattern thereof at the following 2θ: 7.05±0.20°, 9.10±0.20°, 10.78±0.20°, 11.34±0.20°, 13.07±0.20°, 14.07±0.20°, 14.99±0.20°, 15.86±0.20°, 16.17±0.20°, 18.60±0.20°, 19.57±0.20°, 21.24±0.20°, 21.46±0.20°, 21.74±0.20°, 22.24±0.20°, 22.72±0.20° and 22.90±0.20°.

**10.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 5.56±0.20°, 11.25±0.20° and 14.09±0.20°;

typically, the crystal form has characteristic diffraction peaks in an X-ray powder diffraction pattern thereof at the following 2θ: 5.56±0.20°, 7.54±0.20°, 11.25±0.20, 14.09±0.20° and 19.64±0.20°;
typically, the crystal form has characteristic diffraction peaks in an X-ray powder diffraction pattern thereof at the following 2θ: 5.56±0.20°, 7.54±0.20°, 11.25±0.20°, 14.09±0.20°, 18.07±0.20°, 19.64±0.20° and 20.33±0.20°;
typically, the crystal form has characteristic diffraction peaks in an X-ray powder diffraction pattern thereof at the following 2θ: 5.56±0.20°, 7.54±0.20°, 11.25±0.20°, 14.09±0.20°, 18.07±0.20°, 19.64±0.20°, 20.33±0.20°, 21.65±0.20° and 22.31±0.20°.

**11.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 9.28±0.20°, 10.34±0.20°, 22.66±0.20° and 26.12±0.20°;

typically, the crystal form has characteristic diffraction peaks in an X-ray powder diffraction pattern thereof at the following 2θ: 9.28±0.20°, 10.34±0.20°, 19.45±0.20°, 20.93±0.20°, 22.66±0.20° and 26.12±0.20°;
typically, the crystal form has characteristic diffraction peaks in an X-ray powder diffraction pattern thereof at the following 2θ: 9.28±0.20°, 10.34±0.20°, 12.35±0.20°, 14.95±0.20°, 17.88±0.20°, 19.45±0.20°, 20.93±0.20°, 22.66±0.20° and 26.12±0.20°;
typically, the crystal form has characteristic diffraction peaks in an X-ray powder diffraction pattern thereof at the following 2θ: 3.47±0.20°, 9.28±0.20°, 10.34±0.20°, 10.92±0.20°, 12.35±0.20°, 14.95±0.20°, 17.62±0.20°, 17.88±0.20°, 19.09±0.20°, 19.45±0.20°, 20.08±0.20°, 20.93±0.20°, 22.66±0.20°, 23.98±0.20°, 26.12±0.20° and 28.63±0.20°.

**12.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 7.49±020°, 8.46±0.20°, 15.99±0.20° and 17.02±0.20°;

typically, the crystal form has characteristic diffraction peaks in an X-ray powder diffraction pattern thereof at the following 2θ: 7.49±0.20°, 8.46±0.20°, 13.18±0.20°, 14.43±0.20°, 15.99±0.20° and 17.02±0.20°; typically, the crystal form has characteristic diffraction peaks in an X-ray powder diffraction pattern thereof at the following 2θ: 7.49±0.20°, 8.46±0.20°, 9.91±0.20°, 13.18±0.20°, 14.43±0.20°, 15.99±020°, 17.02±0.20°, 20.97±0.20° and 24.20±0.20°;
typically, the crystal form has characteristic diffraction peaks in an X-ray powder diffraction pattern thereof at the following 2θ: 7.49±0.20°, 8.46±0.20°, 9.12±0.20°, 9.91±0.20°, 10.67±0.20°, 13.18±0.20°, 14.43±0.20°, 15.99±0.20°, 17.02±0.20°, 18.34±0.20°, 19.95±0.20°, 20.29±0.20°, 20.97±0.20°, 21.66±0.20°, 23.03±0.20°, 24.20±0.20°, 24.94±0.20° and 25.69±0.20°.

**13.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 8.53±0.20°, 11.09±0.20°, 22.34±0.20° and 23.12±0.20°;

typically, the crystal form has characteristic diffraction peaks in an X-ray powder diffraction pattern thereof at the following 2θ: 8.53±0.20°, 11. 09±0.20°, 15.00±0.20°, 20.76±0.20°, 22.34±0.20° and 23.12±0.20°; typically, the crystal form has characteristic diffraction peaks in an X-ray powder diffraction pattern thereof at the

following 2θ: 7.03±0.20°, 8.53±0.20°, 11.09±0.20°, 14.14±0.20°, 15.00±0.20°, 20.76±0.20°, 22.34±0.20°, 23.12±0.20° and 26.85±0.20°;
typically, the crystal form has characteristic diffraction peaks in an X-ray powder diffraction pattern thereof at the following 2θ: 7.03±0.20°, 8.53±0.20°, 10.50±0.20°, 11.09±0.20°, 14.14±0.20°, 15.00±0.20°, 20.76±0.20°, 22.34±0.20°, 23.12±0.20° and 26.85±0.20°.

**14.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 9.32±0.20°, 10.43±0.20°, 12.46±0.20° and 19.62±0.20°;

typically, the crystal form has characteristic diffraction peaks in an X-ray powder diffraction pattern thereof at the following 2θ: 9.32±0.20°, 10.43±0.20°, 10.81±0.20°, 12.46±0.20°, 19.62±0.20° and 21.03±0.20°; typically, the crystal form has characteristic diffraction peaks in an X-ray powder diffraction pattern thereof at the following 2θ: 9.32±0.20°, 10.43±0.20°, 10.81±0.20°, 12.46±0.20°, 17.55±0.20°, 17.99±0.20°, 19.62±0.20°, 21.03±0.20° and 22.90±0.20°;
typically, the crystal form has characteristic diffraction peaks in an X-ray powder diffraction pattern thereof at the following 2θ: 9.32±0.20°, 10.43±0.20°, 10.81±0.20°, 12.46±0.20°, 13.00±0.20°, 15.06±0.20°, 17.55±0.20°, 17.99±0.20°, 19.62±0.20°, 21.03±0.20° and 22.90±0.20°.

**15.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 8.47°±0.20°, 12.62±0.20°, 15.70±0.20° and 18.41±0.20°;

typically, the crystal form has characteristic diffraction peaks in an X-ray powder diffraction pattern thereof at the following 2θ: 8.47±0.20°, 11.23±0.20°, 12.62±0.20°, 15.70±0.20°, 18.41±0.20° and 21.49±0.20°; typically, the crystal form has characteristic diffraction peaks in an X-ray powder diffraction pattern thereof at the following 2θ: 7.04±0.20°, 8.47±0.20°, 10.01±0.20°, 11.23±0.20°, 12.62±0.20°, 15.70±0.20°, 18.41±0.20°, 21.49±0.20° and 22.53±0.20°;
typically, the crystal form has characteristic diffraction peaks in an X-ray powder diffraction pattern thereof at the following 2θ: 7.04±0.20°, 8.47±0.20°, 10.01±0.20°, 11.23±0.20°, 12.62±0.20°, 15.70±0.20°, 17.32±0.20°, 18.41±0.20°, 20.31±0.20°, 21.49±0.20°, 22.53±0.20° and 26.34±0.20°.

**16.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 7.50±0.20°, 10.65±0.20° and 11.10±0.20°;

typically, the crystal form has characteristic diffraction peaks in an X-ray powder diffraction pattern thereof at the following 2θ: 7.18±0.20°, 7.50±0.20°, 10.65±0.20°, 11.10±0.20°, 14.04±0.20° and 21.48±0.20°; typically, the crystal form has characteristic diffraction peaks in an X-ray powder diffraction pattern thereof at the following 2θ: 7.18±0.20°, 7.50±0.20°, 10.65±0.20°, 11.10±0.20°, 14.04±0.20°, 21.48±0.20°, 22.79±0.20° and 27.02±0.20°;
typically, the crystal form has characteristic diffraction peaks in an X-ray powder diffraction pattern thereof at the following 2θ: 7.18±0.20°, 7.50±0.20°, 10.65±0.20°, 11.10±0.20°, 14.04±0.20°, 15.67±0.20°, 19.16±0.20°, 21.48±0.20°, 22.79±0.20°, 23.39±0.20°, 26.28±0.20° and 27.02±0.20°.

**17.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 7. 12±0.20° and 21.46±0.20°; typically, the crystal form has characteristic diffraction peaks in an X-ray powder diffraction pattern thereof at the following 2θ: 7.12±0.20°, 21.28±0.20° and 21.46±0.20°;

typically, the crystal form has characteristic diffraction peaks in an X-ray powder diffraction pattern thereof at the following 2θ: 7.12±0.20°, 20.38±0.20°, 21.28±0.20°, 21.46±0.20°, 26.72±0.20° and 27.48±0.20°; typically, the crystal form has characteristic diffraction peaks in an X-ray powder diffraction pattern thereof at the following 2θ: 7.12±0.20°, 20.38±0.20°, 21.46±0.20°, 26.72±0.20° and 27.48±0.20°;
typically, the crystal form has characteristic diffraction peaks in an X-ray powder diffraction pattern thereof at the following 2θ: 7.12±0.20°, 12.96±0.20°, 18.66±0.20°, 20.38±0.20°, 21.46±0.20°, 22.74±0.20°, 26.72±0.20°, 27.48±0.20° and 27.82±0.20°;

typically, the crystal form has characteristic diffraction peaks in an X-ray powder diffraction pattern thereof at the following 2θ: 7.12±0.20°, 12.96±0.20°, 18.66±0.20°, 20.38±0.20°, 21.28±0.20°, 21.46±0.20°, 22.74±0.20°, 24.84±0.20°, 26.72±0.20°, 27.48±0.20° and 27.82±0.20°;
typically, the crystal form has characteristic diffraction peaks in an X-ray powder diffraction pattern thereof at the following 2θ: 7.12±0.20°, 15.40±0.20°, 21.28±0.20°, 21.46±0.20° and 22.74±0.20°;
typically, the crystal form has characteristic diffraction peaks in an X-ray powder diffraction pattern thereof at the following 2θ: 7.12±0.20°, 12.96±0.20°, 15.40±0.20°, 21.28±0.20°, 21.46±0.20°, 22.74±0.20° and 27.48±0.20°;
typically, the crystal form has characteristic diffraction peaks in an X-ray powder diffraction pattern thereof at the following 2θ: 7.12±0.20°, 12.96±0.20°, 15.40±0.20°, 20.38±0.20°, 21.28±0.20°, 21.46±0.20°, 22.74±0.20°, 26.72±0.20° and 27.48±020°;
typically, the crystal form has characteristic diffraction peaks in an X-ray powder diffraction pattern thereof at the following 2θ: 7.12±0.20°, 12.96±0.20°, 15.40±0.20°, 18.66±0.20°, 20.38±0.20°, 21.28±0.20°, 21.46±0.20°, 22.74±0.20°, 26.72±0.20°, 27.48±0.20° and 27.82±0.20°;
typically, the crystal form has characteristic diffraction peaks in an X-ray powder diffraction pattern thereof at the following 2θ: 7.12±0.20°, 8.74±0.20°, 12.96±0.20°, 15.40±0.20°, 15.92±0.20°, 18.66±0.20°, 20.38±0.20°, 21.28±0.20°, 21.46±0.20°, 22.74±0.20°, 26.72±0.20° and 27.48±0.20°;
typically, the crystal form has characteristic diffraction peaks in an X-ray powder diffraction pattern thereof at the following 2θ: 7.12±0.20°, 8.74±0.20°, 11.66±0.20°, 12.96±0.20°, 15.40±0.20°, 15.92±0.20°, 16.22±0.20°, 18.66±0.20°, 20.38±020°, 21.28±0.20°, 21.46±0.20°, 22.74±0.20°, 26.72±0.20°, 27.48±020° and 27.82±0.20°;
typically, the crystal form has characteristic diffraction peaks in an X-ray powder diffraction pattern thereof at the following 2θ: 7.12±0.20°, 8.74±0.20°, 9.29±0.20°, 11.66±0.20°, 12.96±0.20°, 15.40±0.20°, 15.92±0.20°, 16.22±0.20°, 17.54±0.20°, 18.66±0.20°, 20.38±0.20°, 21.28±0.20°, 21.46±0.20°, 22.74±0.20°, 26.72±0.20°, 27.48±0.20° and 27.82±0.20°.

**18.** The crystal form of the compound of formula (I), the hydrate thereof, the solvate thereof, or the combination of the hydrate and the solvate according to claim 1 or 17, wherein the crystal form belongs to a triclinic crystal system, with a space group being P-1, unit cell parameters being a = 9.407(2)A, b = 11.531(3)A, c = 13.574(4)A, a = 66.982(8)°, p = 75.337(8)° and y = 68.492(8)°, a volume of a unit cell being V = 1250.4(6)A 3, and a number of asymmetric units in a unit cell being Z = 2.

**19.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 7.03±0.20°, 8.22±0.20° and 14.10±0.20°;

typically, the crystal form has characteristic diffraction peaks in an X-ray powder diffraction pattern thereof at the following 2θ: 7.03±0.20°, 8.22±0.20°, 10.34±0.20°, 14.10±0.20°, 14.66±0.20° and 21.61±020°; typically, the crystal form has characteristic diffraction peaks in an X-ray powder diffraction pattern thereof at the following 2θ: 7.03±0.20°, 8.22±0.20°, 8.53±020°, 10.34±0.20°, 14.10±0.20°, 14.66±0.20°, 16.47±0.20°, 17.07±0.20° and 21.61±0.20°;
typically, the crystal form has characteristic diffraction peaks in an X-ray powder diffraction pattern thereof at the following 2θ: 7.03±0.20°, 8.22±0.20°, 8.53±0.20°, 8.95±0.20°, 10.34±0.20°, 14.10±0.20°, 14.66±0.20°, 15.90±0.20°, 16.47±0.20°, 17.07±0.20°, 19.45±0.20°, 21.61±0.20°, 22.89±0.20° and 23.36±0.20°.

**20.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 6.60±0.20°, 8.55±0.20° and 15.21±0.20°;
typically, the crystal form has characteristic diffraction peaks in an X-ray powder diffraction pattern thereof at the following 2θ: 6.60±0.20°, 8.55±0.20°, 11.67±0.20°, 15.21±0.20°, 17.60±0.20° and 24.56±0.20°; typically, the crystal form has characteristic diffraction peaks in an X-ray powder diffraction pattern thereof at the following 2θ: 6.60±0.20°, 8.55±0.20°, 11.67±0.20°, 15.21±0.20°, 17.12±0.20°, 17.60±0.20°, 23.25±0.20°, 24.56±0.20° and 27.31±0.20°.

**21.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 6.15±0.20°, 8.43±0.20°, 21.57±0.20° and 23.90±0.20°;

typically, the crystal form has characteristic diffraction peaks in an X-ray powder diffraction pattern thereof at the following 2θ: 6.15±0.20°, 8.43±0.20°, 14.94±0.20°, 16.29±0.20°, 16.84±0.20°, 21.57±0.20° and 23.90±0.20°;

typically, the crystal form has characteristic diffraction peaks in an X-ray powder diffraction pattern thereof at the following 2θ: 6.15±0.20°, 6.57±0.20°, 8.43±0.20°, 11.27±0.20°, 14.94±0.20°, 16.29±0.20°, 16.84±0.20°, 17.71±0.20°, 21.57±0.20° and 23.90±020°;

typically, the crystal form has characteristic diffraction peaks in an X-ray powder diffraction pattern thereof at the following 2θ: 6.15±0.20°, 6.57±0.20°, 8.43±0.20°, 8.83±0.20°, 11.27±0.20°, 13.97±0.20°, 14.23±0.20°, 14.94±0.20°, 16.29±0.20°, 16.84±0.20°, 17.20±0.20°, 17.71±0.20°, 18.48±0.20°, 19.19±0.20°, 20.36±0.20°, 20.74±0.20°, 21.57±0.20°, 22.61±0.20°, 23.02±0.20°, 23.90±0.20°, 26.16±0.20°, 26.67±0.20° and 27.74±0.20°;

typically, the crystal form has characteristic diffraction peaks in an X-ray powder diffraction pattern thereof at the following 2θ: 6.15±0.20°, 6.57±0.20°, 8.43±0.20°, 8.83±0.20°, 9.90±0.20°, 11.27±0.20°, 11.54±0.20°, 13.13±0.20°, 13.97±0.20°, 14.23±0.20°, 14.94±0.20°, 16.29±0.20°, 16.84±0.20°, 17.20±0.20°, 17.71±0.20°, 18.48±0.20°, 19.19±0.20°, 20.36±0.20°, 20.74±0.20°, 21.57±0.20°, 22.20±0.20°, 22.61±0.20°, 23.02±0.20°, 23.90±0.20°, 24.92±0.20°, 25.58±0.20°, 26.16±0.20°, 26.67±0.20°, 27.74±0.20°, 28.25±0.20° and 31.28±0.20°.

**22.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 7.23±0.20°, 8.45±0.20°, 16.18±0.20° and 26.35±0.20°;

typically, the crystal form has characteristic diffraction peaks in an X-ray powder diffraction pattern thereof at the following 2θ: 7.23±0.20°, 8.45±0.20°, 12.85±0.20°, 16.18±0.20°, 19.41±0.20° and 26.35±0.20°; typically, the crystal form has characteristic diffraction peaks in an X-ray powder diffraction pattern thereof at the following 2θ: 7.23±0.20°, 8.45±0.20°, 12.85±0.20°, 16.18±0.20°, 18.46±0.20°, 19.41±0.20°, 19.97±0.20°, 21.46±0.20° and 26.35±0.20°;

typically, the crystal form has characteristic diffraction peaks in an X-ray powder diffraction pattern thereof at the following 2θ: 723±0.20°, 8.45±0.20°, 9.72±0.20°, 12.85±0.20°, 14.41±0.20°, 16.18±0.20°, 18.46±0.20°, 19.41±0.20°, 19.97±0.20°, 21.46±0.20°, 24.95±0.20° and 26.35±0.20°.

**23.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 3.50±0.20°, 6.97±0.20°, 9.51±0.20° and 19.13±0.20°;

typically, the crystal form has characteristic diffraction peaks in an X-ray powder diffraction pattern thereof at the following 2θ: 3.50±0.20°, 6.97±0.20°, 9.51±0.20°, 11.55±0.20°, 17.53±0.20°, 19.13±0.20° and 19.62±0.20°;

typically, the crystal form has characteristic diffraction peaks in an X-ray powder diffraction pattern thereof at the following 2θ: 3.50±0.20°, 6.97±0.20°, 9.51±0.20°, 10.29±0.20°, 11.55±0.20°, 14.00±0.20°, 17.53±0.20°, 19.13±0.20°, 19.62±0.20° and 21.09±0.20°;

typically, the crystal form has characteristic diffraction peaks in an X-ray powder diffraction pattern thereof at the following 2θ: 3.50±0.20°, 6.97±0.20°, 9.51±0.20°, 9.98±0.20°, 10.29±0.20°, 11.55±0.20°, 14.00±0.20°, 17.53±0.20°, 19.13±0.20°, 19.62±0.20°, 20.71±0.20°, 21.09±0.20°, 21.41±0.20°, 22.32±0.20°, 24.35±0.20°, 26.98±0.20° and 35.53±0.20°.

**24.** The crystal form of the compound of formula (I), the hydrate thereof, the solvate thereof, or the combination of the hydrate and the solvate according to claim 1, wherein the crystal form has characteristic diffraction peaks in an X-ray powder diffraction pattern thereof at the following 2θ: 7.17±0.20°, 9.44±0.20°, 19.06±0.20° and 19.56±0.20°;

typically, the crystal form has characteristic diffraction peaks in an X-ray powder diffraction pattern thereof at the following 2θ: 7.17±0.20°, 9.44±0.20°, 10.22±0.20°, 11.48±0.20°, 19.06±0.20°, 19.56±0.20° and 21.35±0.20°;

typically, the crystal form has characteristic diffraction peaks in an X-ray powder diffraction pattern thereof at the following 2θ: 6.90±0.20°, 7.17±0.20°, 8.99±0.20°, 9.44±020°, 9.91±0.20°, 10.22±0.20°, 11.48±0.20°, 19.06±0.20°, 19.56±0.20° and 21.35±0.20°;

typically, the crystal form has characteristic diffraction peaks in an X-ray powder diffraction pattern thereof at the following 2θ: 6.90±0.20°, 7.17±0.20°, 8.99±0.20°, 9.44±0.20°, 9.91±0.20°, 10.22±0.20°, 11.48±0.20°,

14.50±0.20°, 17.46±0.20°, 19.06±0.20°, 19.56±0.20°, 21.01±0.20°, 21.35±0.20°, 21.85±0.20°, 22.25±0.20° and 24.27±0.20°;

typically, the crystal form has characteristic diffraction peaks in an X-ray powder diffraction pattern thereof at the following 2θ: 6.90±0.20°, 7.17±0.20°, 8.99±0.20°, 9.44±020°, 9.91±0.20°, 10.22±0.20°, 10.41±0.20°, 10.57±0.20°, 11.48±0.20°, 13.93±0.20°, 14.50±0.20°, 17.46±0.20°, 19.06±0.20°, 19.56±0.20°, 20.64±0.20°, 21.01±0.20°, 21.35±0.20°, 21.85±0.20°, 22.25±0.20°, 24.27±0.20°, 26.88±0.20° and 29.30±0.20°.

**25.** A crystal form composition comprising the crystal form according to any one of claims 1 to 24, wherein the crystal form accounts for 50% or more, preferably 80% or more, more preferably 90% or more and most preferably 95% or more of the weight of the crystal form composition.

**26.** A pharmaceutical composition, comprising a therapeutically effective amount of the crystal form according to any one of claims 1 to 24 or the crystal form composition according to claim 25.

**27.** Use of the crystal form according to any one of claims 1 to 24, the crystal form composition according to claim 25, or the pharmaceutical composition according to claim 26 for preparing a medicament for inhibiting nucleoprotein.

**28.** The use according to claim 27, wherein the medicament for inhibiting nucleoprotein is a medicament for treating or preventing HBV infection related diseases.

**29.** Use of the crystal form according to any one of claims 1 to 24, the crystal form composition according to claim 25, or the pharmaceutical composition according to claim 26 for preparing a medicament for treating or preventing HBV infection related diseases.

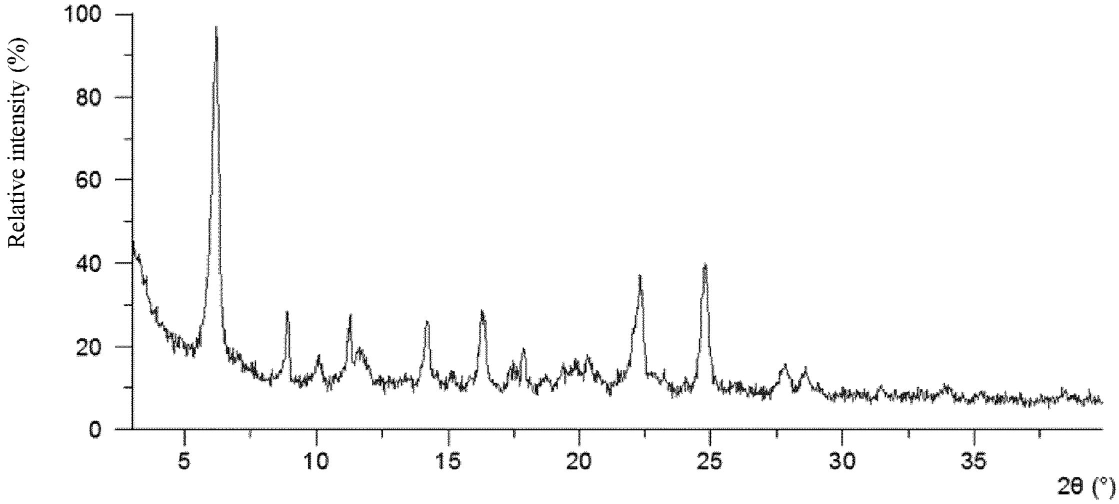
Relative intensity (%)
100
80
60
40
20
0
5
10
15
20
25
30
35
2θ (°)

FIG. 1

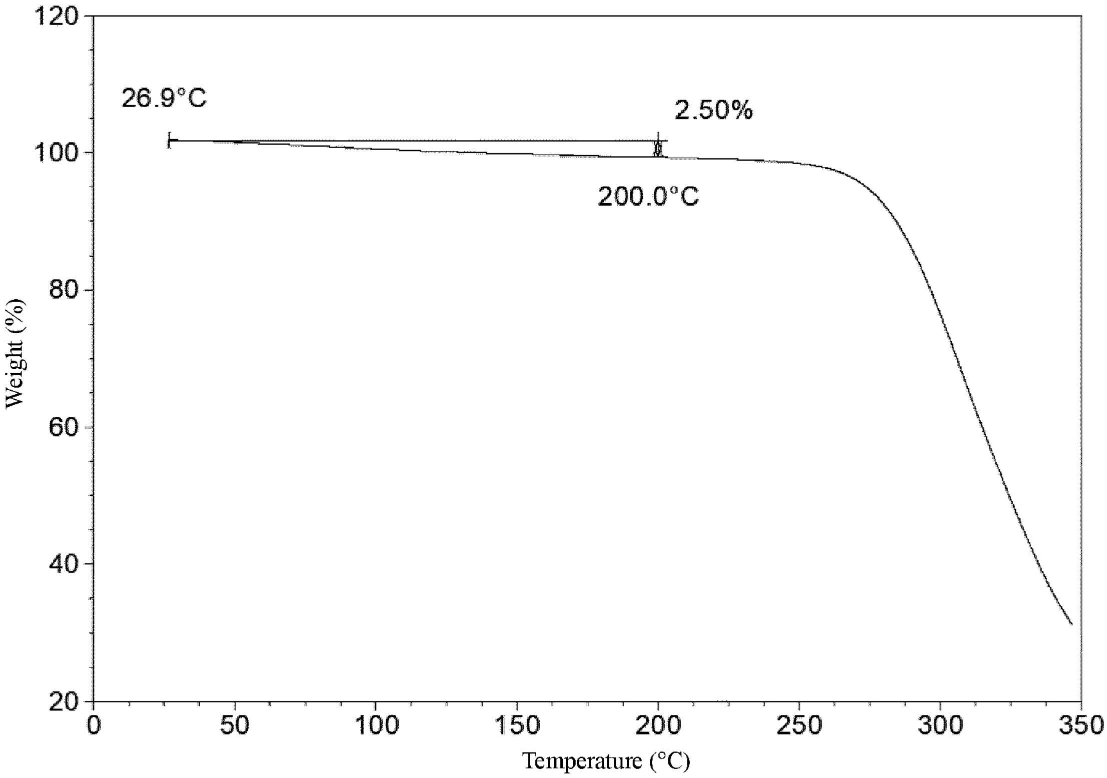
120
100
80
60
40
20
Weight (%)
26.9°C
2.50%
200.0°C
0
50
100
150
200
250
300
350
Temperature (°C)

FIG. 2

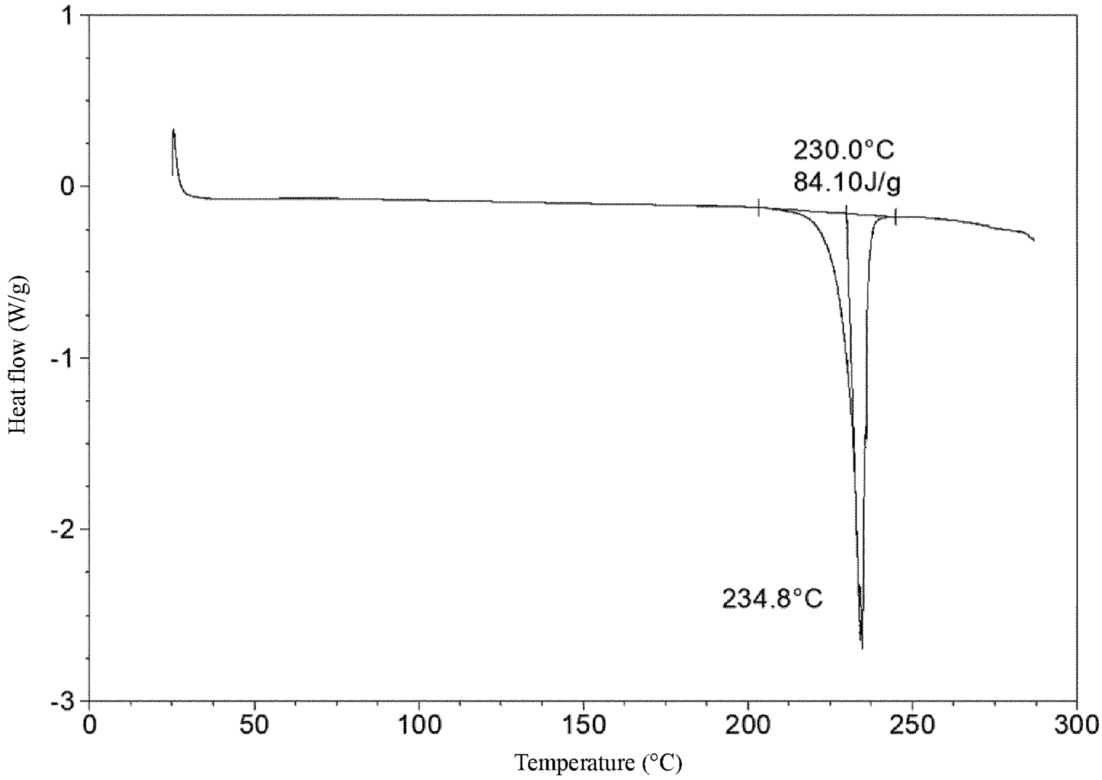
230.0°C
84.10J/g
234.8°C
Heat flow (W/g)
Temperature (°C)
1
0
-1
-2
-3
0
50
100
150
200
250
300

FIG. 3

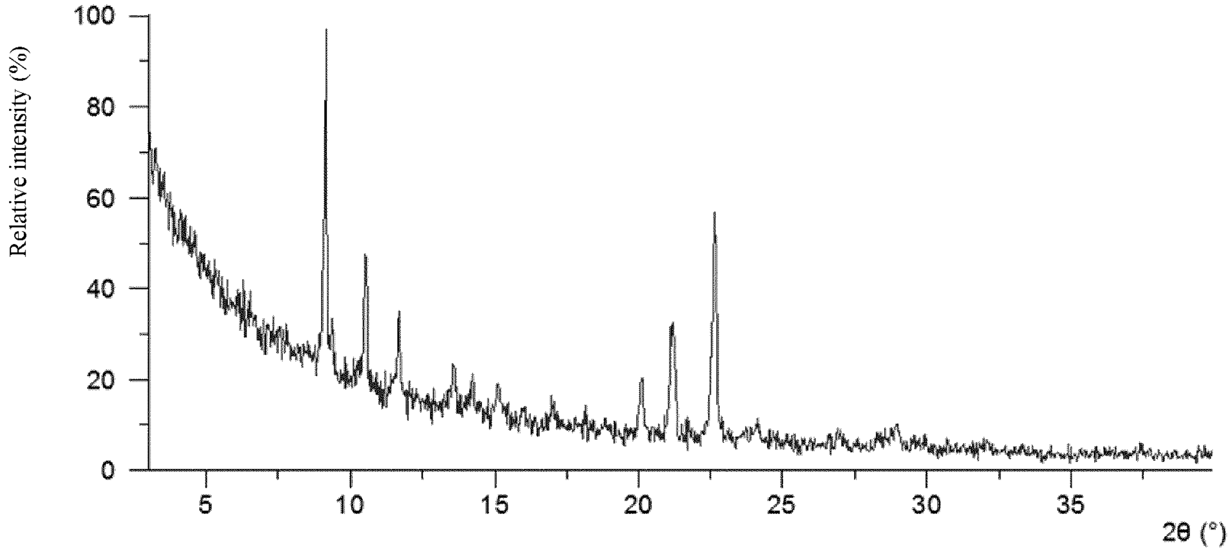
Relative intensity (%)
2θ (°)
100
80
60
40
20
0
5
10
15
20
25
30
35

FIG. 4

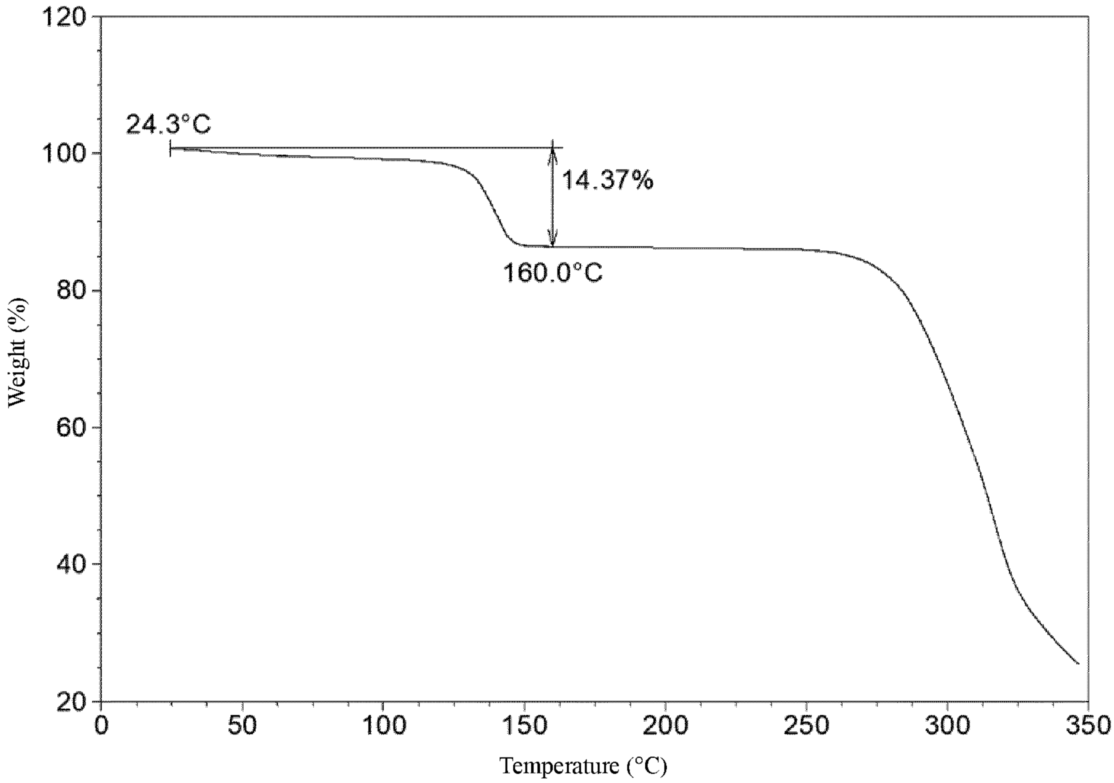
24.3°C
14.37%
160.0°C
Weight (%)
Temperature (°C)
120
100
80
60
40
20
0
50
100
150
200
250
300
350

FIG. 5

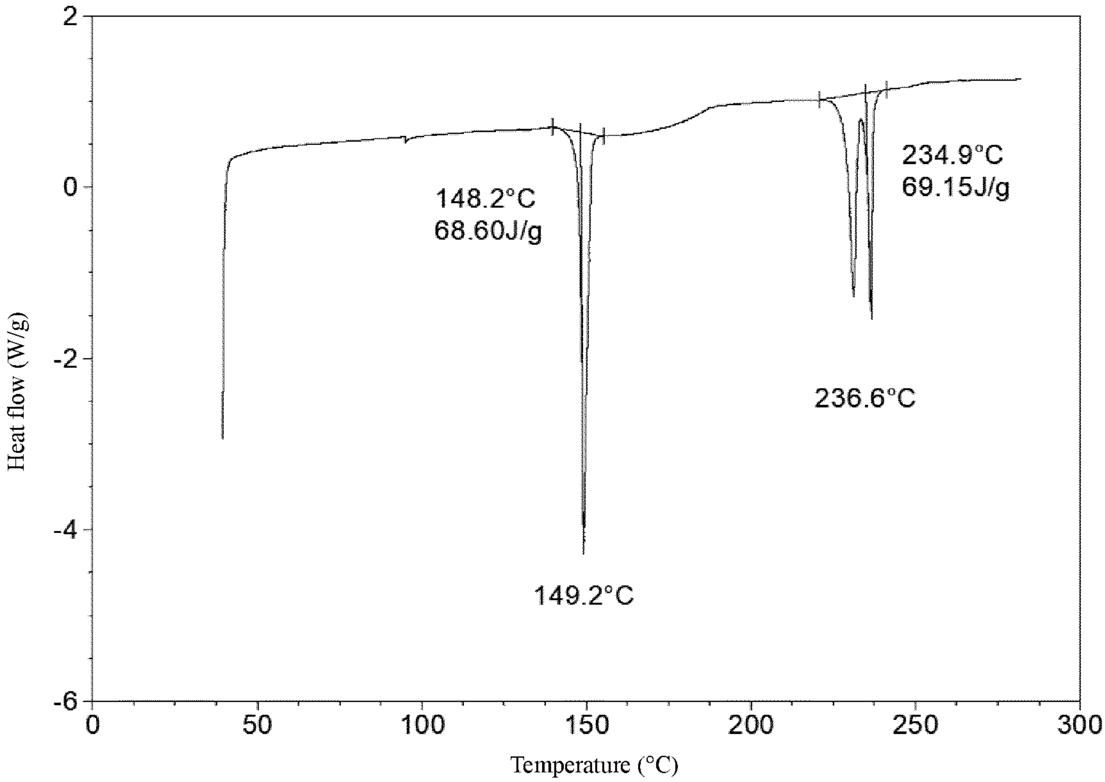
148.2°C
68.60J/g
234.9°C
69.15J/g
236.6°C
149.2°C
Heat flow (W/g)
Temperature (°C)
2
0
-2
-4
-6
0
50
100
150
200
250
300

FIG. 6

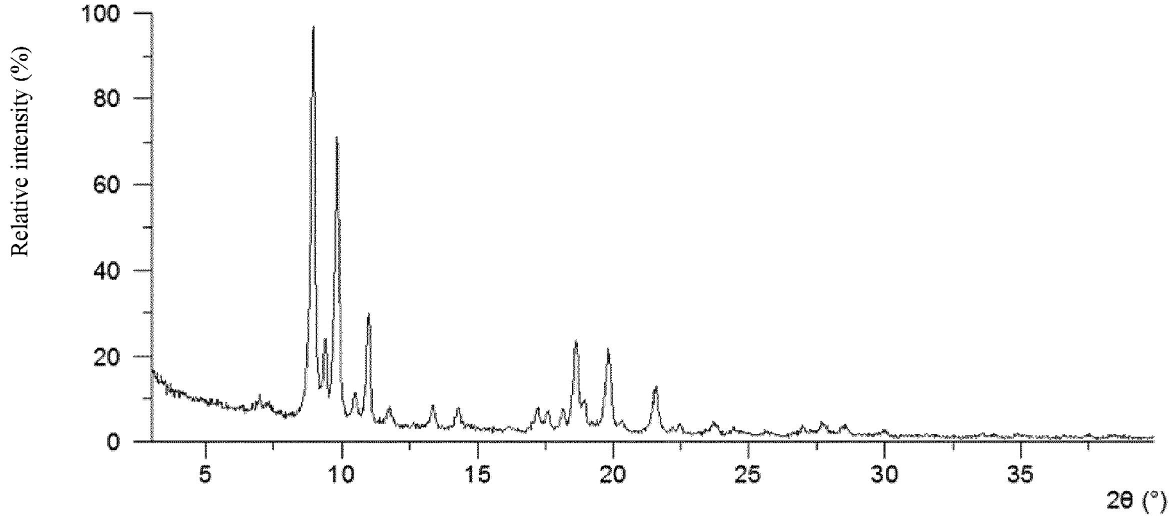
Relative intensity (%)
100
80
60
40
20
0
5
10
15
20
25
30
35
2θ (°)

FIG. 7

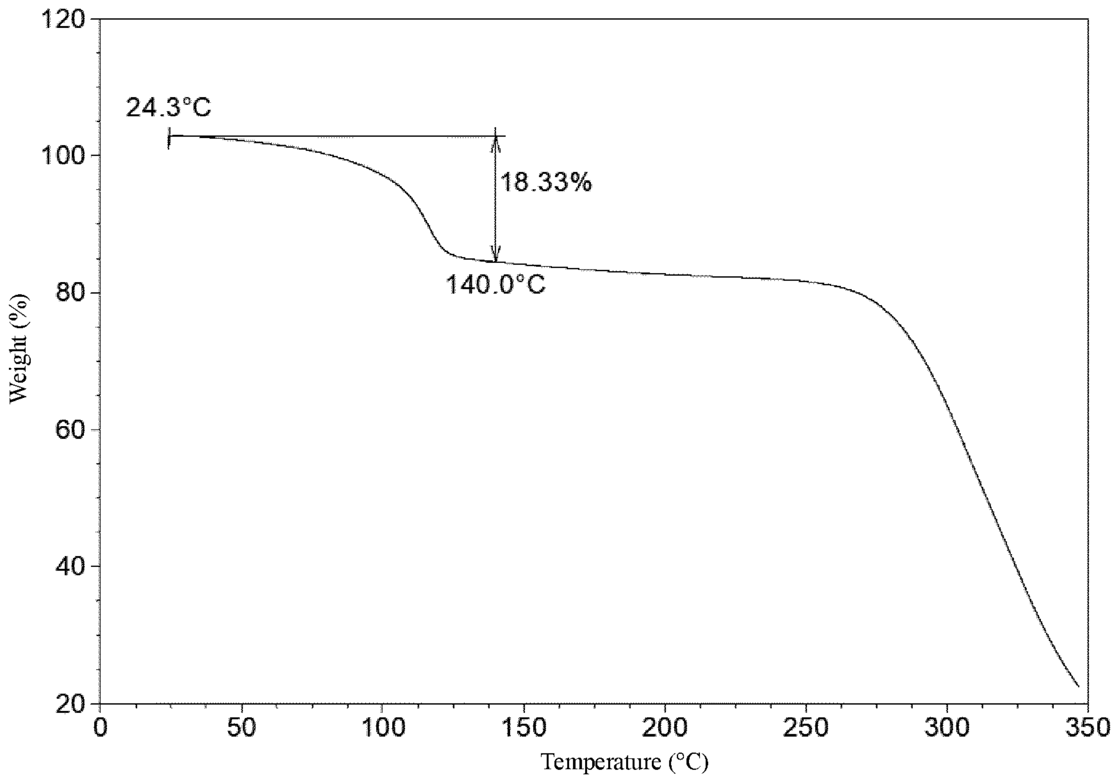
120
100
80
60
40
20
24.3°C
18.33%
140.0°C
Weight (%)
0
50
100
150
200
250
300
350
Temperature (°C)

FIG. 8

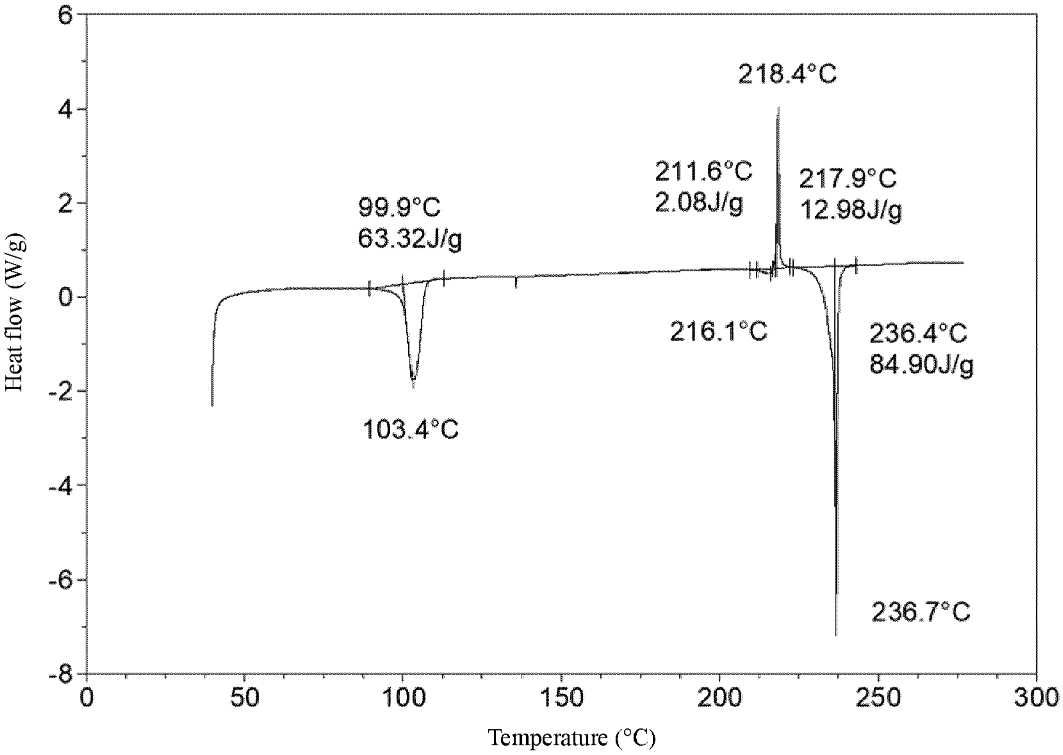
218.4°C
211.6°C
2.08J/g
217.9°C
12.98J/g
99.9°C
63.32J/g
216.1°C
236.4°C
84.90J/g
103.4°C
236.7°C
Heat flow (W/g)
Temperature (°C)

FIG. 9

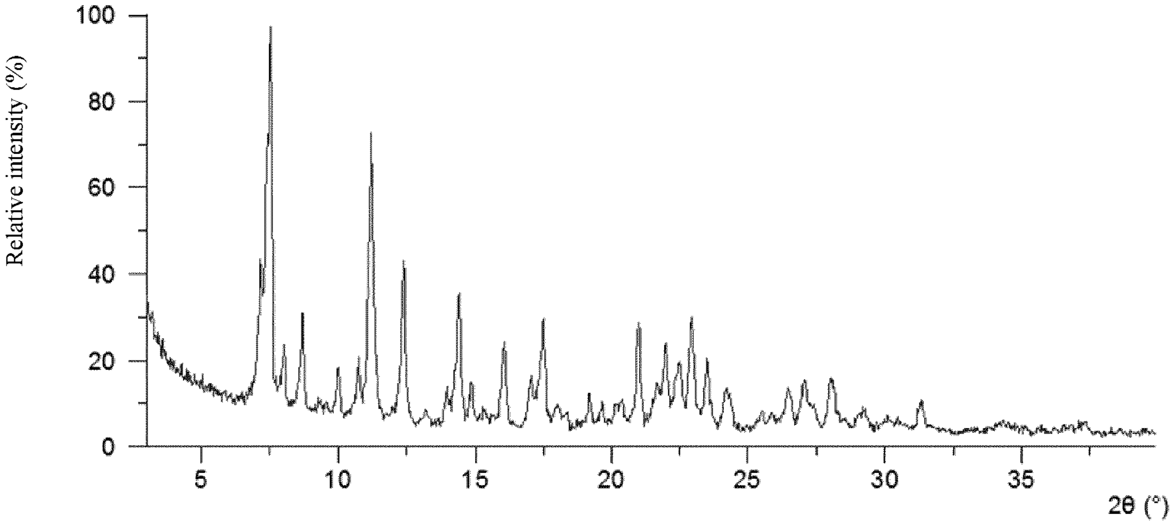
Relative intensity (%)
2θ (°)

FIG. 10

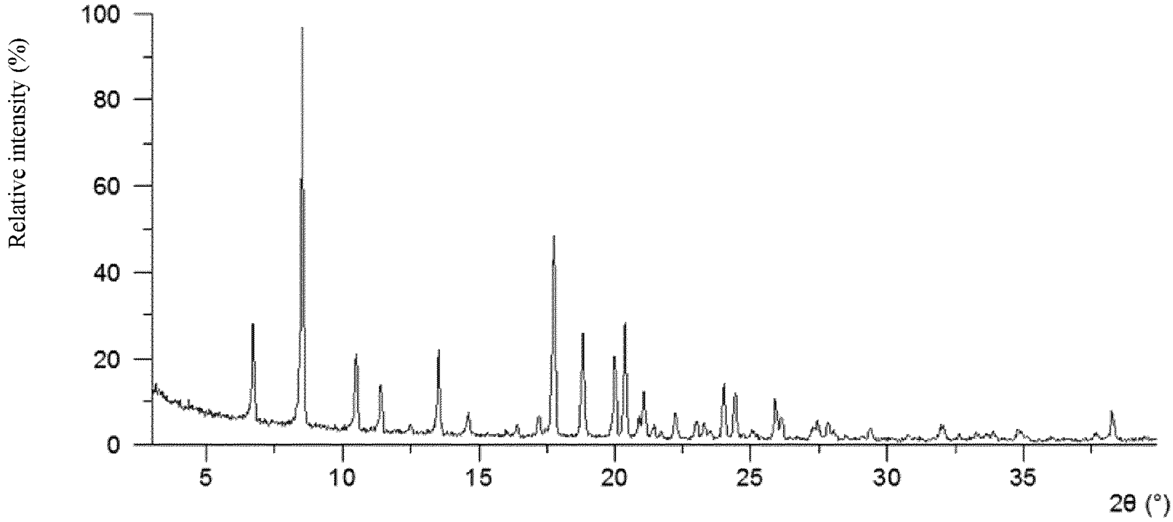
Relative intensity (%)
100
80
60
40
20
0
5
10
15
20
25
30
35
2θ (°)

FIG. 11

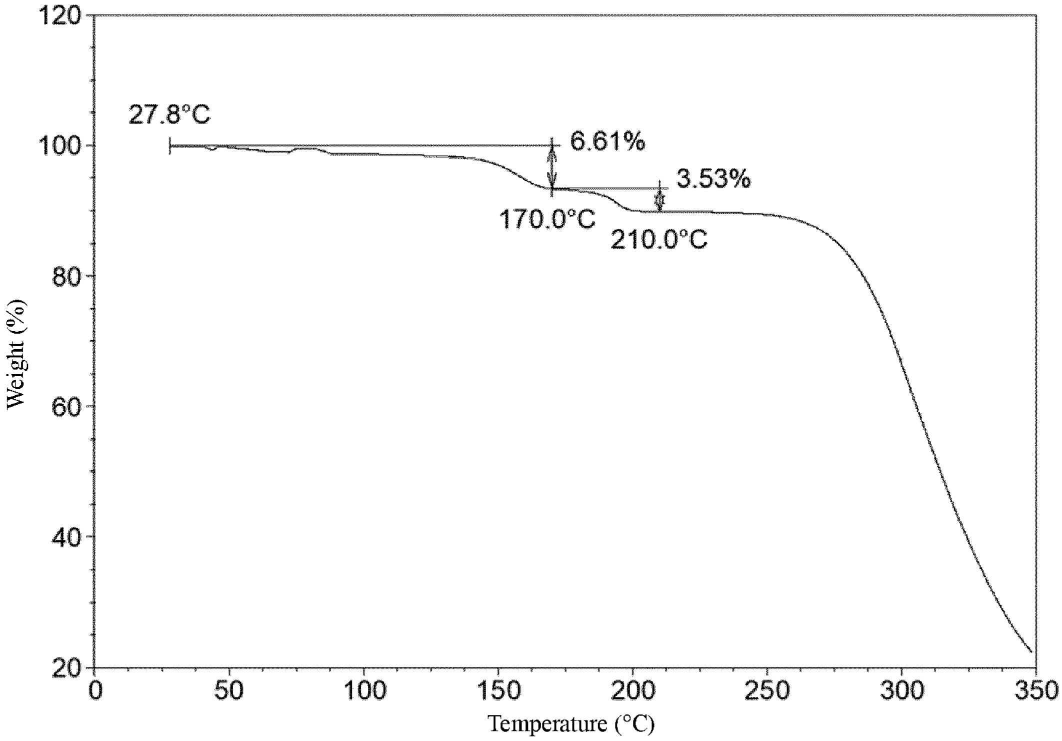
120
100
80
60
40
20
Weight (%)
27.8°C
6.61%
3.53%
170.0°C
210.0°C
0
50
100
150
200
250
300
350
Temperature (°C)

FIG. 12

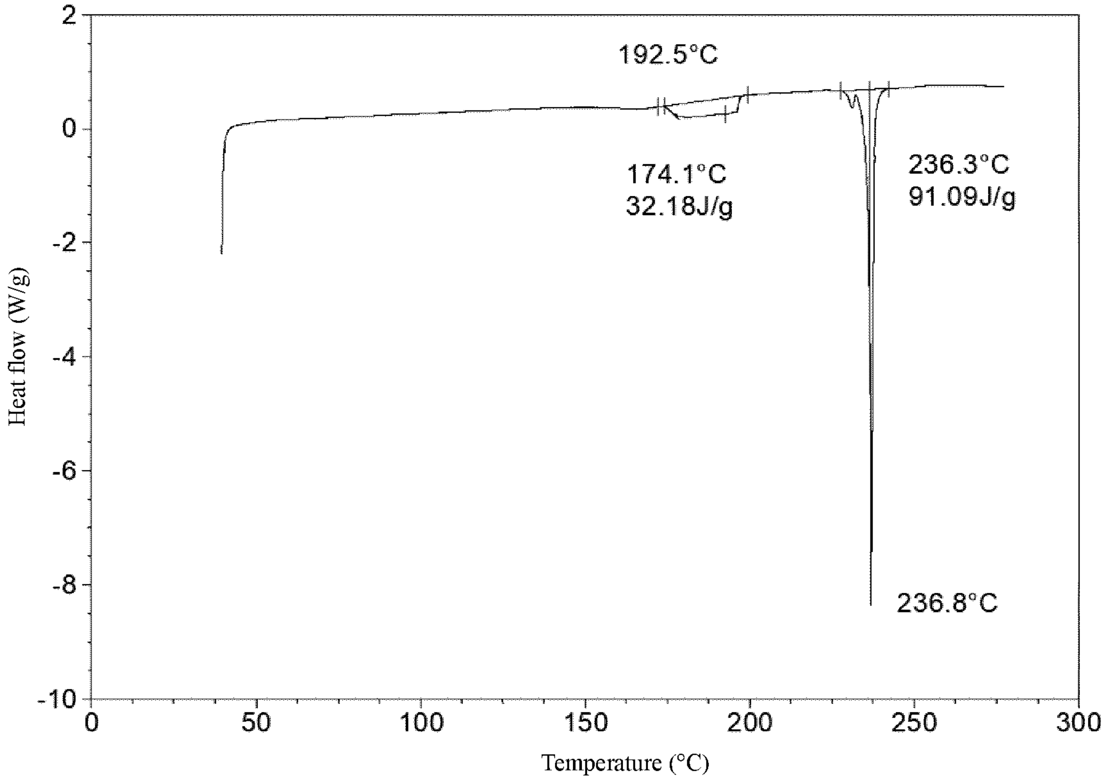
2
0
-2
-4
-6
-8
-10
Heat flow (W/g)
0
50
100
150
200
250
300
Temperature (°C)
192.5°C
174.1°C
32.18J/g
236.3°C
91.09J/g
236.8°C

FIG. 13

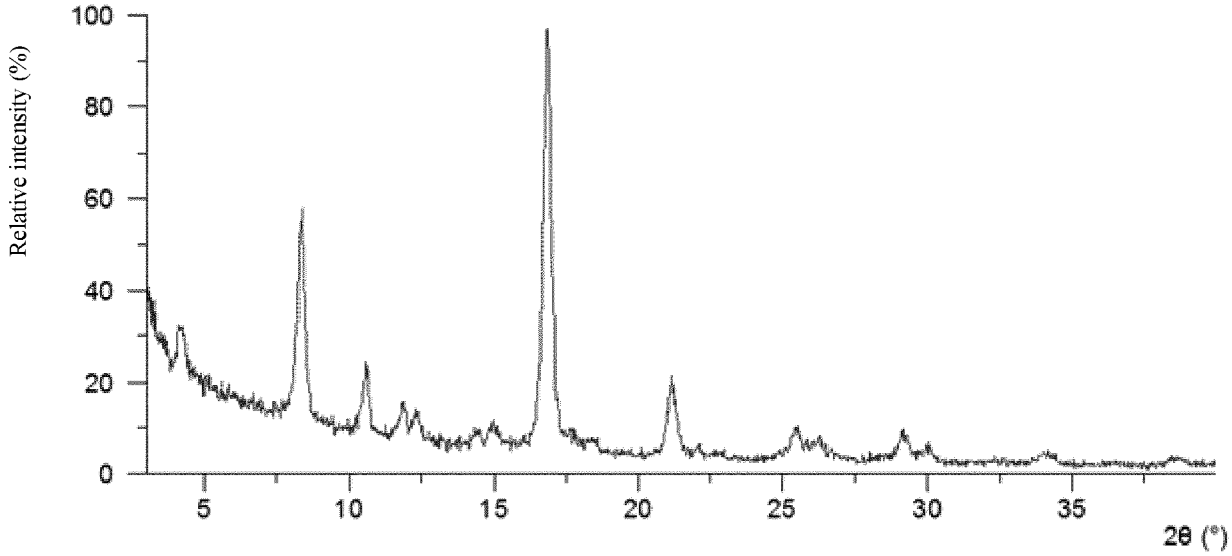
100
80
60
40
20
0
Relative intensity (%)
5
10
15
20
25
30
35
2θ (°)

FIG. 14

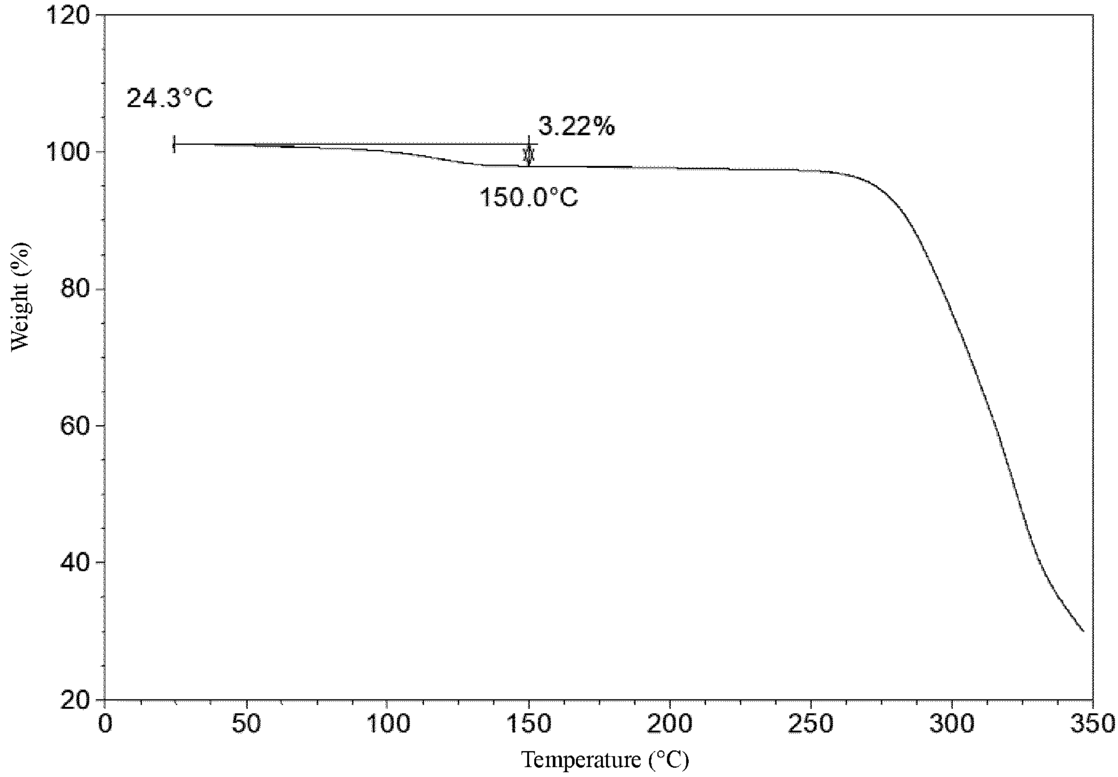
24.3°C
3.22%
150.0°C
Weight (%)
Temperature (°C)
120
100
80
60
40
20
0
50
100
150
200
250
300
350

FIG. 15

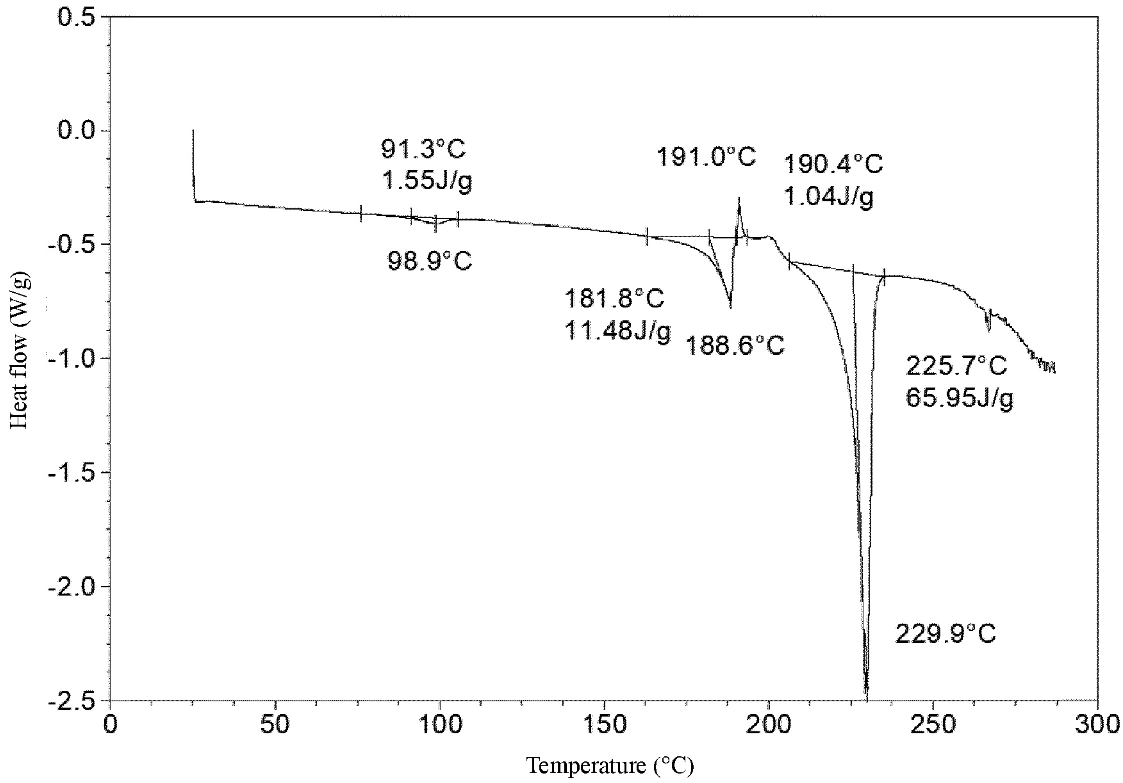
91.3°C
1.55J/g
98.9°C
191.0°C
190.4°C
1.04J/g
181.8°C
11.48J/g
188.6°C
225.7°C
65.95J/g
229.9°C
Heat flow (W/g)
Temperature (°C)
0.5
0.0
-0.5
-1.0
-1.5
-2.0
-2.5
0
50
100
150
200
250
300

FIG. 16

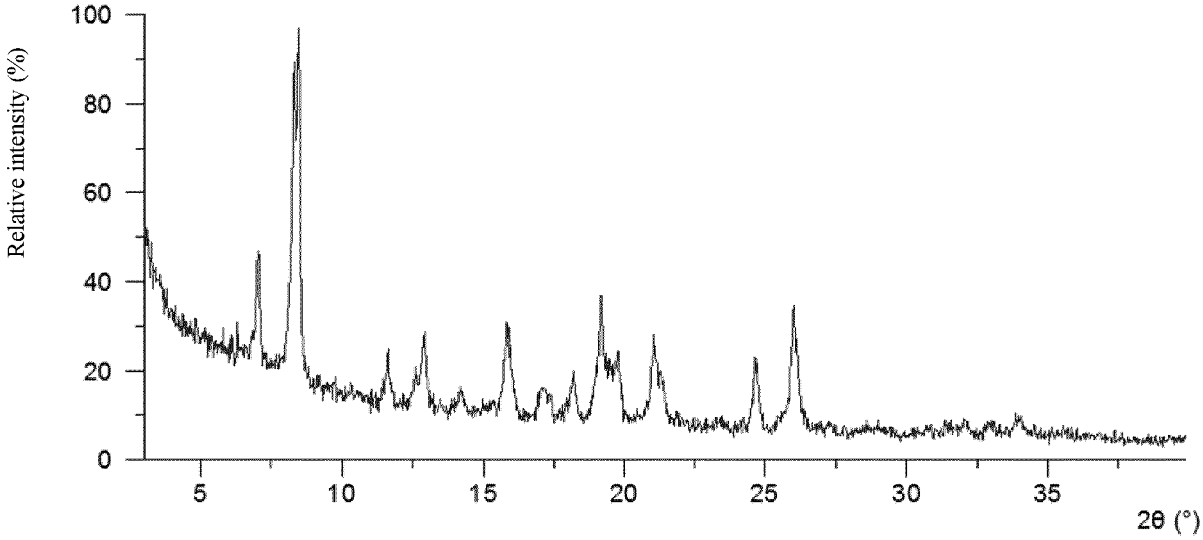
100
80
60
40
20
0
Relative intensity (%)
5
10
15
20
25
30
35
2θ (°)

FIG. 17

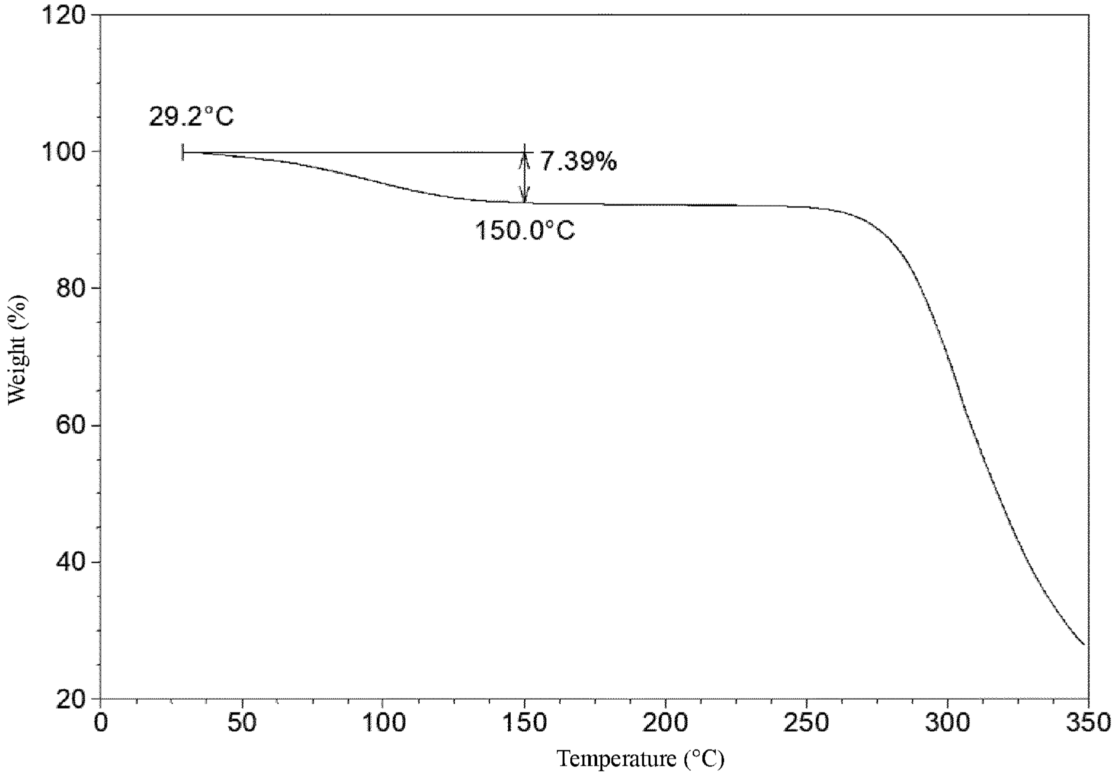
120
100
80
60
40
20
Weight (%)
29.2°C
7.39%
150.0°C
0
50
100
150
200
250
300
350
Temperature (°C)

FIG. 18

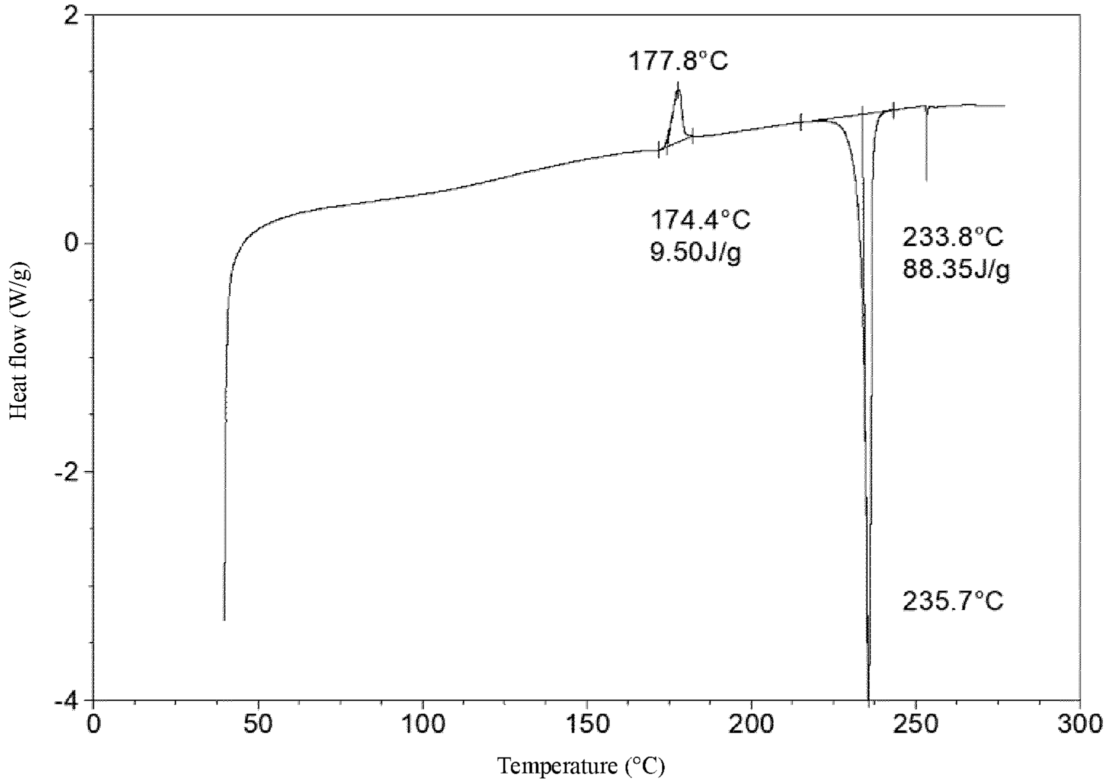
177.8°C
174.4°C
9.50J/g
233.8°C
88.35J/g
235.7°C
Heat flow (W/g)
Temperature (°C)
2
0
-2
-4
0
50
100
150
200
250
300

FIG. 19

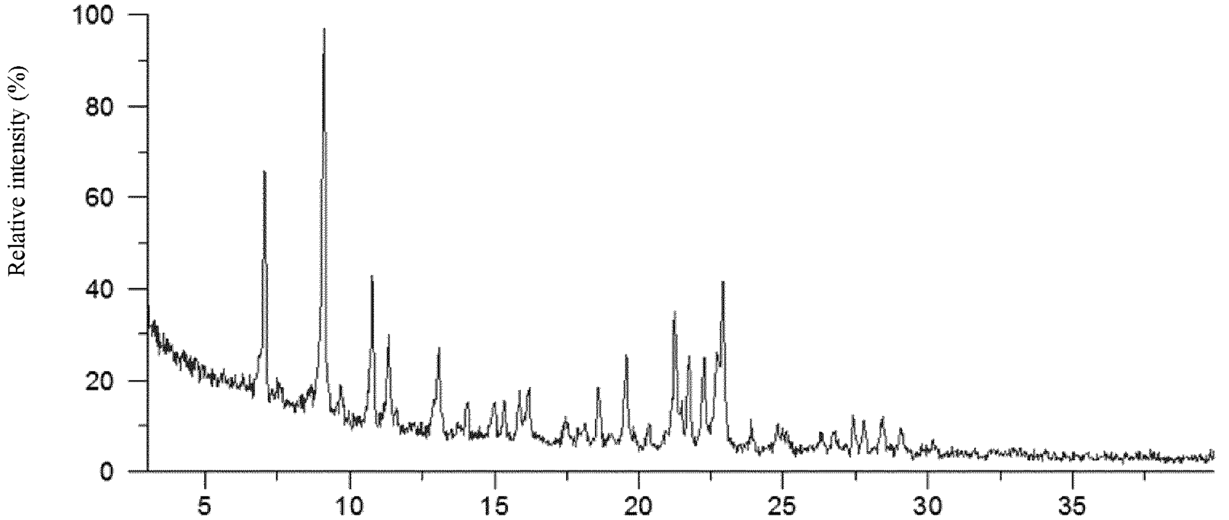
Relative intensity (%)
2θ (°)
100
80
60
40
20
0
5
10
15
20
25
30
35

FIG. 20

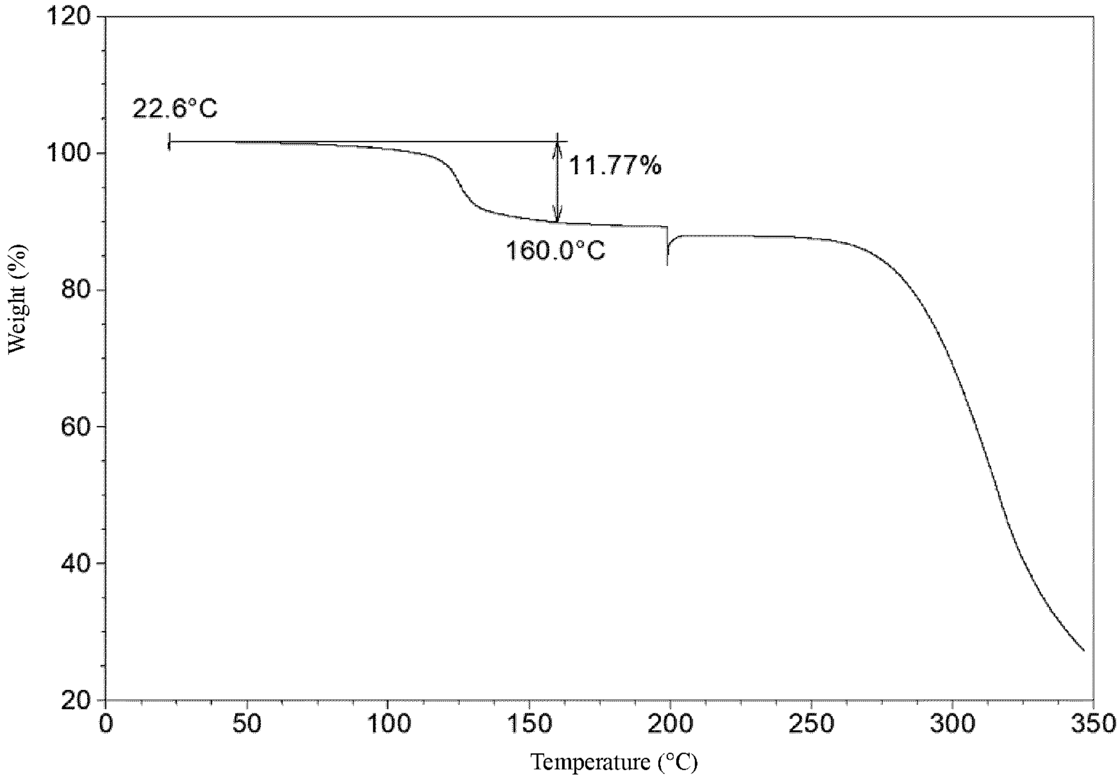
120
100
80
60
40
20
Weight (%)
22.6°C
11.77%
160.0°C
0
50
100
150
200
250
300
350
Temperature (°C)

FIG. 21

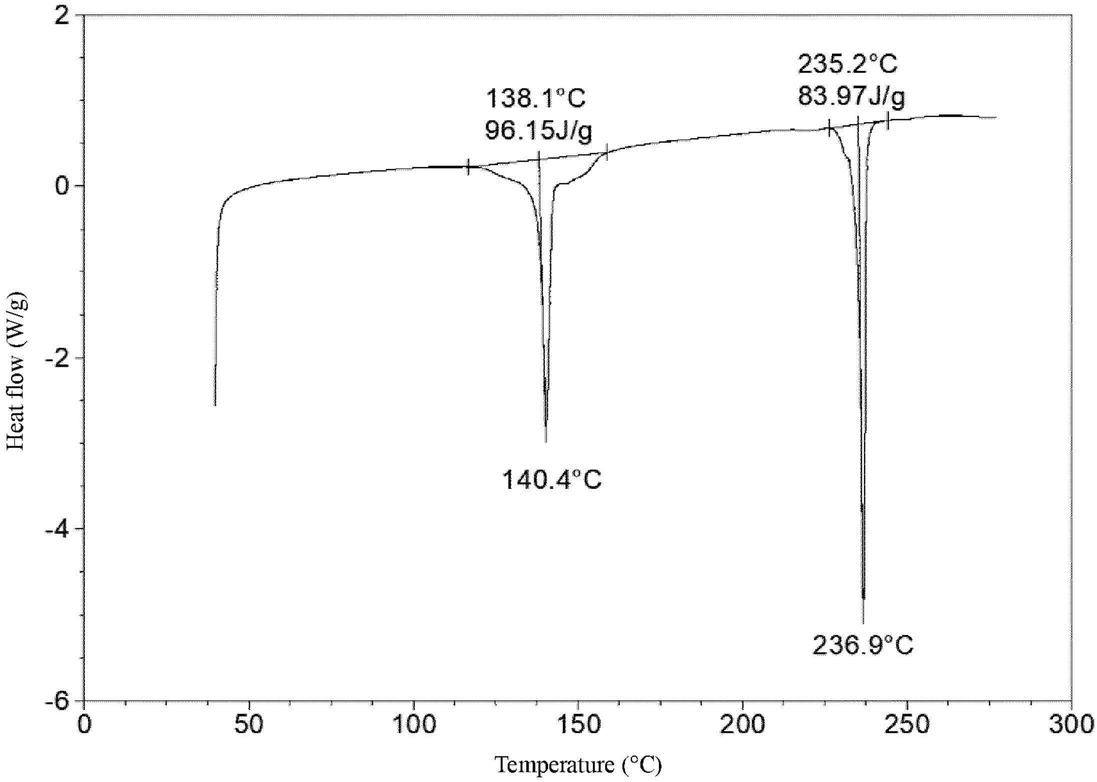
2
0
-2
-4
-6
Heat flow (W/g)
138.1°C
96.15J/g
140.4°C
235.2°C
83.97J/g
236.9°C
0
50
100
150
200
250
300
Temperature (°C)

FIG. 22

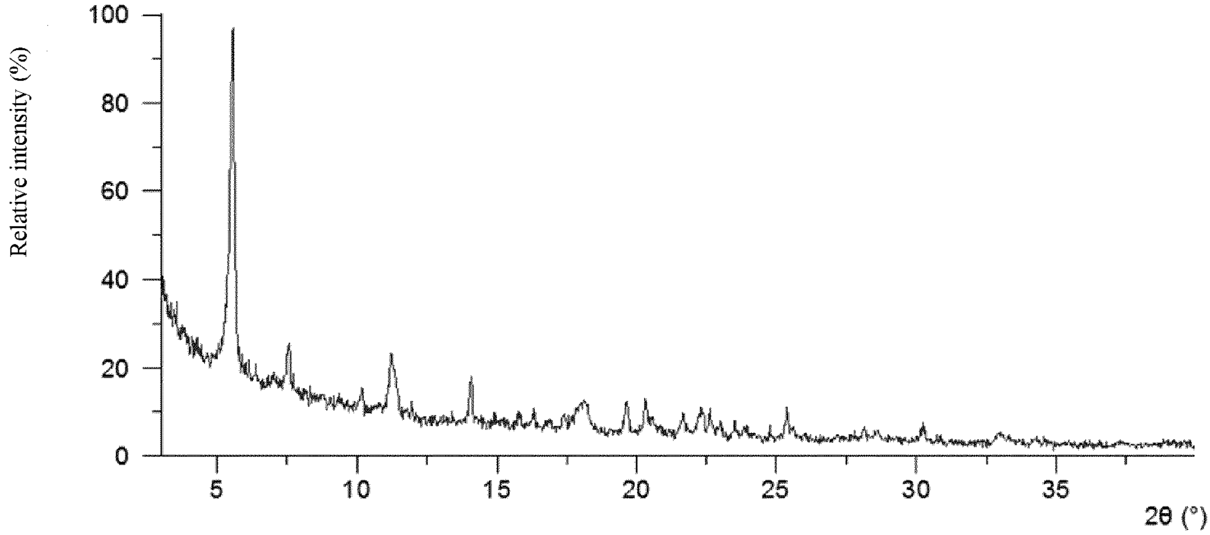

Relative intensity (%)
100
80
60
40
20
0
5
10
15
20
25
30
35
2θ (°)


FIG. 23

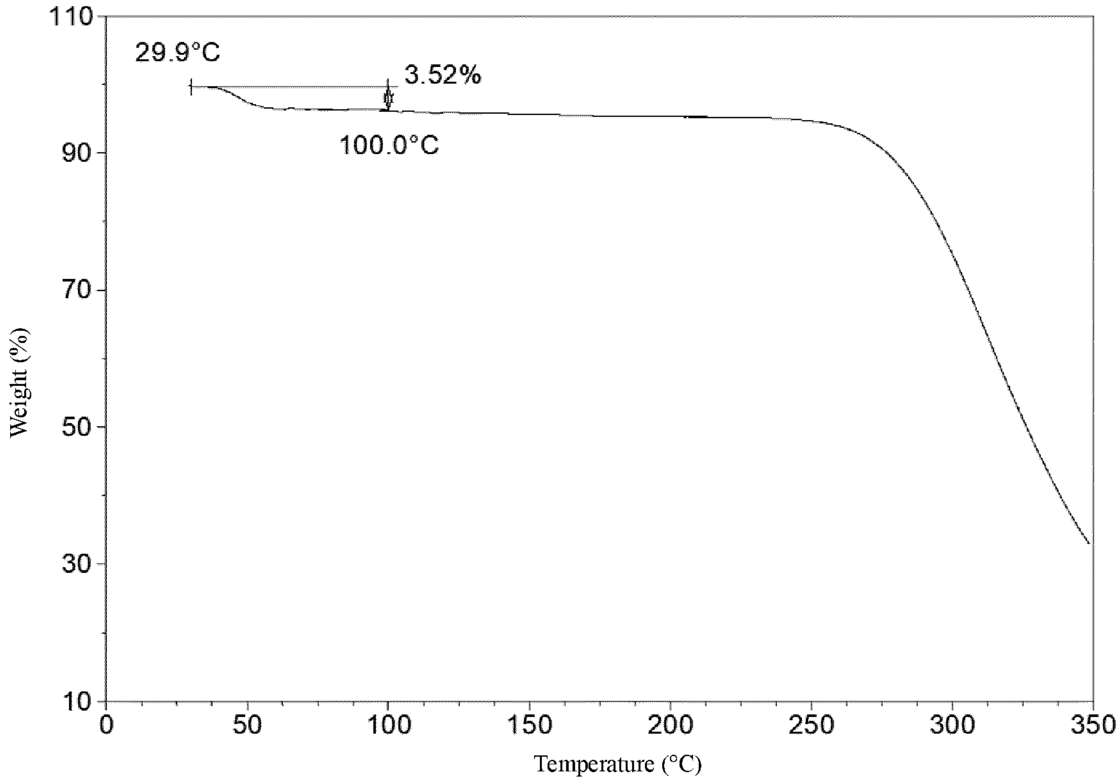

110
90
70
50
30
10
Weight (%)
29.9°C
3.52%
100.0°C
0
50
100
150
200
250
300
350
Temperature (°C)


FIG. 24

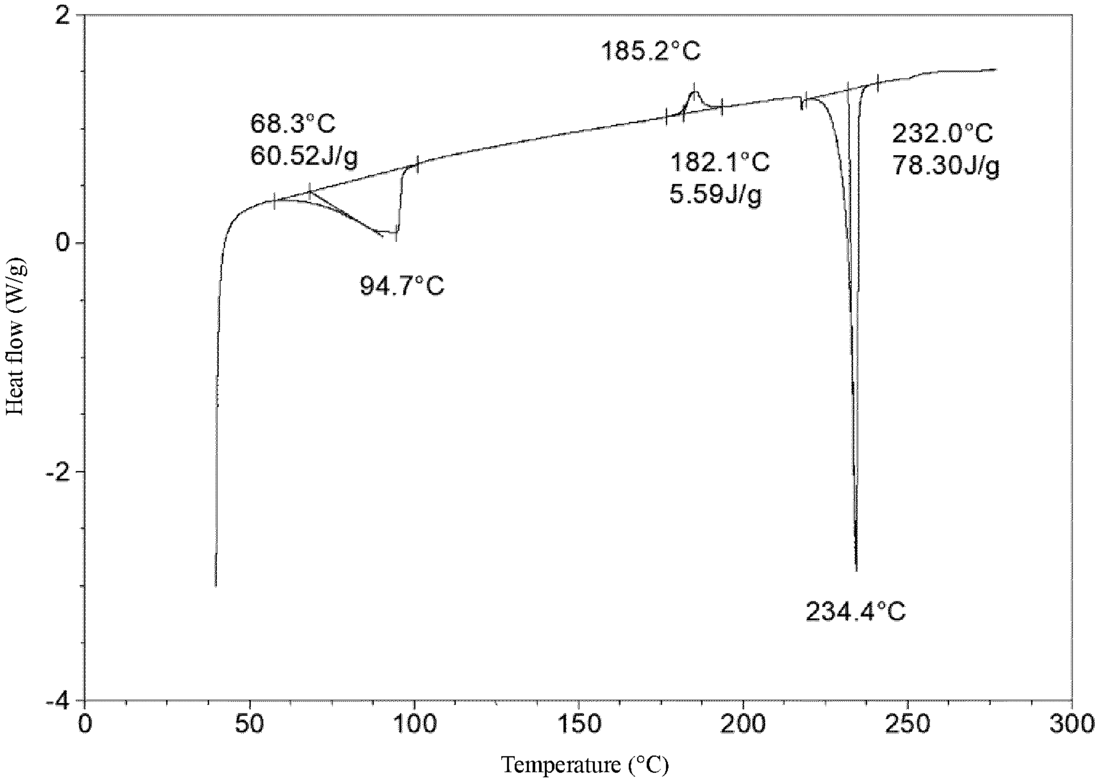
185.2°C
68.3°C
60.52J/g
182.1°C
5.59J/g
232.0°C
78.30J/g
94.7°C
234.4°C
Heat flow (W/g)
Temperature (°C)
2
0
-2
-4
0
50
100
150
200
250
300

FIG. 25

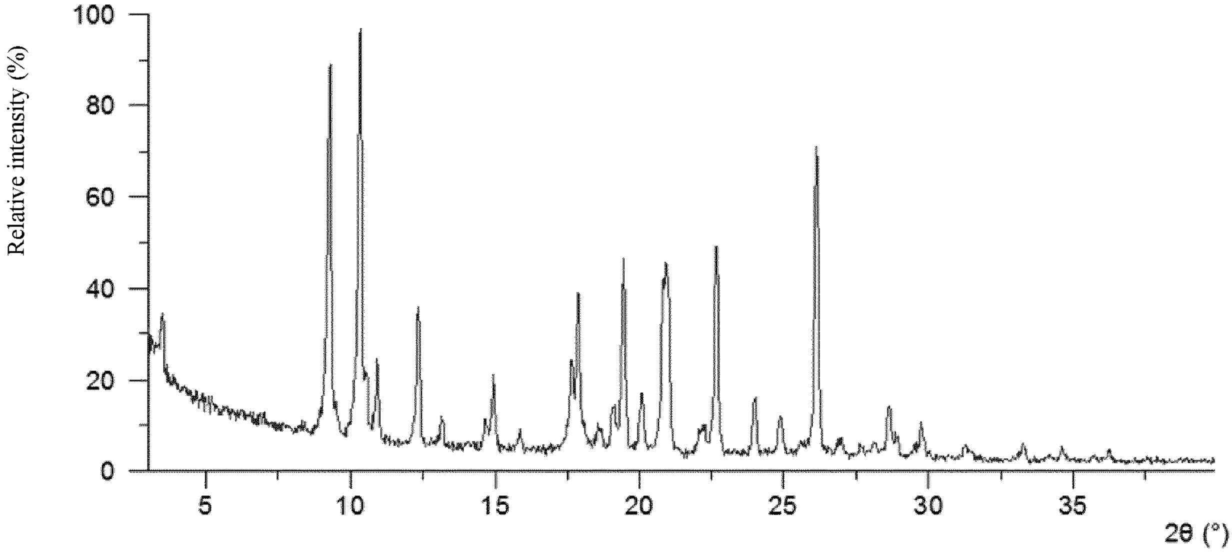
Relative intensity (%)
100
80
60
40
20
0
5
10
15
20
25
30
35
2θ (°)

FIG. 26

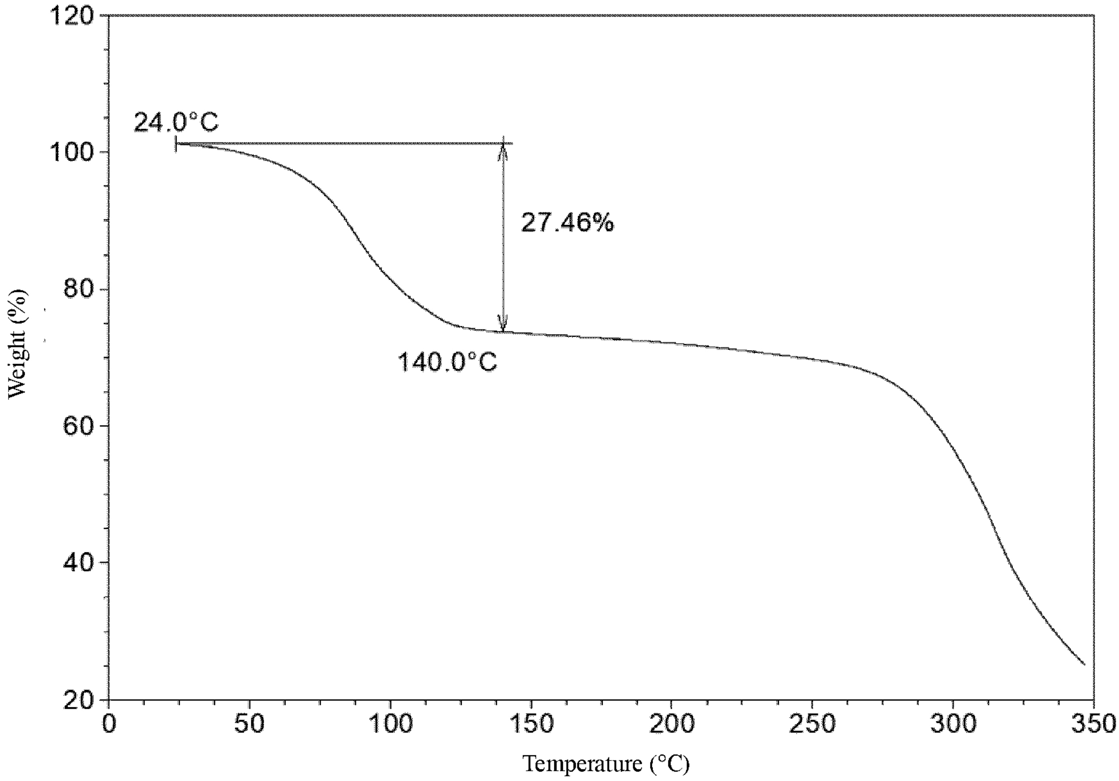
24.0°C
27.46%
140.0°C
Weight (%)
Temperature (°C)
120
100
80
60
40
20
0
50
100
150
200
250
300
350

FIG. 27

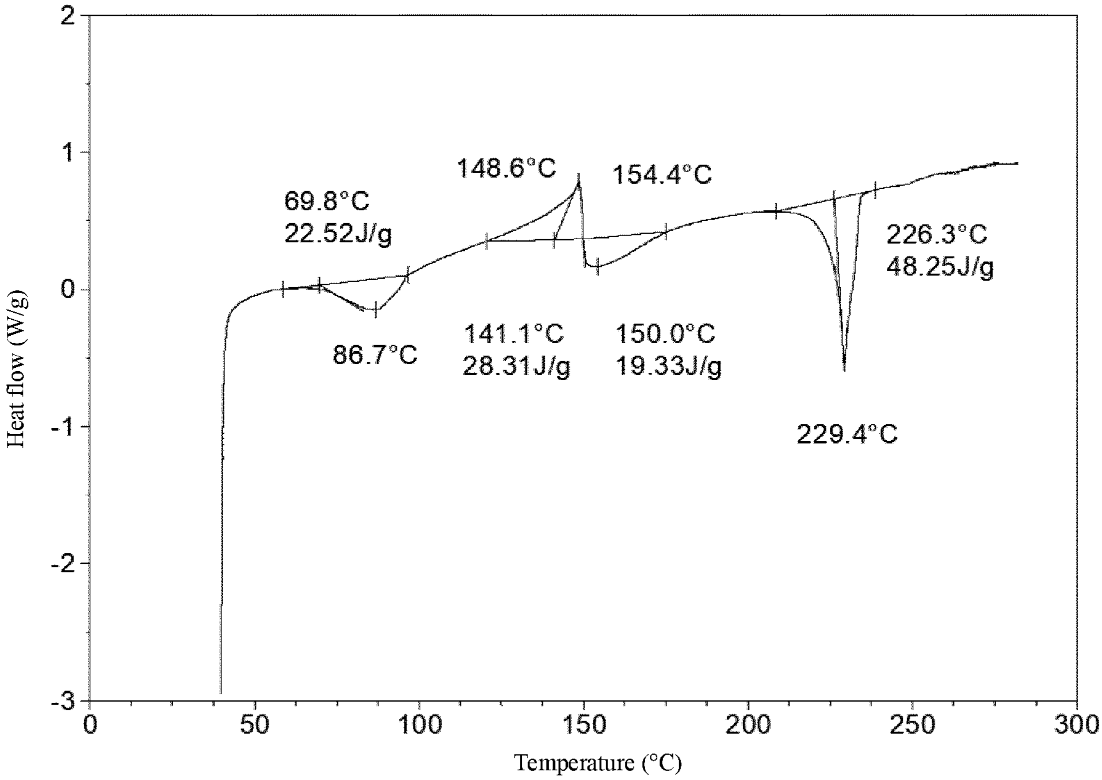
148.6°C
154.4°C
69.8°C
22.52J/g
226.3°C
48.25J/g
141.1°C
28.31J/g
150.0°C
19.33J/g
86.7°C
229.4°C
Heat flow (W/g)
Temperature (°C)
2
1
0
-1
-2
-3
0
50
100
150
200
250
300

FIG. 28

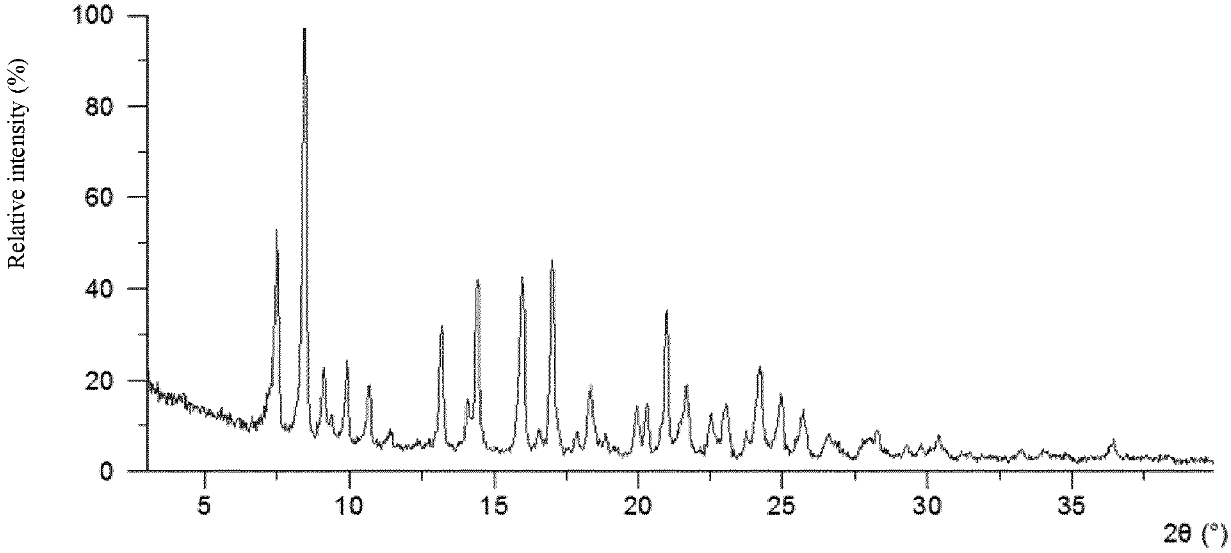
Relative intensity (%)
100
80
60
40
20
0
5
10
15
20
25
30
35
2θ (°)

FIG. 29

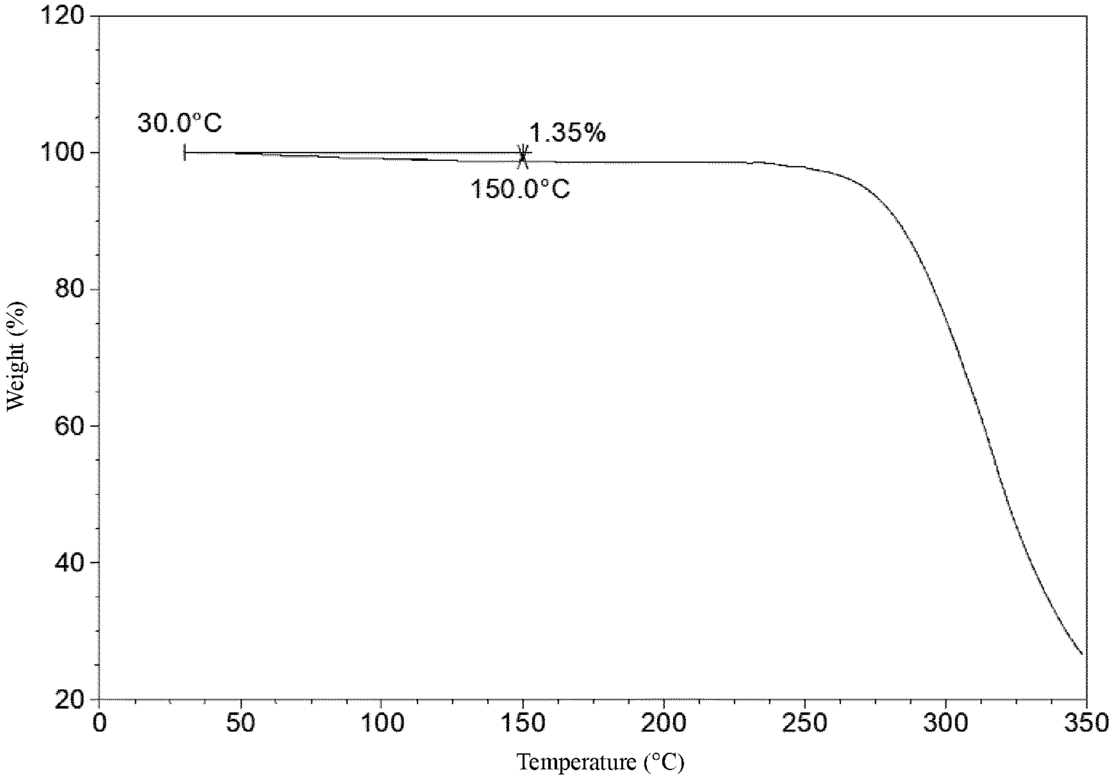
120
100
80
60
40
20
30.0°C
1.35%
150.0°C
Weight (%)
0
50
100
150
200
250
300
350
Temperature (°C)

FIG. 30

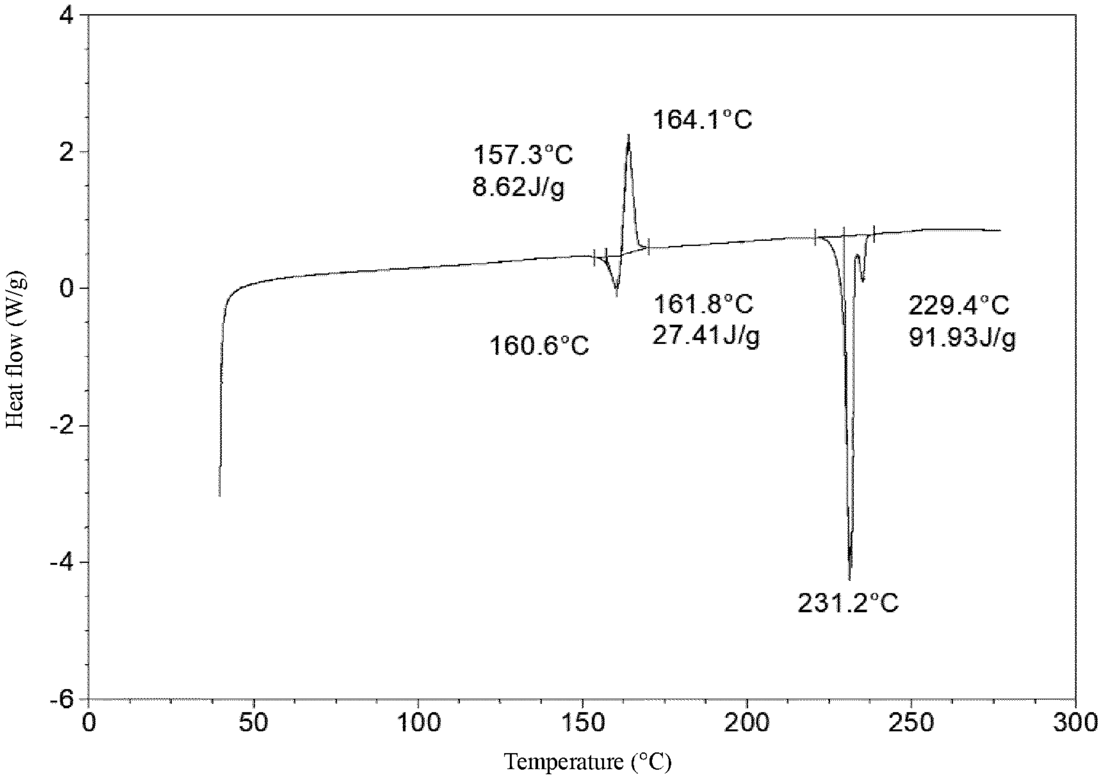

4
2
0
-2
-4
-6
0
50
100
150
200
250
300
Heat flow (W/g)
Temperature (°C)
164.1°C
157.3°C
8.62J/g
161.8°C
27.41J/g
160.6°C
229.4°C
91.93J/g
231.2°C


FIG. 31

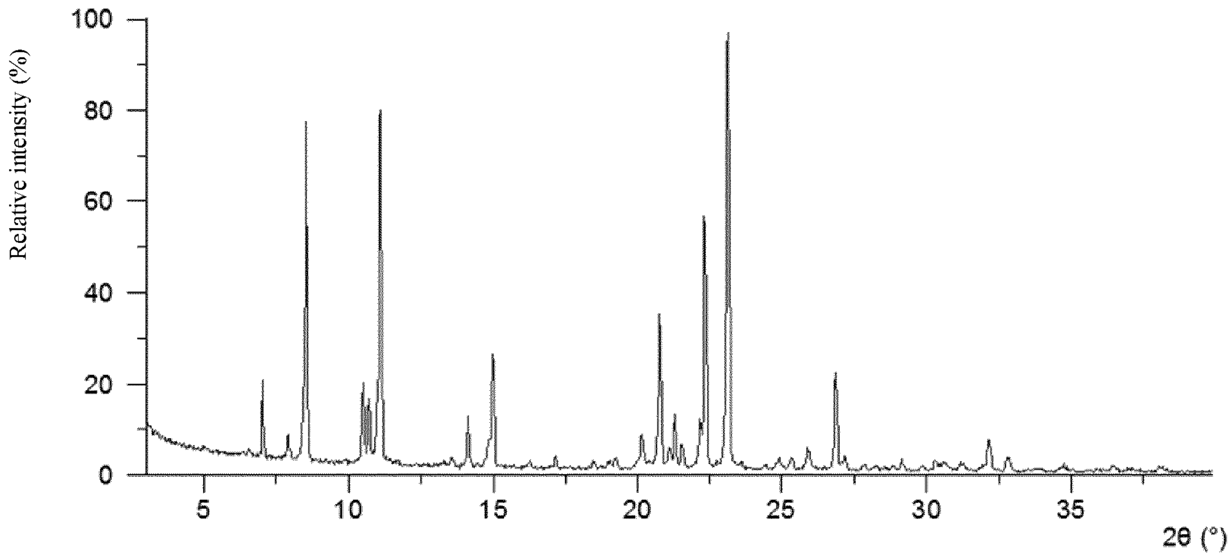

100
80
60
40
20
0
5
10
15
20
25
30
35
Relative intensity (%)
2θ (°)


FIG. 32

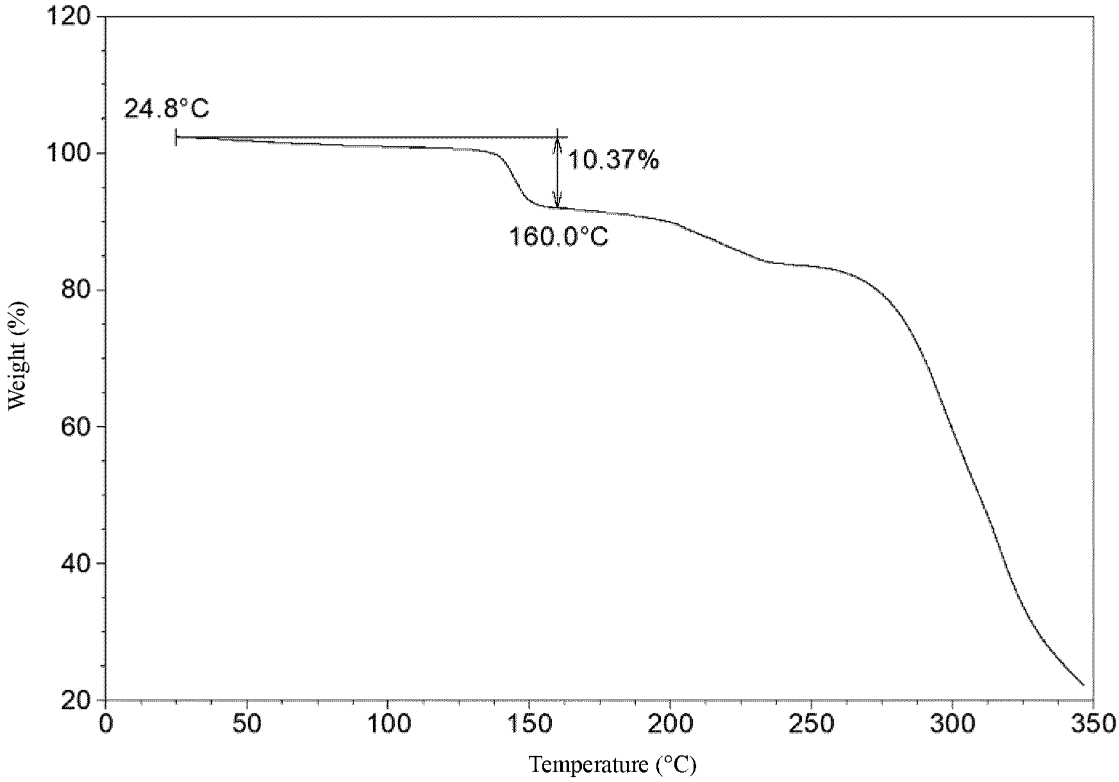
120
100
80
60
40
20
Weight (%)
24.8°C
10.37%
160.0°C
0
50
100
150
200
250
300
350
Temperature (°C)

FIG. 33

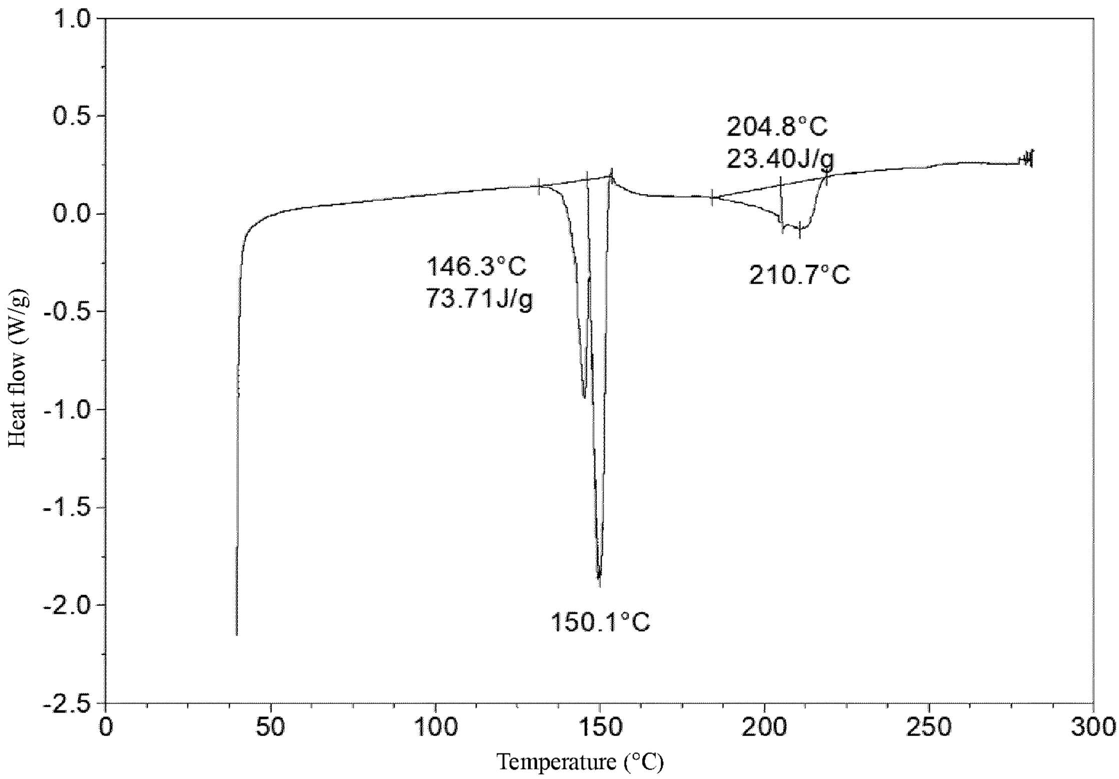
1.0
0.5
0.0
-0.5
-1.0
-1.5
-2.0
-2.5
Heat flow (W/g)
204.8°C
23.40J/g
146.3°C
73.71J/g
210.7°C
150.1°C
0
50
100
150
200
250
300
Temperature (°C)

FIG. 34

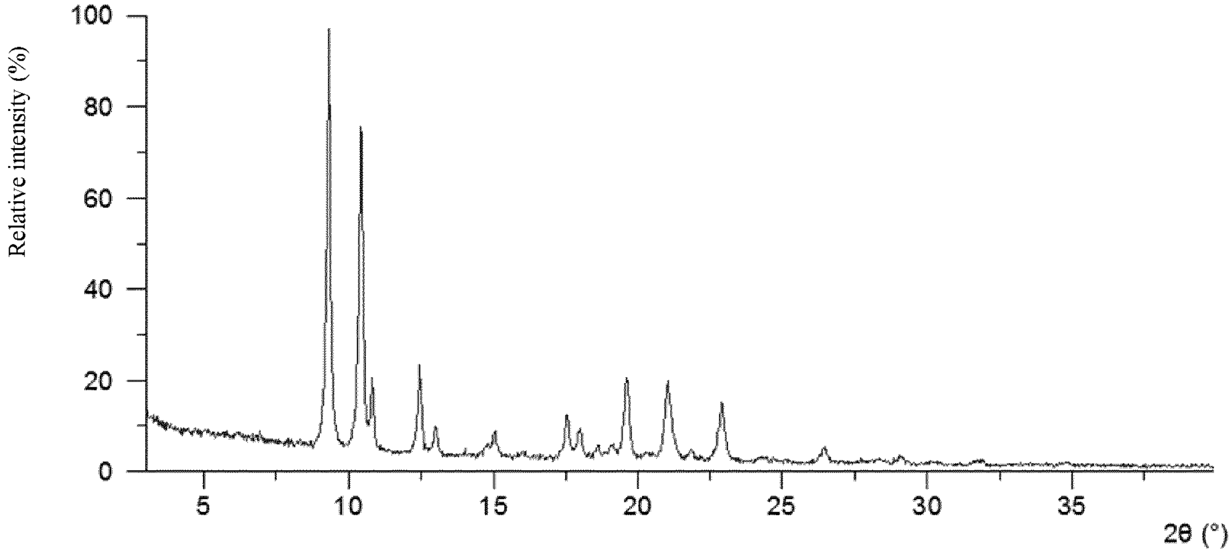
100
80
60
40
20
0
Relative intensity (%)
5
10
15
20
25
30
35
2θ (°)

FIG. 35

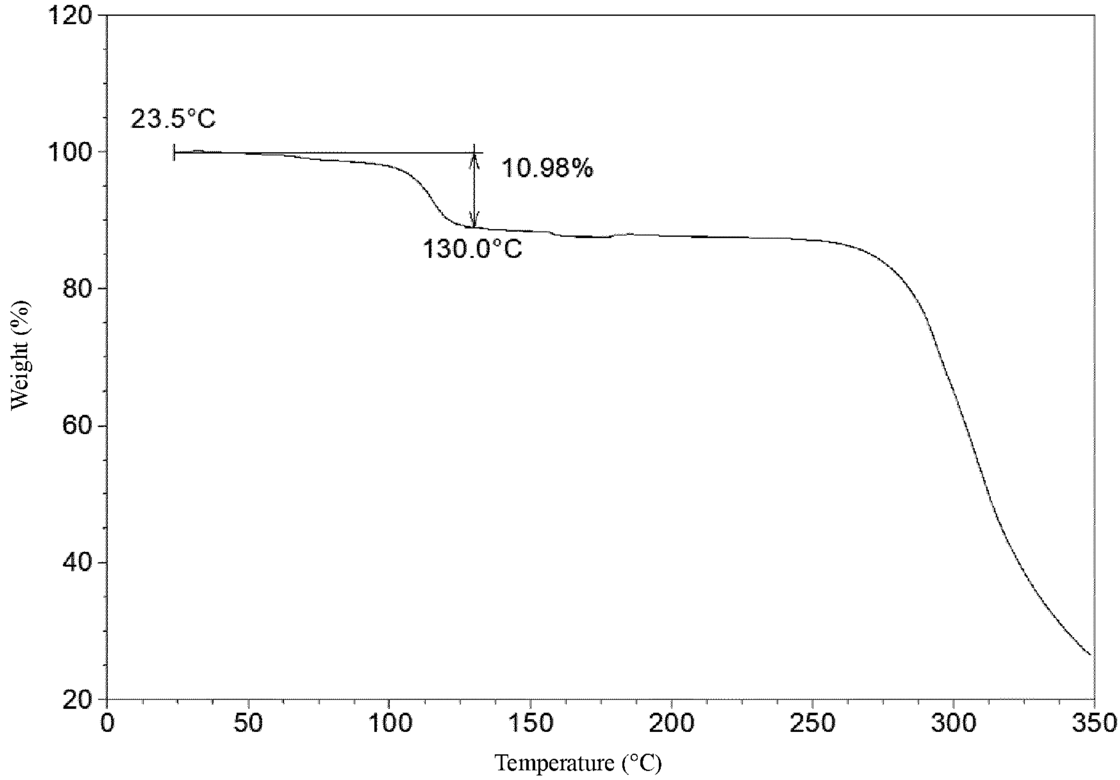
120
100
80
60
40
20
Weight (%)
23.5°C
10.98%
130.0°C
0
50
100
150
200
250
300
350
Temperature (°C)

FIG. 36

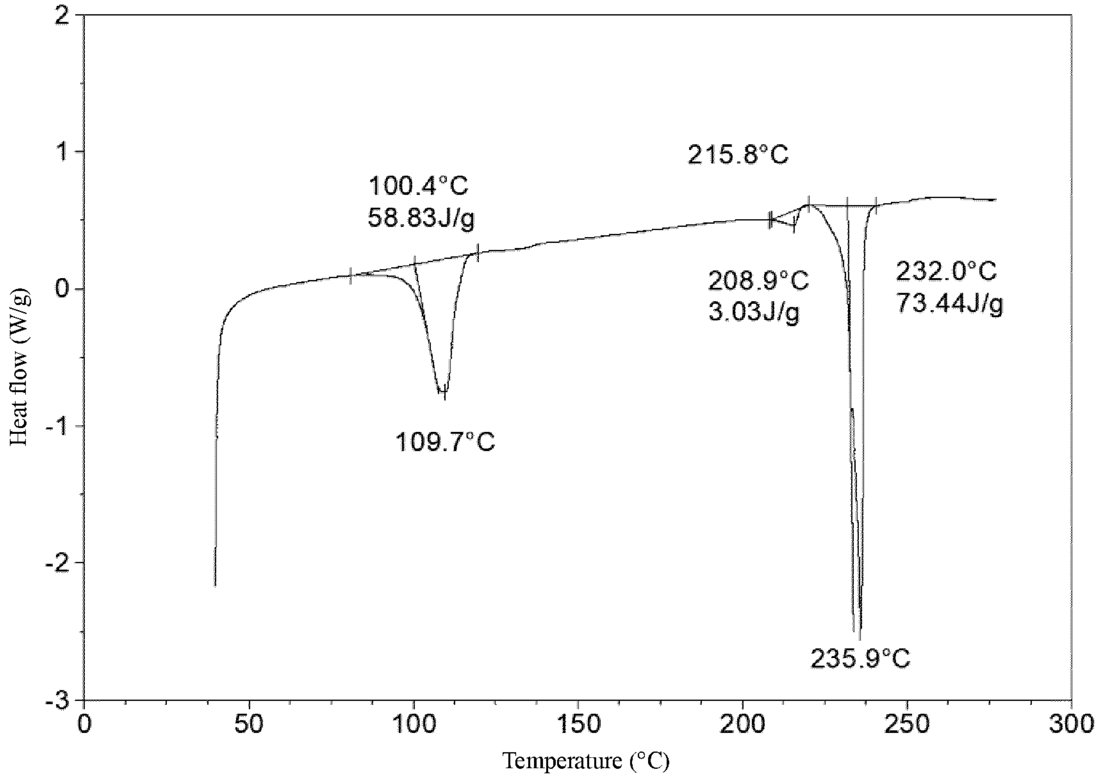
100.4°C
58.83J/g
215.8°C
208.9°C
3.03J/g
232.0°C
73.44J/g
109.7°C
235.9°C
Heat flow (W/g)
Temperature (°C)

FIG. 37

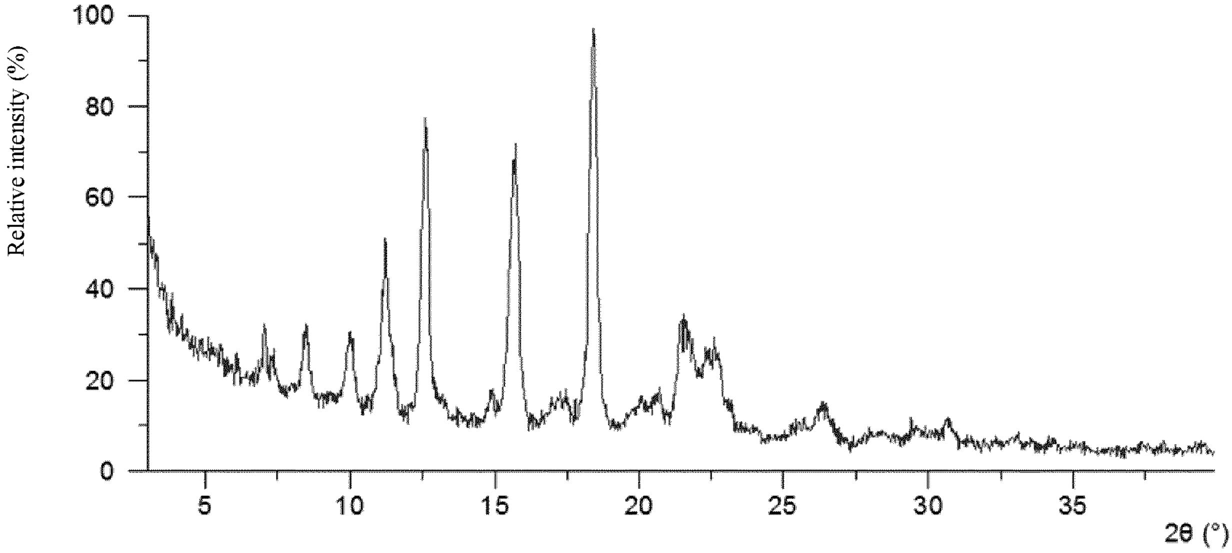
Relative intensity (%)
2θ (°)

FIG. 38

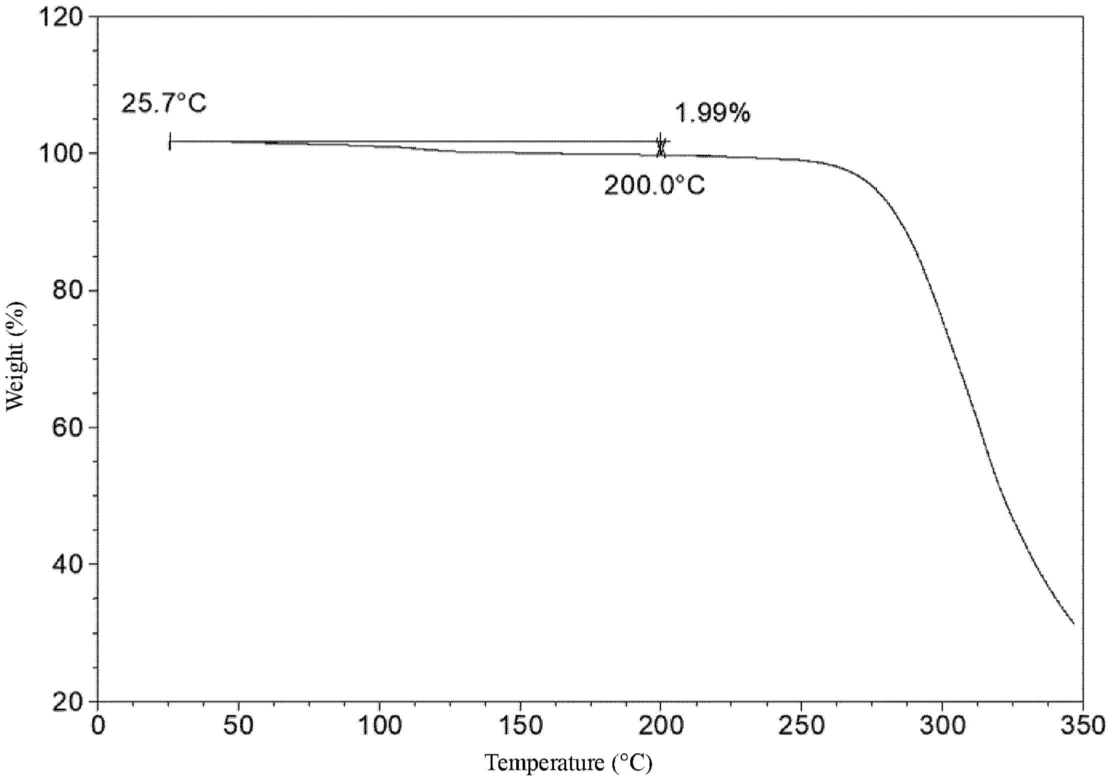
25.7°C
1.99%
200.0°C
Weight (%)
Temperature (°C)
120
100
80
60
40
20
0
50
100
150
200
250
300
350

FIG. 39

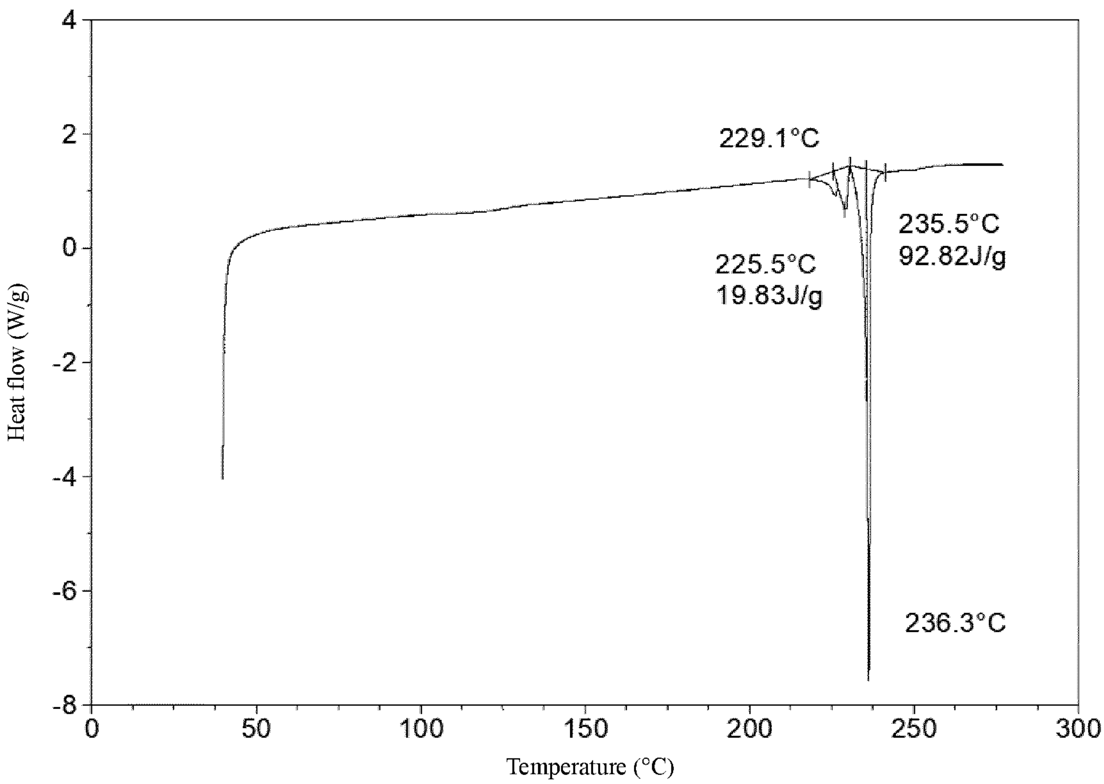
229.1°C
235.5°C
92.82J/g
225.5°C
19.83J/g
236.3°C
Heat flow (W/g)
Temperature (°C)
4
2
0
-2
-4
-6
-8
0
50
100
150
200
250
300

FIG. 40

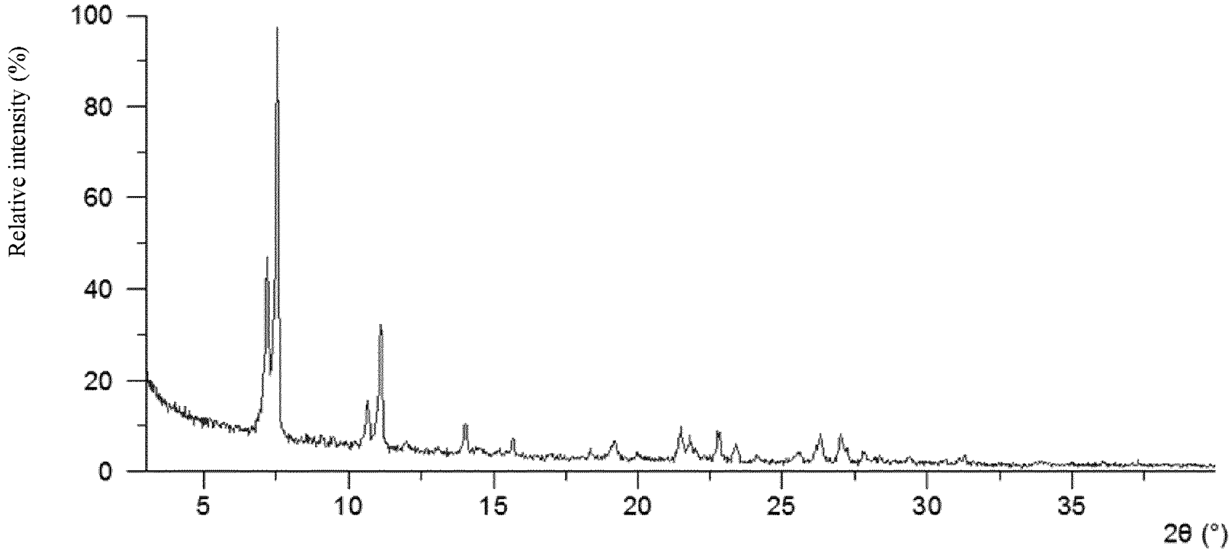
Relative intensity (%)
100
80
60
40
20
0
5
10
15
20
25
30
35
2θ (°)

FIG. 41

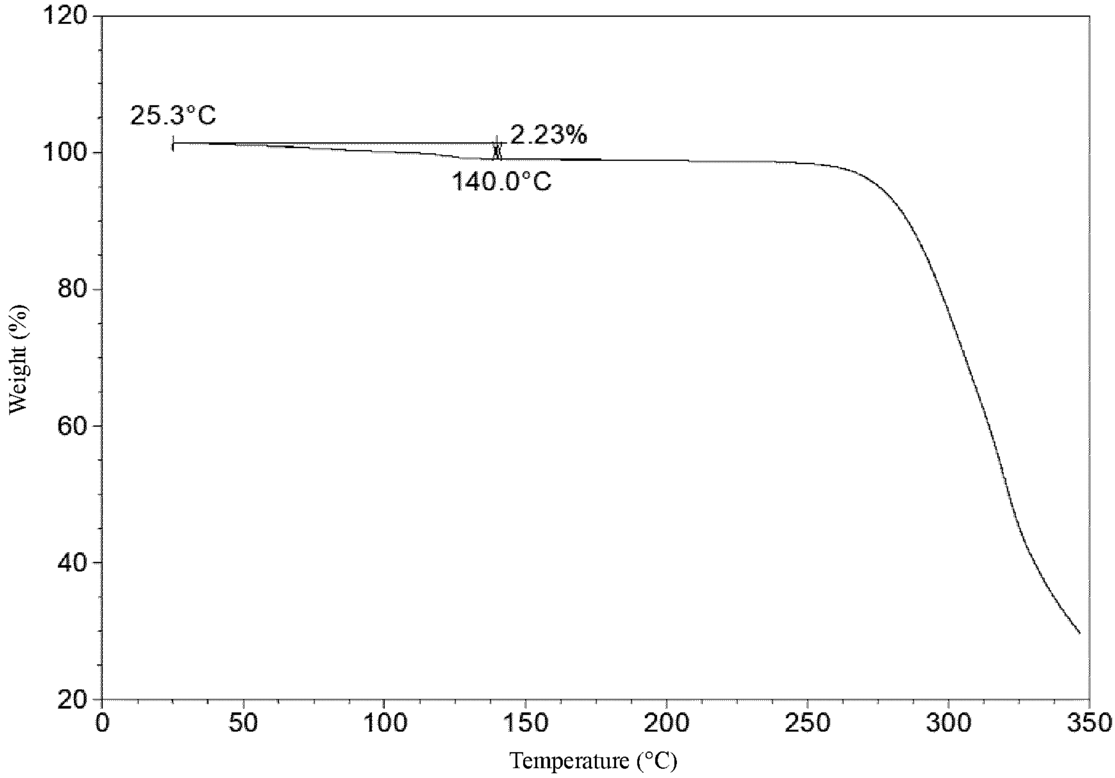
120
100
80
60
40
20
25.3°C
2.23%
140.0°C
Weight (%)
0
50
100
150
200
250
300
350
Temperature (°C)

FIG. 42

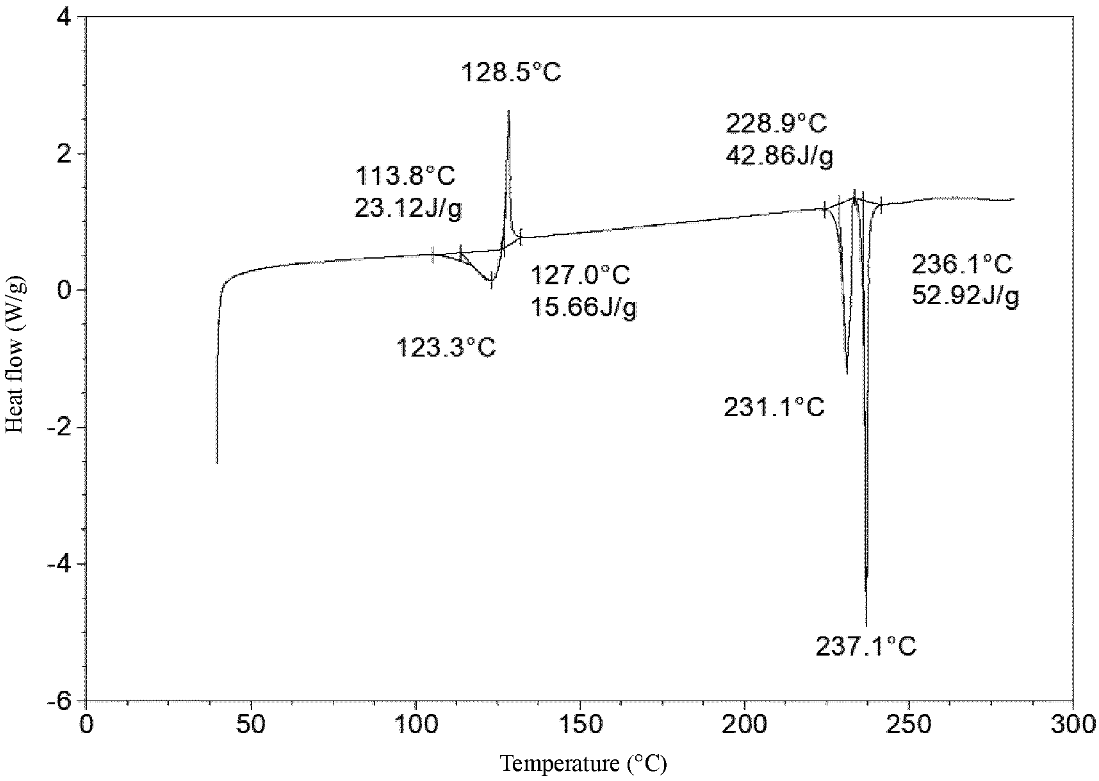


FIG. 43

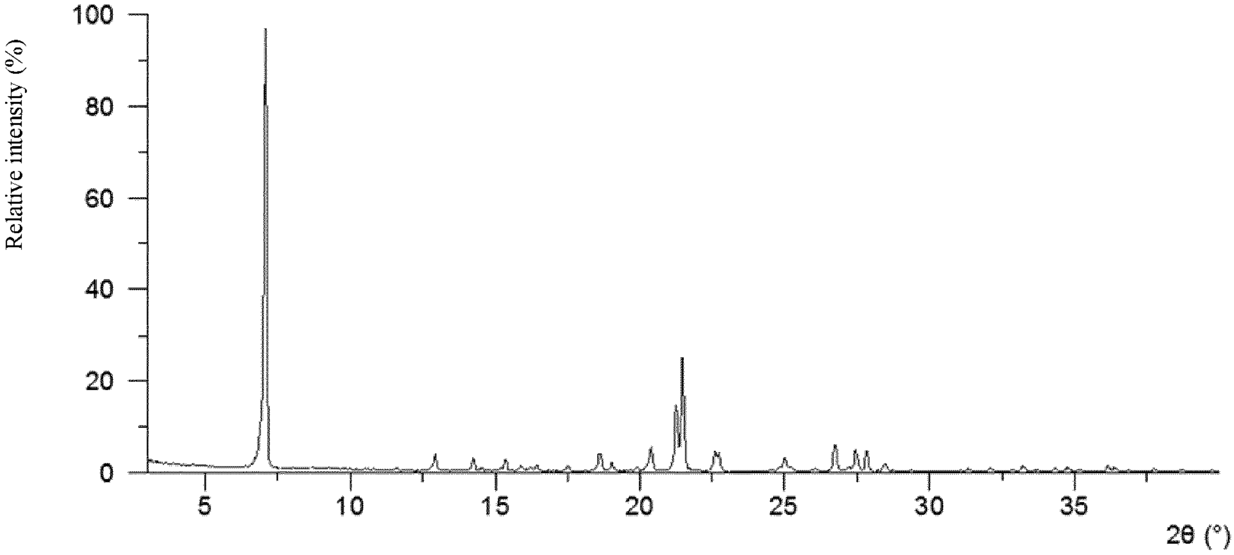


FIG. 44A

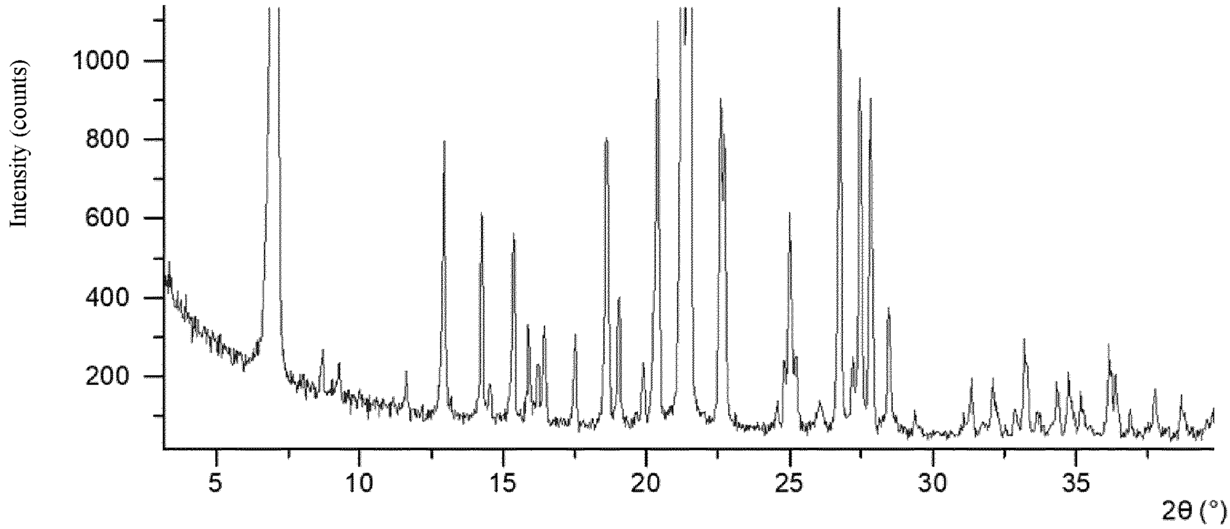


FIG. 44B

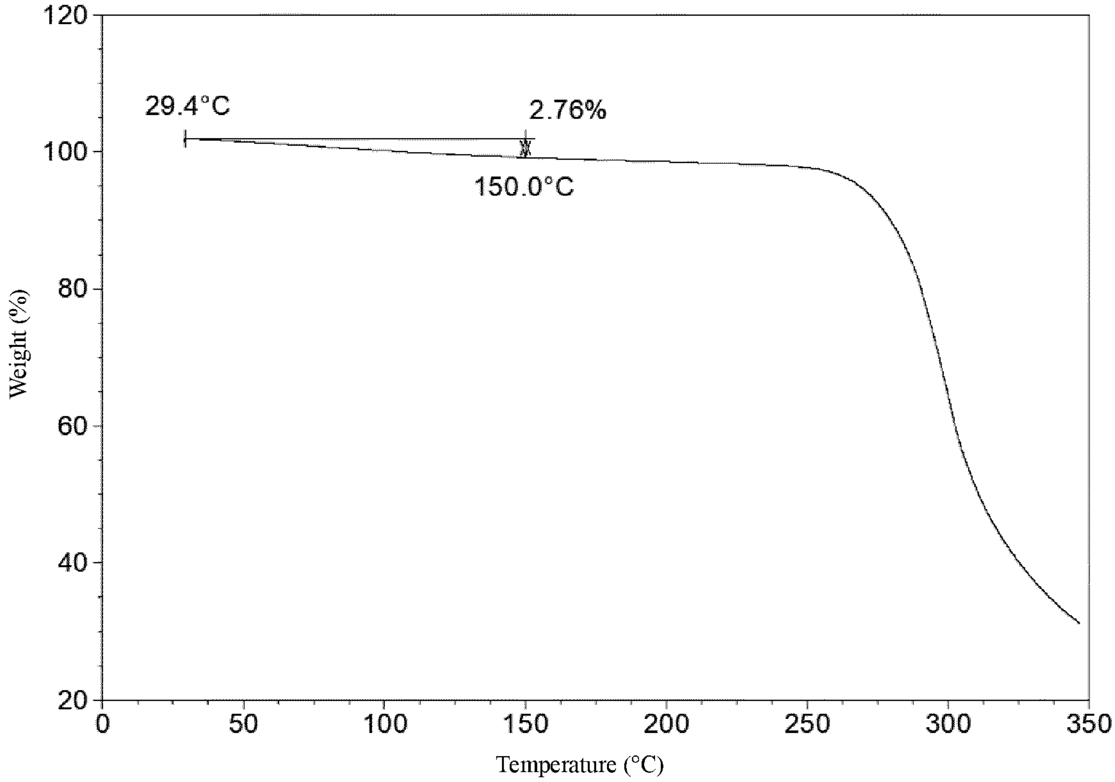


FIG. 45

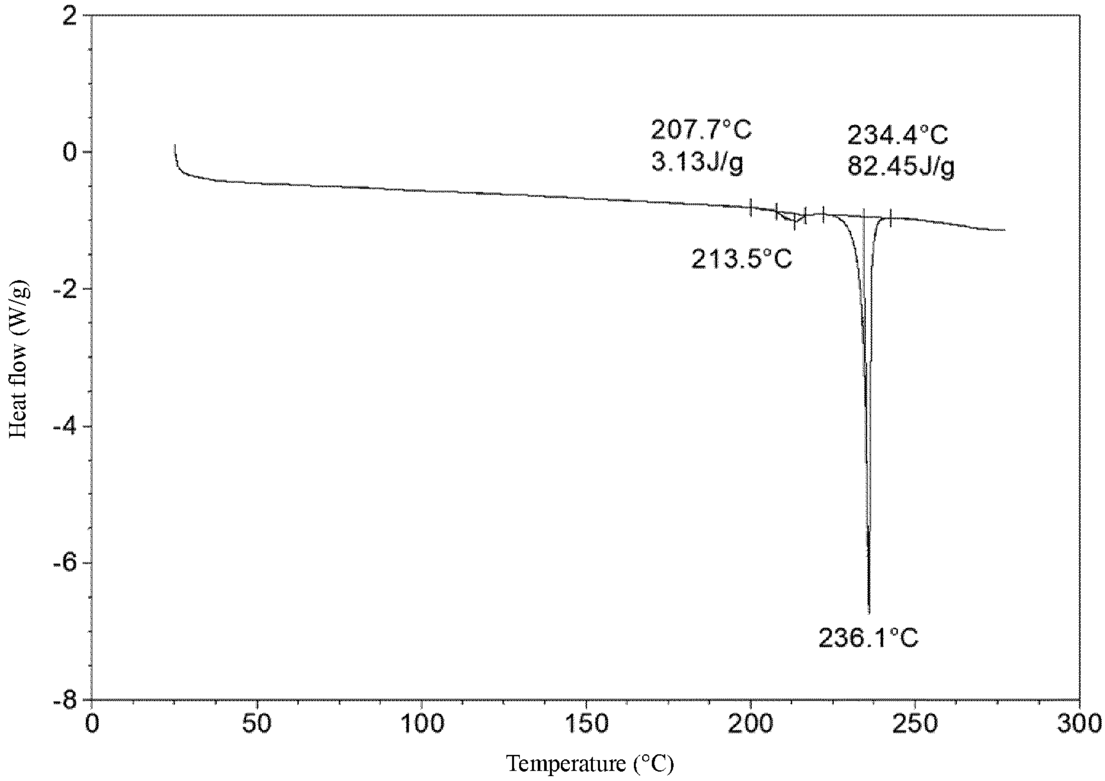
207.7°C
3.13J/g
234.4°C
82.45J/g
213.5°C
236.1°C
Heat flow (W/g)
Temperature (°C)
2
0
-2
-4
-6
-8
0
50
100
150
200
250
300

FIG. 46

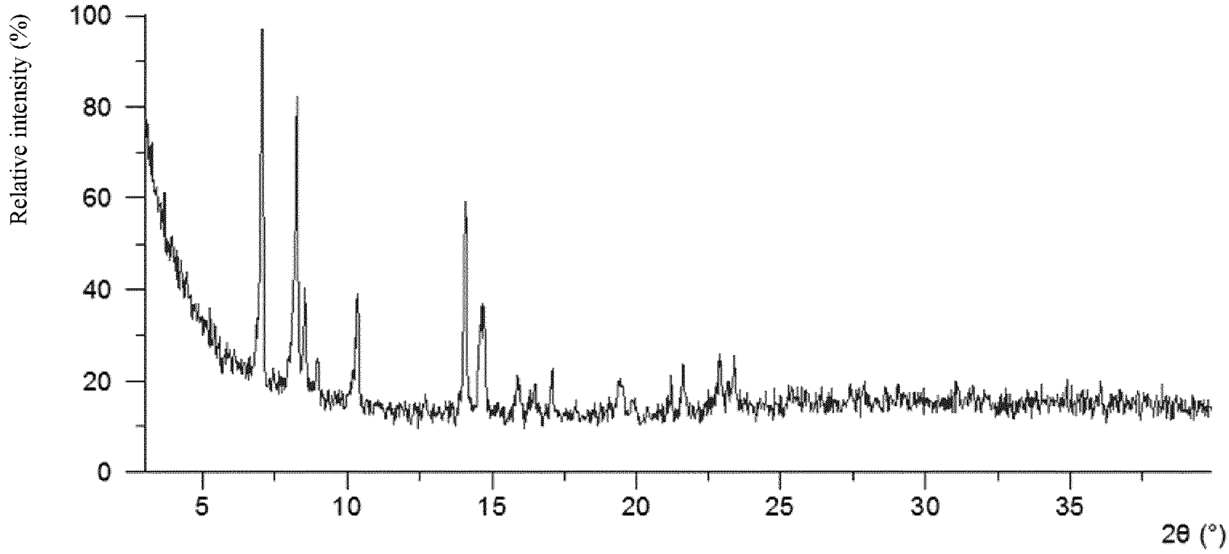
Relative intensity (%)
100
80
60
40
20
0
5
10
15
20
25
30
35
2θ (°)

FIG. 47

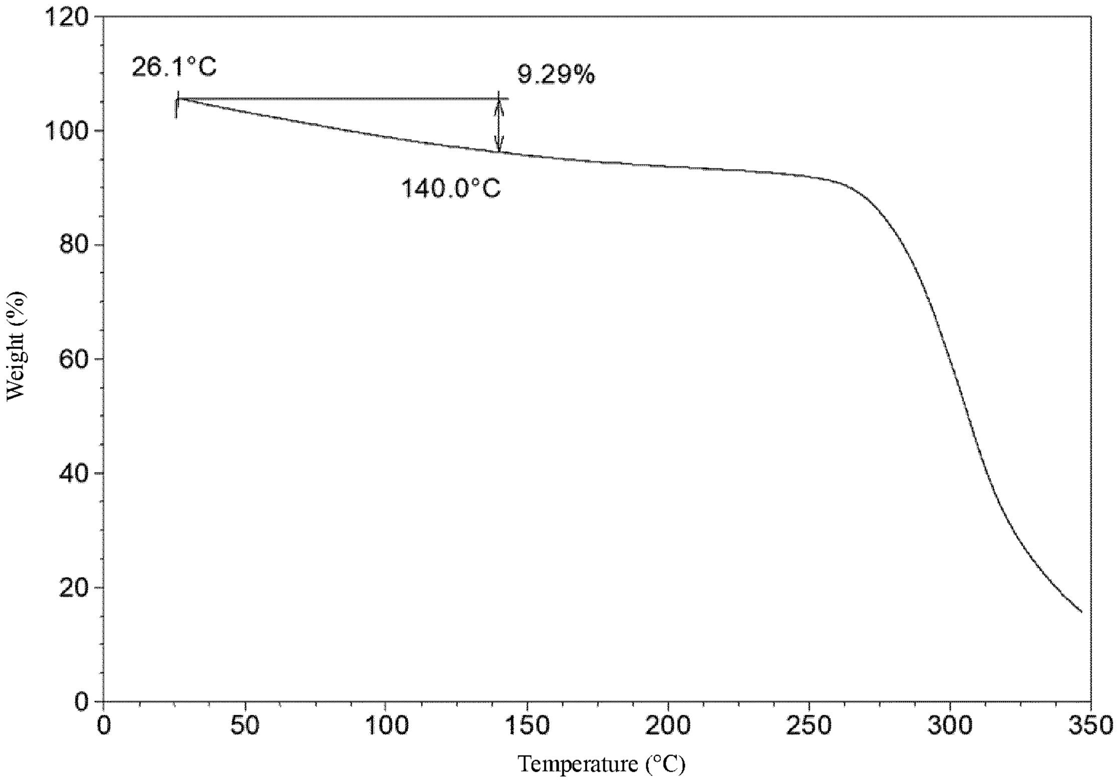
26.1°C
9.29%
140.0°C
Weight (%)
Temperature (°C)
120
100
80
60
40
20
0
0
50
100
150
200
250
300
350

FIG. 48

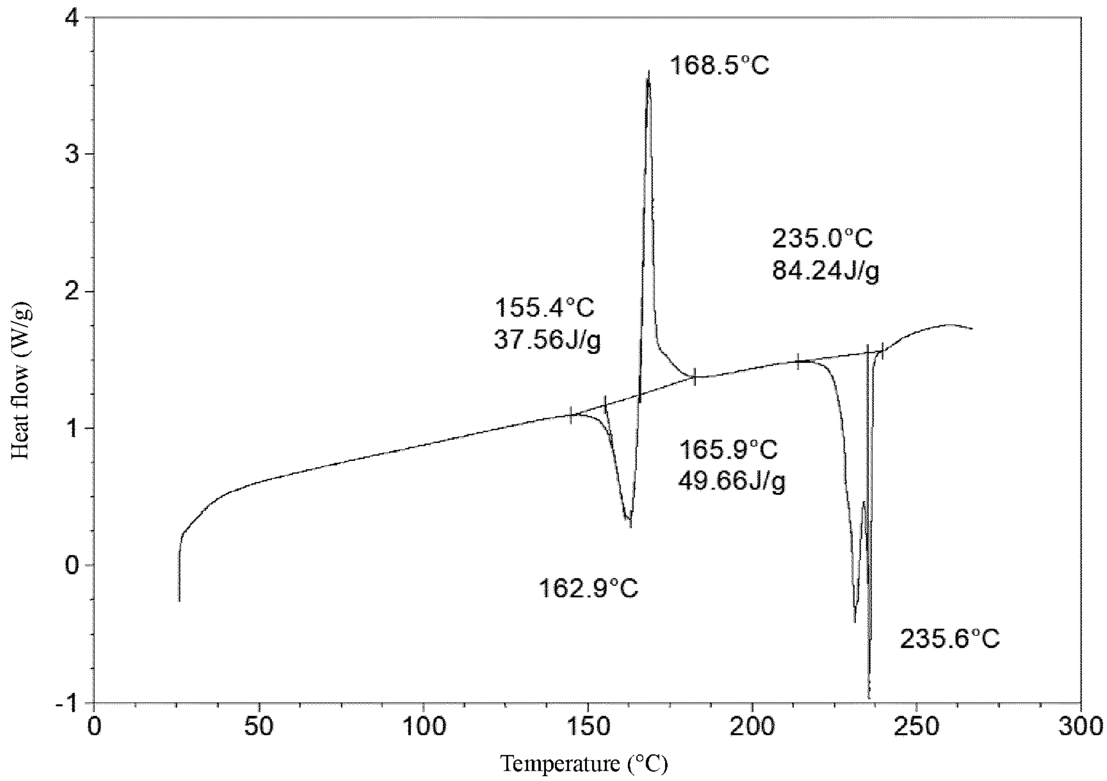
168.5°C
235.0°C
84.24J/g
155.4°C
37.56J/g
165.9°C
49.66J/g
162.9°C
235.6°C
Heat flow (W/g)
Temperature (°C)
4
3
2
1
0
-1
0
50
100
150
200
250
300

FIG. 49

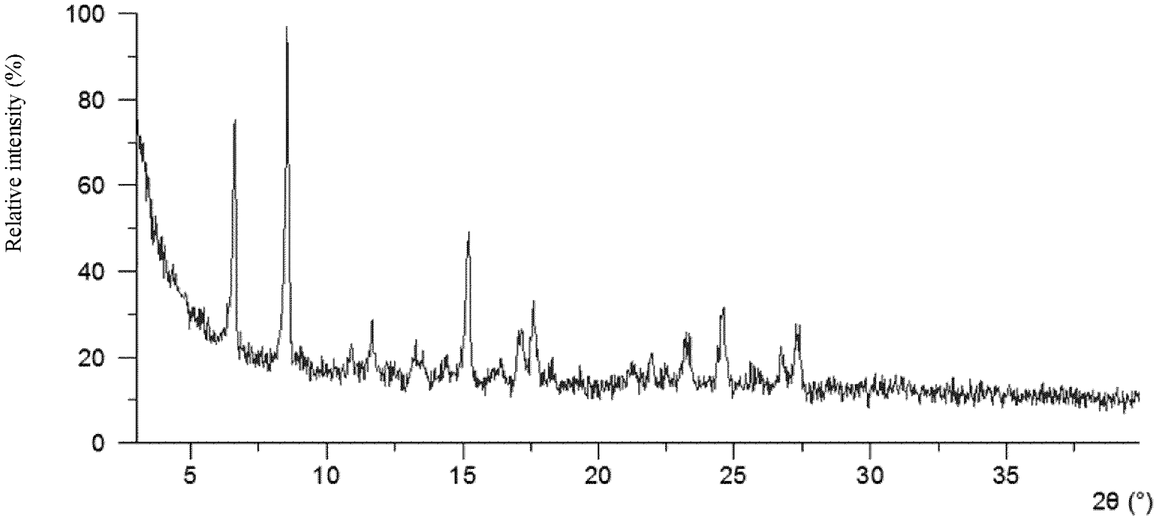


FIG. 50

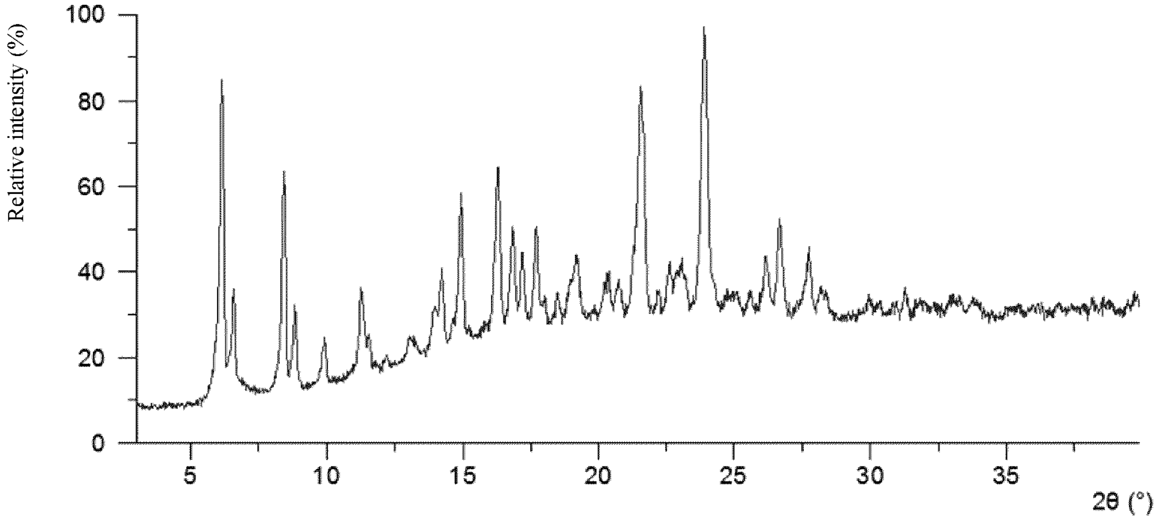


FIG. 51

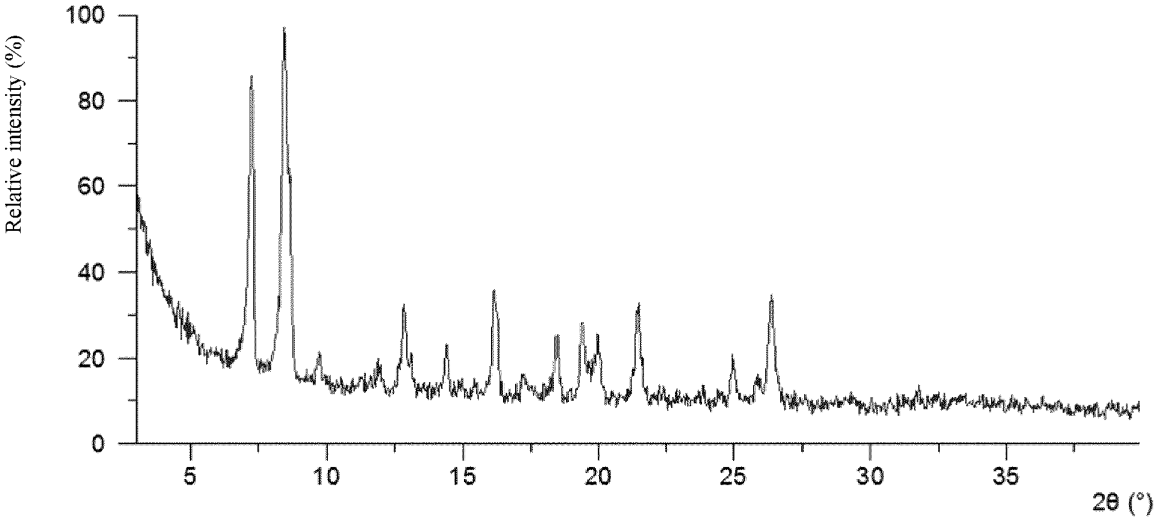


FIG. 52

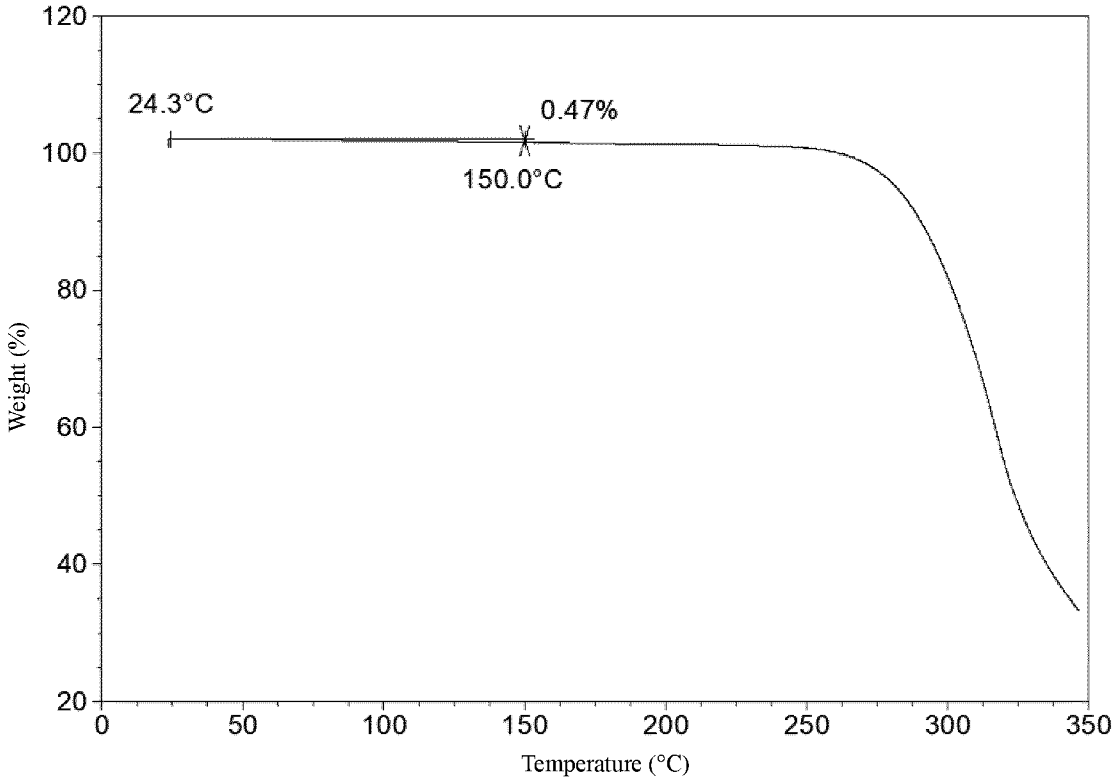
120
100
80
60
40
20
Weight (%)
24.3°C
0.47%
150.0°C
0
50
100
150
200
250
300
350
Temperature (°C)

FIG. 53

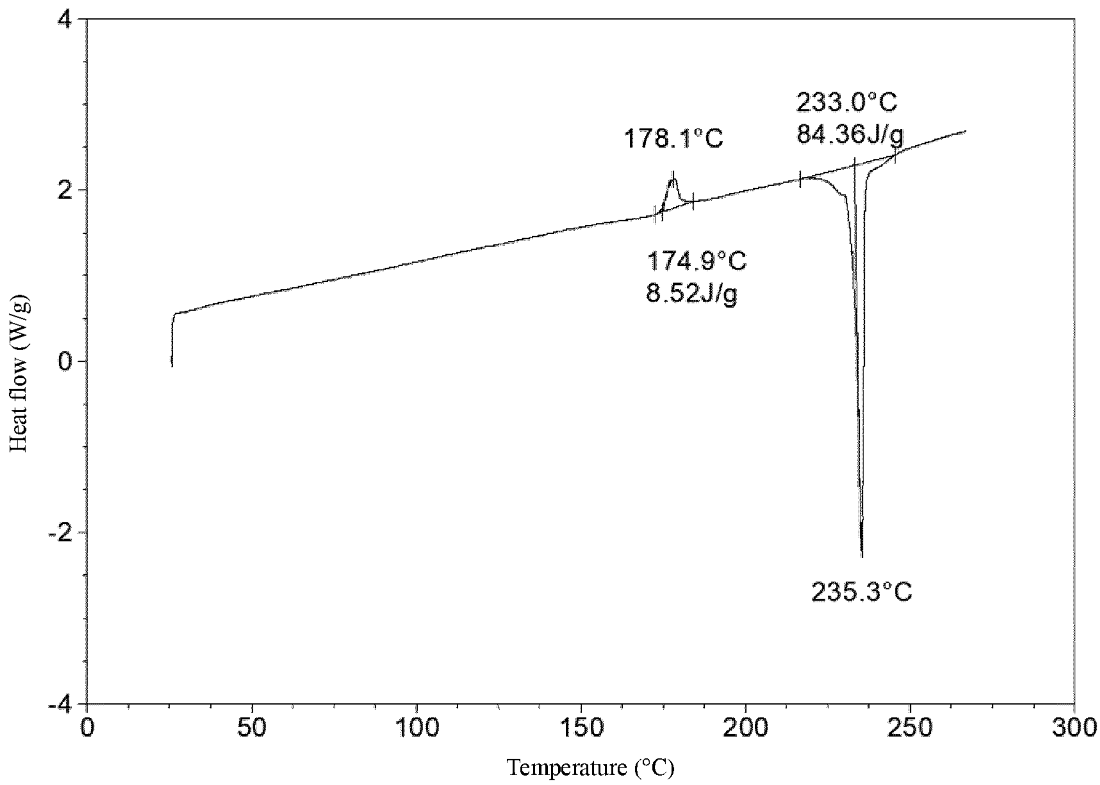
4
2
0
-2
-4
Heat flow (W/g)
178.1°C
174.9°C
8.52J/g
233.0°C
84.36J/g
235.3°C
0
50
100
150
200
250
300
Temperature (°C)

FIG. 54

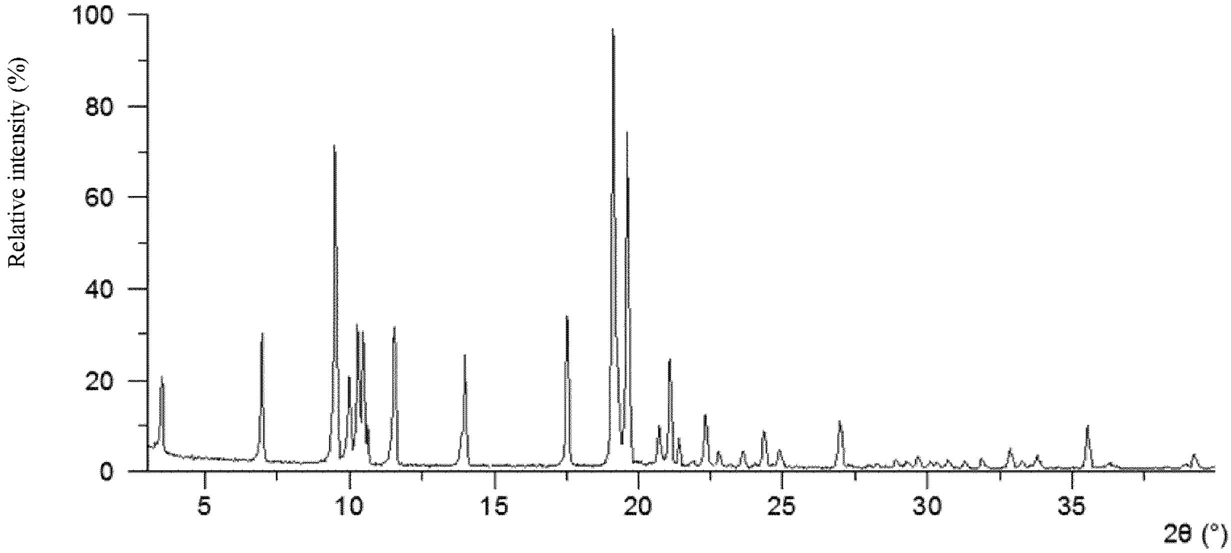
100
80
60
40
20
0
Relative intensity (%)
5
10
15
20
25
30
35
2θ (°)

FIG. 55

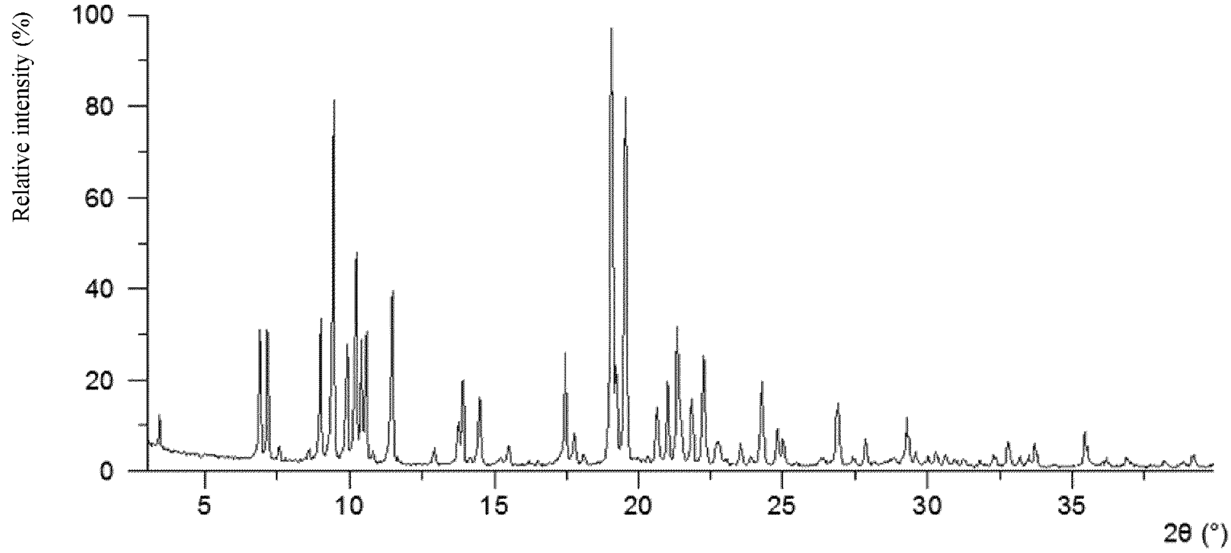
100
80
60
40
20
0
Relative intensity (%)
5
10
15
20
25
30
35
2θ (°)

FIG. 56

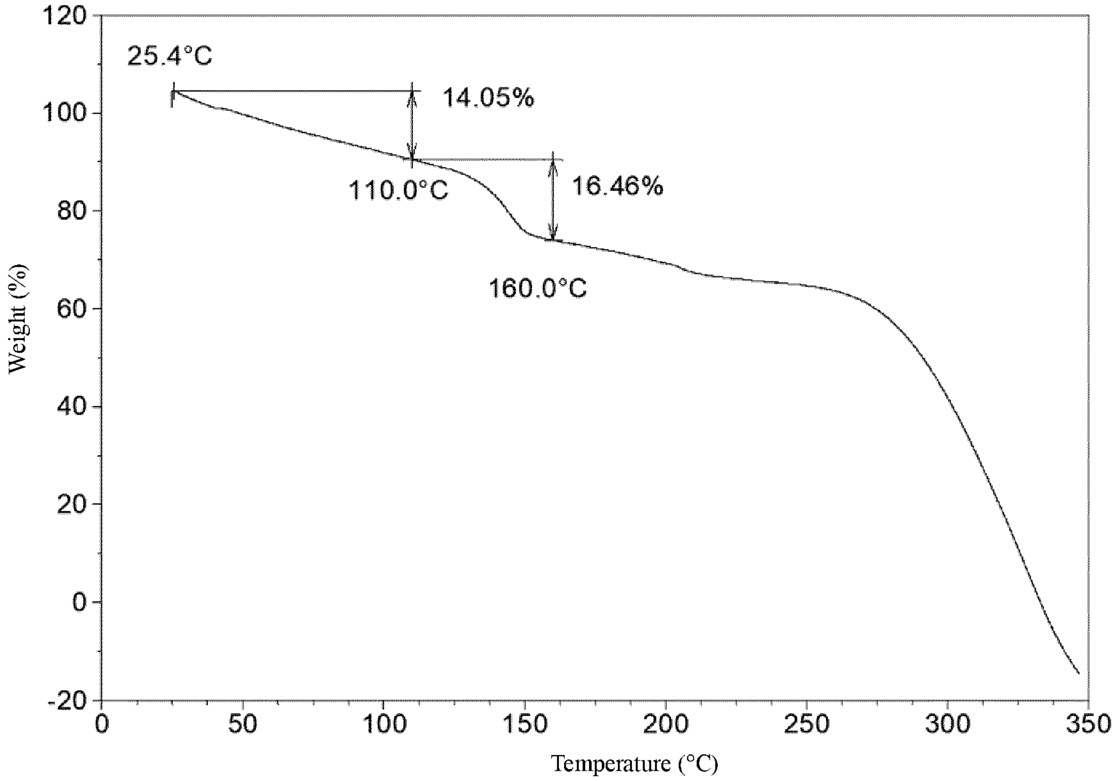
120
100
80
60
40
20
0
-20
0
50
100
150
200
250
300
350
Weight (%)
Temperature (°C)
25.4°C
14.05%
110.0°C
16.46%
160.0°C

FIG. 57

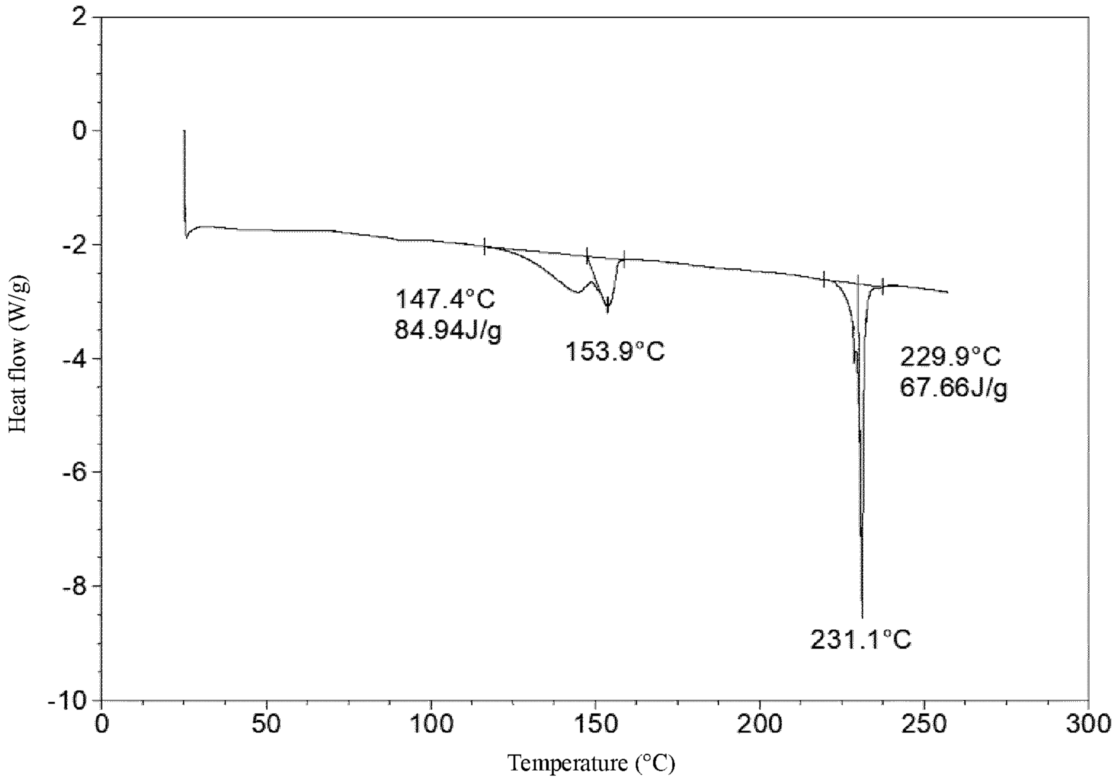
2
0
-2
-4
-6
-8
-10
0
50
100
150
200
250
300
Heat flow (W/g)
Temperature (°C)
147.4°C
84.94J/g
153.9°C
229.9°C
67.66J/g
231.1°C

FIG. 58

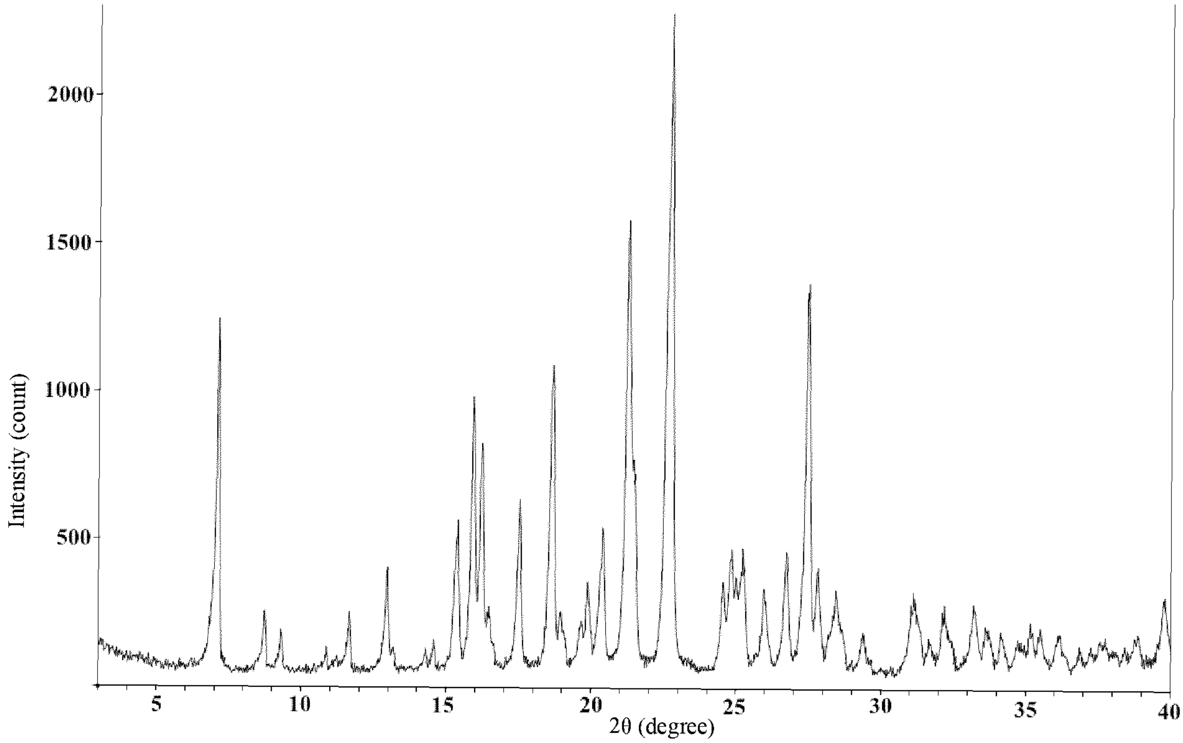

2000
1500
1000
500
Intensity (count)
5
10
15
20
25
30
35
40
2θ (degree)


FIG. 59

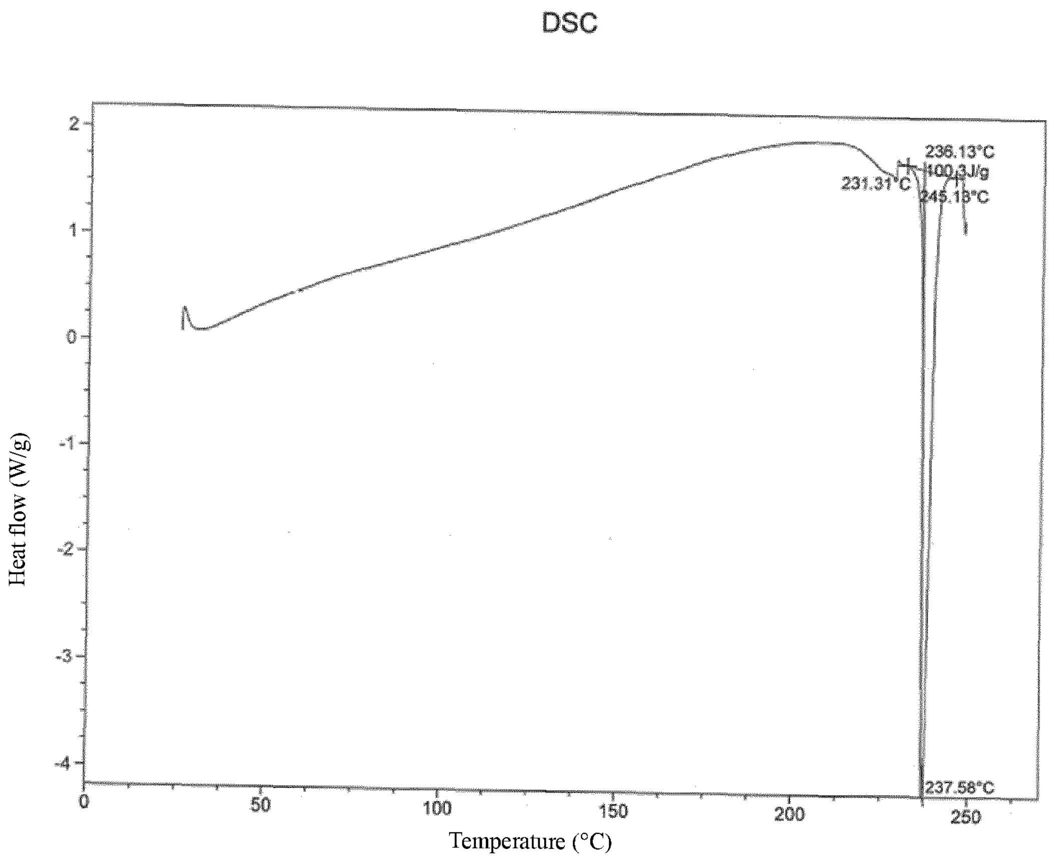

DSC
2
1
0
-1
-2
-3
-4
Heat flow (W/g)
236.13°C
100.3J/g
231.31°C
245.18°C
237.58°C
0
50
100
150
200
250
Temperature (°C)


FIG. 60

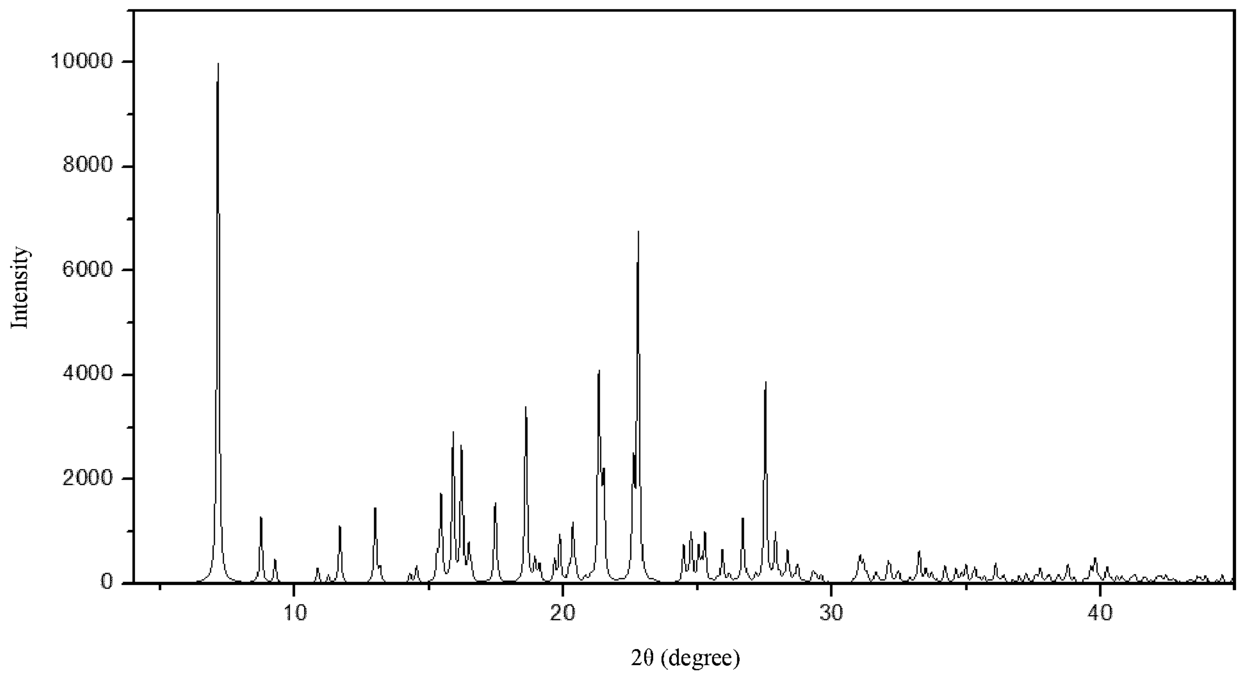
10000
8000
6000
4000
2000
0
Intensity
10
20
30
40
2θ (degree)

FIG. 61

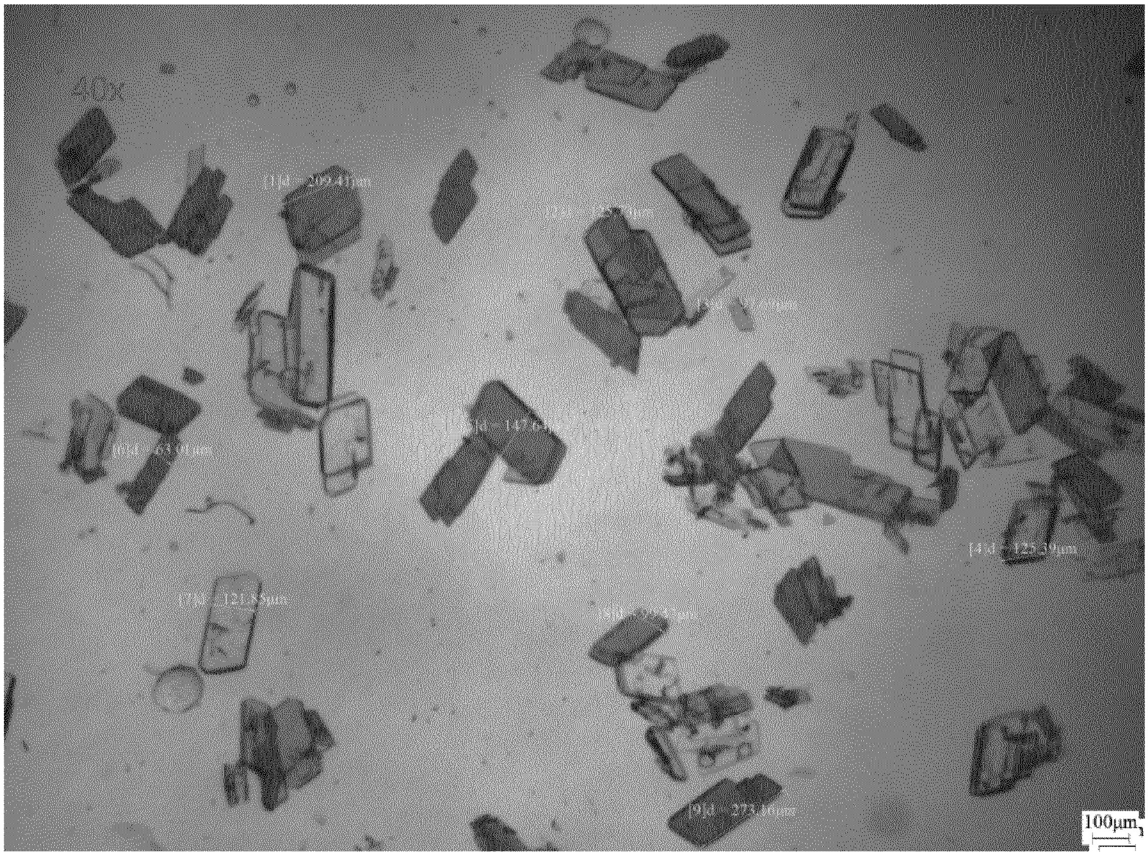
40x
100μm

FIG. 62

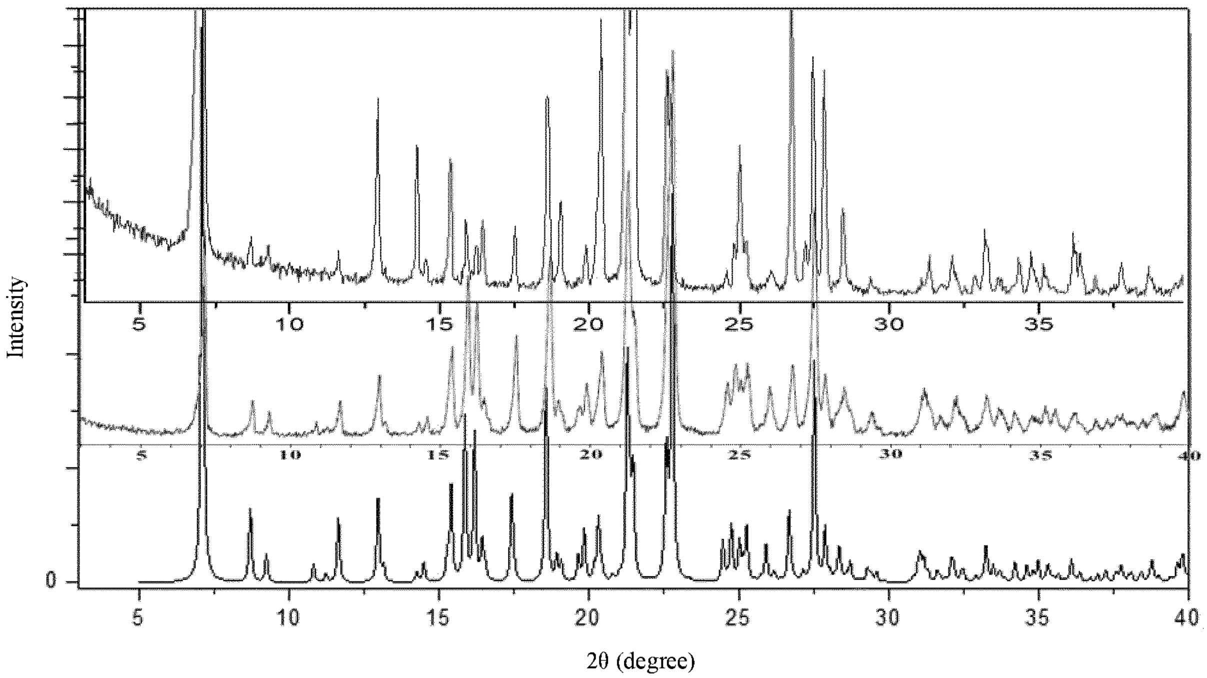


FIG. 63

**INTERNATIONAL SEARCH REPORT**

International application No.
**PCT/CN2020/130612**

**A. CLASSIFICATION OF SUBJECT MATTER**

C07D 487/04(2006.01)i; A61K 31/407(2006.01)i; A61P 31/12(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS; CNTXT; SIPOABS; WPI; WOTXT; USTXT; EPTXT; STN; 百度学术: 核蛋白抑制剂, 2387919-77-7, 晶体, 乙肝, HBV, crystal+, hepatitis B virus, 正大天晴药业集团股份有限公司, 南京明德新药研发有限公司, 贺海鹰, 夏建华, 谭海忠

**C. DOCUMENTS CONSIDERED TO BE RELEVANT**

| Category* | Citation of document, with indication, where appropriate, of the relevant passages | Relevant to claim No. |
|---|---|---|
| PX | WO 2019223791 A1 (CHIA TAI TIANQING PHARMACEUTICAL GROUP CO., LTD. et al.) 28 November 2019 (2019-11-28)<br>claims 22-24 | 1-29 |
| A | WO 2019165374 A1 (GILEAD SCIENCES, INC.) 29 August 2019 (2019-08-29)<br>abstract, claims 1-52, 63 | 1-29 |
| A | CN 109790168 A (GILEAD SCIENCES, INC.) 21 May 2019 (2019-05-21)<br>abstract, claims 1-58, 69 | 1-29 |
| A | CN 108347943 A (ASSEMBLY BIOSCIENCES INC et al.) 31 July 2018 (2018-07-31)<br>abstract, and claims 1-25 | 1-29 |
| A | CN 109069488 A (ENANTA PHARMACEUTICALS, INC.) 21 December 2018 (2018-12-21)<br>abstract, and claims 1-12 | 1-29 |
| A | CN 107778310 A (SHANGHAI FIRST PEOPLES HOSPITAL) 09 March 2018 (2018-03-09)<br>abstract, and claims 1-9 | 1-29 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 January 2021** | **24 February 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/130612**

| Patent document cited in search report | | | Publication date (day/month/year) | Patent family member(s) | | | Publication date (day/month/year) |
|---|---|---|---|---|---|---|---|
| WO | 2019223791 | A1 | 28 November 2019 | CN | 111247151 | A | 05 June 2020 |
| WO | 2019165374 | A1 | 29 August 2019 | CA | 3091142 | A1 | 29 August 2019 |
| | | | | US | 2019292187 | A1 | 26 September 2019 |
| | | | | TW | 201945366 | A | 01 December 2019 |
| | | | | CN | 111788204 | A | 16 October 2020 |
| | | | | AU | 2019223182 | A1 | 27 August 2020 |
| CN | 109790168 | A | 21 May 2019 | US | 2020061024 | A1 | 27 February 2020 |
| | | | | KR | 20190039798 | A | 15 April 2019 |
| | | | | CL | 2019001976 | A1 | 06 December 2019 |
| | | | | PE | 20190631 | A1 | 26 April 2019 |
| | | | | PH | 12019500393 | A1 | 20 January 2020 |
| | | | | AU | 2020203499 | A1 | 18 June 2020 |
| | | | | CR | 20190100 | A | 02 May 2019 |
| | | | | CA | 3033681 | A1 | 01 March 2018 |
| | | | | EC | SP19012917 | A | 29 March 2019 |
| | | | | BR | 112019003415 | A2 | 21 May 2019 |
| | | | | JP | 2019530652 | A | 24 October 2019 |
| | | | | US | 2018161307 | A1 | 14 June 2018 |
| | | | | CL | 2019000473 | A1 | 12 July 2019 |
| | | | | SG | 11201901131V | A | 28 March 2019 |
| | | | | MX | 2019002049 | A | 08 July 2019 |
| | | | | WO | 2018039531 | A1 | 01 March 2018 |
| | | | | US | 10328053 | B2 | 25 June 2019 |
| | | | | NZ | 750757 | A | 28 August 2020 |
| | | | | TW | 201819384 | A | 01 June 2018 |
| | | | | AU | 2017315863 | A1 | 07 March 2019 |
| | | | | CO | 2019001634 | A2 | 08 March 2019 |
| | | | | JP | 6774562 | B2 | 28 October 2020 |
| | | | | UY | 37374 | A | 23 March 2018 |
| | | | | EP | 3504212 | A1 | 03 July 2019 |
| | | | | AU | 2017315863 | B2 | 05 March 2020 |
| | | | | DO | P2019000040 | A | 15 March 2019 |
| CN | 108347943 | A | 31 July 2018 | EP | 3349580 | B1 | 19 August 2020 |
| | | | | MX | 2018003206 | A | 06 September 2018 |
| | | | | PH | 12018500571 | A1 | 01 October 2018 |
| | | | | US | 10766890 | B2 | 08 September 2020 |
| | | | | WO | 2017048962 | A1 | 23 March 2017 |
| | | | | TW | 201718570 | A | 01 June 2017 |
| | | | | CN | 108348529 | A | 31 July 2018 |
| | | | | CL | 2019001433 | A1 | 27 September 2019 |
| | | | | US | 10392379 | B2 | 27 August 2019 |
| | | | | CN | 108347943 | B | 10 July 2020 |
| | | | | US | 2018258084 | A1 | 13 September 2018 |
| | | | | KR | 20180050406 | A | 14 May 2018 |
| | | | | US | 2020131168 | A1 | 30 April 2020 |
| | | | | EP | 3349761 | A4 | 06 March 2019 |
| | | | | EP | 3349581 | A4 | 24 April 2019 |
| | | | | JP | 2018531921 | A | 01 November 2018 |
| | | | | JP | 2018531918 | A | 01 November 2018 |
| | | | | AU | 2016323297 | A1 | 12 April 2018 |

Form PCT/ISA/210 (patent family annex) (January 2015)

**INTERNATIONAL SEARCH REPORT**
**Information on patent family members**

International application No.
**PCT/CN2020/130612**

| Patent document cited in search report | Publication date (day/month/year) | Patent family member(s) | Publication date (day/month/year) |
|---|---|---|---|
| | | TW 201720802 A | 16 June 2017 |
| | | US 2018273520 A1 | 27 September 2018 |
| | | WO 2017048950 A1 | 23 March 2017 |
| | | TW 201720820 A | 16 June 2017 |
| | | EP 3349580 A4 | 15 May 2019 |
| | | EA 201890731 A1 | 31 October 2018 |
| | | IL 258124 D0 | 31 May 2018 |
| | | MX 2018003207 A | 21 August 2018 |
| | | US 2018265484 A1 | 20 September 2018 |
| | | HK 1259141 A1 | 29 November 2019 |
| | | EP 3349581 A1 | 25 July 2018 |
| | | EP 3349761 A1 | 25 July 2018 |
| | | DO P2018000074 A | 30 June 2018 |
| | | US 10377748 B2 | 13 August 2019 |
| | | EP 3349580 A1 | 25 July 2018 |
| | | CA 2998803 A1 | 23 March 2017 |
| | | HK 1258761 A1 | 22 November 2019 |
| | | JP 2018531919 A | 01 November 2018 |
| | | WO 2017048954 A1 | 23 March 2017 |
| | | CL 2018000684 A1 | 28 December 2018 |
| | | CA 2998741 A1 | 23 March 2017 |
| | | IL 258097 D0 | 31 May 2018 |
| | | MX 2018003198 A | 23 August 2018 |
| | | EA 201890735 A1 | 31 October 2018 |
| | | KR 20180050405 A | 14 May 2018 |
| | | KR 20180053363 A | 21 May 2018 |
| | | CA 2998793 A1 | 23 March 2017 |
| | | AR 106041 A1 | 06 December 2017 |
| | | HK 1258697 A1 | 15 November 2019 |
| | | EA 201890736 A1 | 31 October 2018 |
| | | PH 12018500573 A1 | 17 September 2018 |
| CN 109069488 A | 21 December 2018 | US 2019084994 A1 | 21 March 2019 |
| | | EP 3426245 A1 | 16 January 2019 |
| | | CO 2018010748 A2 | 14 December 2018 |
| | | US 10179792 B2 | 15 January 2019 |
| | | IL 261599 D0 | 31 October 2018 |
| | | CA 3017020 A1 | 14 September 2017 |
| | | JP 2019513129 A | 23 May 2019 |
| | | WO 2017155844 A1 | 14 September 2017 |
| | | US 10538532 B2 | 21 January 2020 |
| | | US 2020199143 A1 | 25 June 2020 |
| | | PH 12018550148 A1 | 25 March 2019 |
| | | KR 20180134875 A | 19 December 2018 |
| | | AU 2017229007 A1 | 25 October 2018 |
| | | US 2017253609 A1 | 07 September 2017 |
| | | CL 2018002528 A1 | 08 February 2019 |
| | | SG 11201807543 Y A | 27 September 2018 |
| | | BR 112018067964 A2 | 15 January 2019 |
| | | MX 2018010805 A | 28 March 2019 |
| | | EP 3426245 A4 | 31 July 2019 |

Form PCT/ISA/210 (patent family annex) (January 2015)

**INTERNATIONAL SEARCH REPORT**
**Information on patent family members**

International application No.
**PCT/CN2020/130612**

| Patent document cited in search report | Publication date (day/month/year) | Patent family member(s) | Publication date (day/month/year) |
|---|---|---|---|
| CN 107778310 A | 09 March 2018 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201911171978


### Non-patent literature cited in the description


- X-ray Structure Analysis. Tongji University Press, April 2003
- Measurement of Crystal Structure by Powder Diffraction Method. Science Press, April 2003